# EUROPEAN PATENT APPLICATION

(11) **EP 4 775 586 A1**
(43) Date of publication of application: **15.07.2026**
(21) Application number: 24861945.4
(22) Date of filing: 03.09.2024
(51) Int. Cl.: C07D 493/04, A61K 31/343, A61P 31/04

(54) **DEUTERATED TETRACYCLIC DERIVATIVE AND USE THEREOF**

(30) Priority: 04.09.2023 CN 202311138113
(71) Applicant: Zhejiang Wolwo Yifang Pharmaceutical Co., Ltd, Huzhou, Zhejiang 313299 (CN)
(72) Inventor: HE, Jianming, Huzhou, Zhejiang 313299 (CN)
(74) Representative: Simmons & Simmons LLP (Munich)
(86) International application number: PCT/CN2024/116454
(87) International publication number: WO 2025/051102

(57) **Abstract**

Disclosed are a deuterated tetracyclic derivative and a use thereof in prevention or treatment of diseases caused by bacterial infections, particularly in pulmonary diseases caused by mycobacterium tuberculosis or drug-resistant mycobacterium tuberculosis.

## Description

### TECHNICAL FIELD

The present disclosure relates to a deuterated compound, a pharmaceutical composition comprising the deuterated compound, and use thereof, and in particular, to a deuterated tetracyclic derivative and pharmaceutical use thereof.

### BACKGROUND

Deuterated drugs refer to drugs in which some of the hydrogen atoms in the drug molecule are replaced with deuterium. Deuterium (D), as a non-radioactive stable isotope of hydrogen, has a form and volume similar to hydrogen in drugs, allowing the biological activity and selectivity of the drug molecule to remain substantially unchanged. In addition, the C-D bond is more stable than the C-H bond, which can delay the decomposition process of the drug, prolong the action time of the drug *in vivo,* and affect the metabolism, thereby improving the pharmacokinetics, reducing the drug metabolism toxicity, and improving the bioavailability. Ultimately, the purposes of reducing the administration frequency, improving the medication compliance of the patient, achieving the same therapeutic effect at lower doses, and reducing the adverse drug reactions to obtain clinical benefits are achieved. The first deuterated drug, deutetrabenazine (trade name: Austedo), was approved by the U.S. Food and Drug Administration (FDA) in 2017.

*Mycobacterium tuberculosis* can cause infections at multiple body parts with drug resistance, such as lung, intestine, peritoneum, kidney, bladder, ureter, pleura, bone, joint, brain, and reproductive system. The most common form is pulmonary infection-tuberculosis. Currently, first-line treatment drugs for tuberculosis include isoniazid, rifampicin, streptomycin, ethambutol, and the like. However, these drugs now commonly exhibit drug resistance and serious side effects, and new anti-tuberculosis drugs and diversified treatment strategies are urgently needed.

### SUMMARY

In one aspect, the present disclosure provides a compound represented by formula I, or a pharmaceutically acceptable salt, a stereoisomer, a tautomer, an N-oxide, a hydrate, a solvate, or a prodrug thereof: 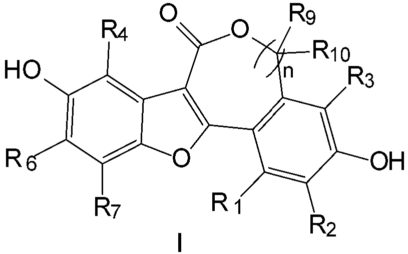
wherein n is 0 or 1;
R₄ is selected from H, halogen, an aldehyde group, hydroxy, sulfhydryl, - NR'R", cyano, nitro, optionally substituted C₁-C₁₀ alkyl, optionally substituted C₂-C₁₀ alkenyl, optionally substituted C₂-C₁₀ alkynyl, optionally substituted C₃-C₈ carbocyclyl, optionally substituted C₆-C₁₄ aryl, optionally substituted C₅-C₁₀ heteroaryl, optionally substituted C₄-C₁₀ heterocyclyl, and respective deuterated variants thereof; preferably, R₄ is selected from optionally substituted C₆-C₁₄ aryl C₁-C₁₀ alkyl, optionally substituted C₅-C₁₀ heteroaryl C₁-C₁₀ alkyl, optionally substituted C₄-C₁₀ heterocyclyl C₁-C₁₀ alkyl, and respective deuterated variants thereof;
R₇ is selected from H, hydroxy, sulfhydryl, cyano, carboxyl, nitro, -NR'R", optionally substituted C₁-C₁₀ alkyl, optionally substituted C₂-C₁₀ alkenyl, optionally substituted C₂-C₁₀ alkynyl, C₁-C₁₀ ester group, amido, halogen, optionally substituted C₃-C₈ carbocyclyl, optionally substituted C₁-C₁₀ alkoxy, optionally substituted C₆-C₁₄ aryl, optionally substituted C₅-C₁₀ heteroaryl, optionally substituted C₄-C₁₀ heterocyclyl, and respective deuterated variants thereof, wherein R' and R" are each independently selected from: H, optionally substituted C₁-C₁₀ alkyl, optionally substituted C₂-C₈ alkenyl, and optionally substituted C₁-C₁₀ alkoxy;
R₁, R₂, R₃, R₆, R₉, and R₁₀ are independently selected from hydrogen and deuterium; and
the structure represented by formula I comprises at least one deuterium atom, e.g., 2, 3, 4, 5, 6, 7, 8, 9, 10, 11, 12, 13, 14, 15, 16, 17, 18, 19, or 20 deuterium atoms.

In a preferred embodiment, R₄ is -(CR₁₁R₁₂)ₘ₋NRₐR_{b},
wherein m is 1, 2, or 3;
R₁₁ and R₁₂ are independently selected from hydrogen and deuterium; and
Rₐ and R_{b} are each independently selected from the following substituents and respective deuterated variants thereof: H; C₁-C₁₀ alkyl optionally substituted with 1-5 substituents selected from halogen and hydroxy; C₂-C₈ alkenyl optionally substituted with 1-5 substituents selected from halogen and hydroxy; 3- to 14-membered carbocyclyl optionally substituted with 1-5 substituents selected from halogen and C₁-C₄ alkyl; C₁-C₆ alkyl substituted with 3- to 14-membered carbocyclyl optionally substituted with 1-5 substituents selected from halogen and C₁-C₄ alkyl; C₁-C₆ alkyl substituted with 4- to 10-membered heterocyclyl optionally substituted with 1-5 substituents selected from halogen, hydroxy, and C₁-C₄ alkyl; C₆-C₁₄ aryl optionally substituted with 1-5 substituents selected from halogen, hydroxy, and C₁-C₄ alkyl; and C₆-C₁₄ aryl-C₁-C₆ alkyl optionally substituted with 1-5 substituents selected from halogen, hydroxy, and C₁-C₄ alkyl; or Rₐ and R_{b}, together with the nitrogen atom to which they are attached, form an optionally substituted 4- to 7-membered heterocyclyl or benzo 4- to 7-membered heterocyclyl or a deuterated variant thereof, wherein the 4-to 7-membered heterocyclyl or benzo 4- to 7-membered heterocyclyl is optionally substituted with 1-5 substituents selected from: C₁-C₆ alkyl optionally substituted with 1-5 substituents selected from halogen and hydroxy; C₁-C₆ alkoxy optionally substituted with 1-5 substituents selected from halogen and hydroxy; cyano; carboxyl; halogen; -NR^{c}R^{d}; hydroxy; C₆-C₁₄ aryl optionally substituted with 1-5 substituents selected from C₁-C₄ acyl, halogen, NR^{c}R^{d}, C₁-C₄ alkoxy, C₁-C₄ alkyl, cyano, hydroxy, C₁-C₄ alkoxycarbonyl, C₆-C₁₄ aryl, and halogenated C₁-C₄ alkyl; C₁-C₆ acyl; C₁-C₆ alkoxycarbonyl; C₂-C₈ alkenyloxycarbonyl; C₁-C₆ acyl substituted with NR^{c}R^{d}; C₁-C₄ acyl substituted with C₆-C₁₄ aryl optionally substituted with 1-5 substituents selected from halogen, C₁-C₄ alkyl, halogenated C₁-C₄ alkyl, and hydroxy; C₁-C₆ alkyl substituted with C₆-C₁₄ aryl optionally substituted with 1-5 substituents selected from halogen, C₁-C₄ alkyl, halogenated C₁-C₄ alkyl, and hydroxy; C₁-C₆ alkyl substituted with C₃-C₈ cycloalkyl optionally substituted with 1-5 substituents selected from halogen, C₁-C₄ alkyl, halogenated C₁-C₄ alkyl, and hydroxy; C₃-C₈ cycloalkyl optionally substituted with 1-5 substituents selected from halogen, C₁-C₄ alkyl, halogenated C₁-C₄ alkyl, and hydroxy; 4- to 7-membered heterocyclyl optionally substituted with 1-5 substituents selected from halogen, C₁-C₄ alkyl, halogenated C₁-C₄ alkyl, and hydroxy; C₁-C₆ alkyl substituted with 4- to 7-membered heterocyclyl optionally substituted with 1-5 substituents selected from halogen, C₁-C₄ alkyl, halogenated C₁-C₄ alkyl, and hydroxy; 5- to 10-membered heteroaryl optionally substituted with 1-5 substituents selected from halogen, C₁-C₄ alkyl, halogenated C₁-C₄ alkyl, and hydroxy; and C₁-C₆ alkyl substituted with 5- to 10-membered heteroaryl optionally substituted with 1-5 substituents selected from halogen, C₁-C₄ alkyl, halogenated C₁-C₄ alkyl, and hydroxy, wherein R^{c} and R^{d} are each independently selected from H, C₁-C₆ alkyl, C₂-C₈ alkenyl, and C₁-C₆ alkoxy.

In a more preferred embodiment, in R₄, -NRₐR_{b} is selected from: 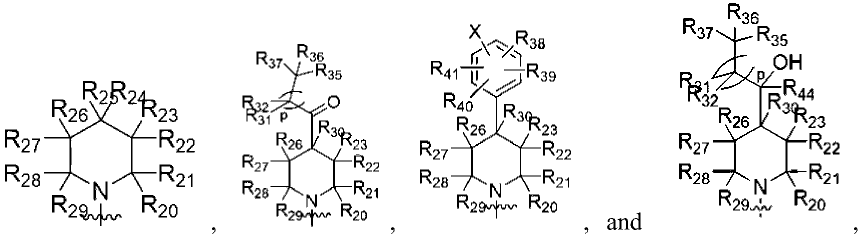
wherein p is independently 0, 1, 2, or 3, X is halogen, and R₂₀ to R₃₂, R₃₅ to R₄₁, and
R₄₄ are independently selected from hydrogen and deuterium;
preferably, -NRₐR_{b} is selected from: 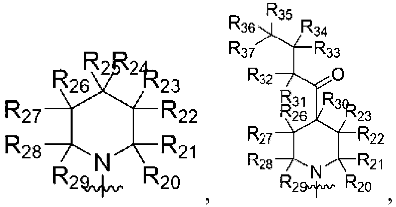 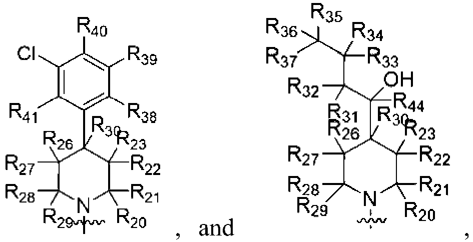 wherein R₂₀ to R₄₁, and R₄₄ are independently selected from hydrogen and deuterium.

In a more preferred embodiment, -NRₐR_{b} is selected from:

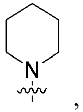

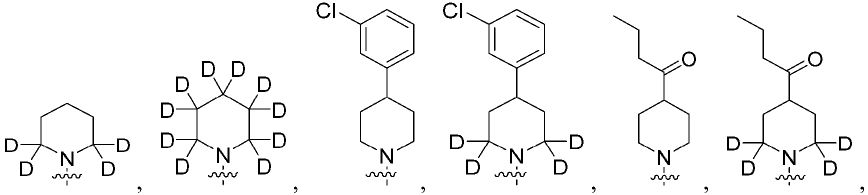

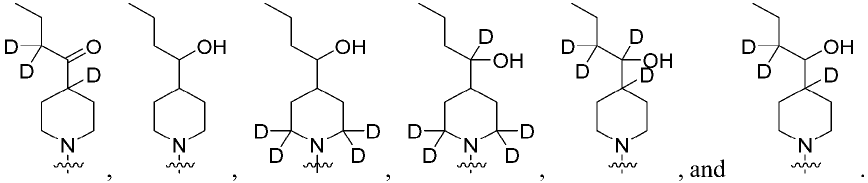

In a preferred embodiment, R₇ is selected from H, C₁-C₆ alkyl optionally substituted with 1-5 substituents selected from halogen and hydroxy, C₂-C₆ alkenyl optionally substituted with 1-5 substituents selected from halogen and hydroxy, saturated or partially saturated C₃-C₈ carbocyclyl, halogen, hydroxy, C₁-C₄ alkoxy substituted with C₆-C₁₄ aryl, 5- to 10-membered heteroaryl, and C₆-C₁₄ aryl optionally substituted with 1-3 substituents selected from C₁-C₄ alkyl, halogenated C₁-C₄ alkyl, halogen, C₂-C₄ alkenyl, and hydroxy, as well as respective deuterated variants thereof; preferably, R₇ is selected from hydrogen, deuterium, and 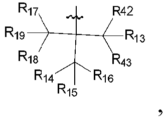 wherein R₁₃ to R₁₉, R₄₂, and R₄₃ are independently selected from hydrogen and deuterium; more preferably, R₇ is selected from hydrogen, deuterium, and

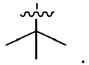

In some embodiments, the present disclosure provides a compound represented by formula II, or a pharmaceutically acceptable salt, a stereoisomer, a tautomer, an N-oxide, a hydrate, a solvate, or a prodrug thereof, 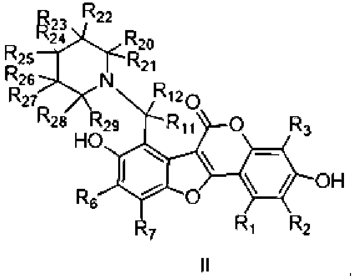
wherein R₇ is selected from hydrogen, deuterium, and 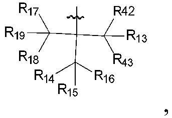 wherein R₁₃ to R₁₉, R₄₂, and R₄₃ are independently selected from hydrogen and deuterium; preferably, R₇ is 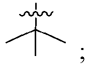
R₁, R₂, R₃, R₆, R₁₁, R₁₂, and R₂₀ to R₂₉ are independently selected from hydrogen and deuterium; and
the structure represented by formula II comprises at least one deuterium atom, preferably 2, 3, 4, 5, 6, 7, 8, 9, 10, 11, 12, 13, 14, 15, 16, 17, 18, 19, or 20 deuterium atoms.

In a preferred embodiment, the compound has a structural formula shown as IIa, IIb, IIc, or IId below:

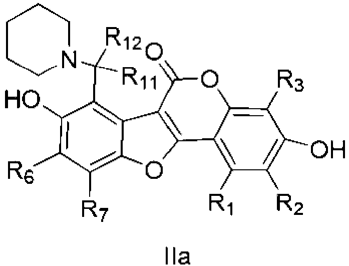

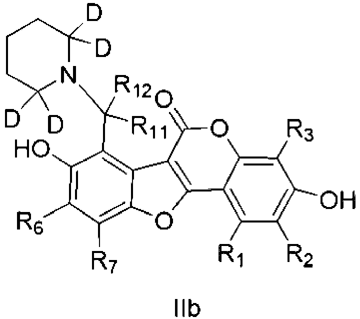

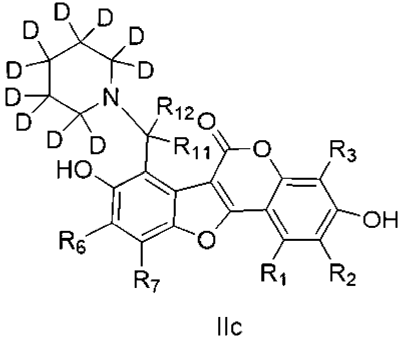

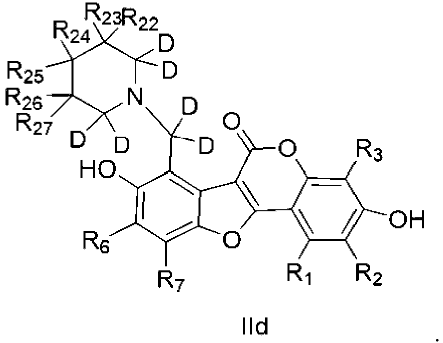

In some embodiments, the present disclosure provides a compound represented by formula III, or a pharmaceutically acceptable salt, a stereoisomer, a tautomer, an N-oxide, a hydrate, a solvate, or a prodrug thereof, 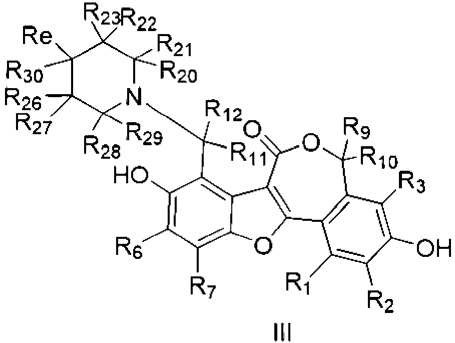
wherein Re is selected from: C₁-C₆ alkyl optionally substituted with 1-5 substituents selected from halogen and hydroxy; C₁-C₆ alkoxy optionally substituted with 1-5 substituents selected from halogen and hydroxy; cyano; carboxyl; halogen; - NR^{c}R^{d}; hydroxy; C₆-C₁₄ aryl optionally substituted with 1-5 substituents selected from C₁-C₄ acyl, halogen, NR^{c}R^{d}, C₁-C₄ alkoxy, C₁-C₄ alkyl, cyano, hydroxy, C₁-C₄ alkoxycarbonyl, C₆-C₁₄ aryl, and halogenated C₁-C₄ alkyl; C₁-C₆ acyl; C₁-C₆ alkoxycarbonyl; C₂-C₈ alkenyloxycarbonyl; C₁-C₆ acyl substituted with NR^{c}R^{d}; C₁-C₄ acyl substituted with C₆-C₁₄ aryl optionally substituted with 1-5 substituents selected from halogen, C₁-C₄ alkyl, halogenated C₁-C₄ alkyl, and hydroxy; C₁-C₆ alkyl substituted with C₆-C₁₄ aryl optionally substituted with 1-5 substituents selected from halogen, C₁-C₄ alkyl, halogenated C₁-C₄ alkyl, and hydroxy; C₁-C₆ alkyl substituted with C₃-C₈ cycloalkyl optionally substituted with 1-5 substituents selected from halogen, C₁-C₄ alkyl, halogenated C₁-C₄ alkyl, and hydroxy; C₃-C₈ cycloalkyl optionally substituted with 1-5 substituents selected from halogen, C₁-C₄ alkyl, halogenated C₁-C₄ alkyl, and hydroxy; 4- to 7-membered heterocyclyl optionally substituted with 1-5 substituents selected from halogen, C₁-C₄ alkyl, halogenated C₁-C₄ alkyl, and hydroxy; C₁-C₆ alkyl substituted with 4- to 7-membered heterocyclyl optionally substituted with 1-5 substituents selected from halogen, C₁-C₄ alkyl, halogenated C₁-C₄ alkyl, and hydroxy; 5- to 10-membered heteroaryl optionally substituted with 1-5 substituents selected from halogen, C₁-C₄ alkyl, halogenated C₁-C₄ alkyl, and hydroxy; and C₁-C₆ alkyl substituted with 5- to 10-membered heteroaryl optionally substituted with 1-5 substituents selected from halogen, C₁-C₄ alkyl, halogenated C₁-C₄ alkyl, and hydroxy; and respective deuterated variants thereof, wherein R^{c} and R^{d} are each independently selected from H, C₁-C₆ alkyl, C₂-C₈ alkenyl, and C₁-C₆ alkoxy;
R₇ is selected from H, C₁-C₆ alkyl optionally substituted with 1-5 substituents selected from halogen and hydroxy, C₂-C₆ alkenyl optionally substituted with 1-5 substituents selected from halogen and hydroxy, saturated or partially saturated C₃-C₈ carbocyclyl, halogen, hydroxy, C₁-C₄ alkoxy substituted with C₆-C₁₄ aryl, 5- to 10-membered heteroaryl, and C₆-C₁₄ aryl optionally substituted with 1-3 substituents selected from C₁-C₄ alkyl, halogenated C₁-C₄ alkyl, halogen, C₂-C₄ alkenyl, and hydroxy, as well as respective deuterated variants thereof, preferably, R₇ is selected from hydrogen, deuterium, and 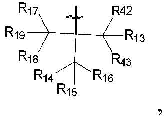 wherein R₁₃ to R₁₉, R₄₂, and R₄₃ are each independently selected from hydrogen and deuterium; more preferably, R₇ is hydrogen or deuterium;
R₁, R₂, R₃, R₆, R₉ to R₁₂, R₂₀ to R₂₃, and R₂₆ to R₃₀ are each independently selected from hydrogen and deuterium; and
the structure represented by formula III comprises at least one deuterium atom, preferably 2, 3, 4, 5, 6, 7, 8, 9, 10, 11, 12, 13, 14, 15, 16, 17, 18, 19, or 20 deuterium atoms.

In a preferred embodiment, the compound has a structural formula shown as IIIa, IIIb, or IIIc below: 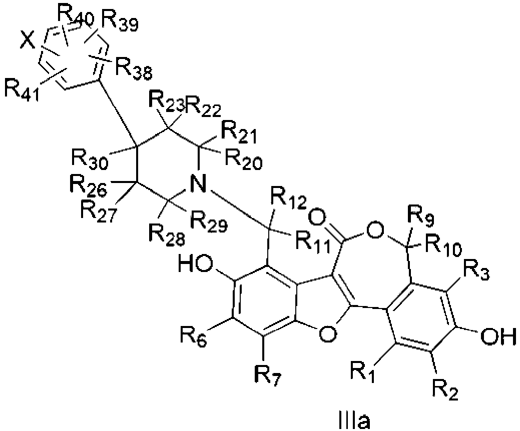 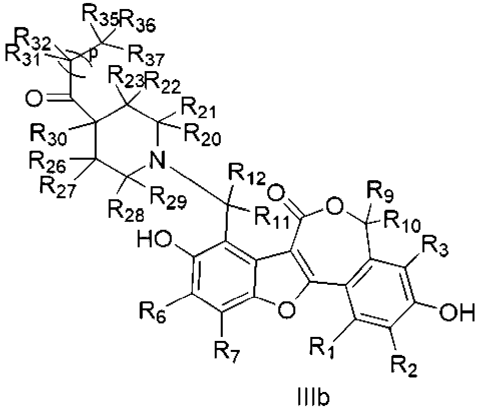 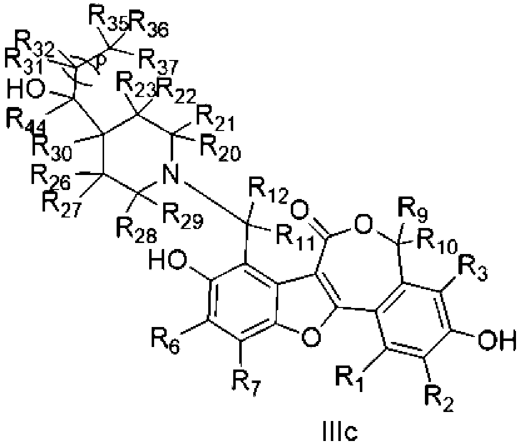
wherein p is independently 0, 1, 2, or 3, X is halogen, and R₃₁, R₃₂, R₃₅ to R₄₁, and R₄₄ are independently selected from hydrogen and deuterium;
in a more preferred embodiment, R₁, R₂, and R₃ are all hydrogen.

In a preferred embodiment, the compound has a structural formula shown as IIId, IIIe, or IIIf below:

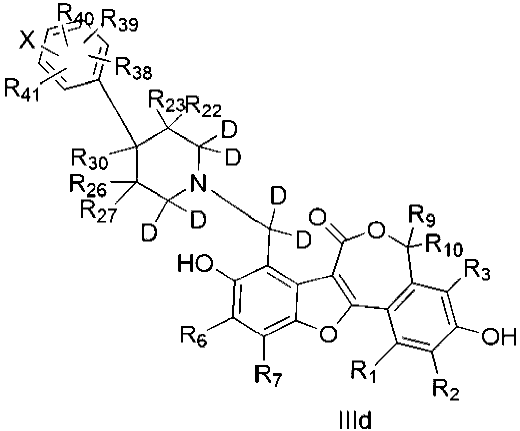

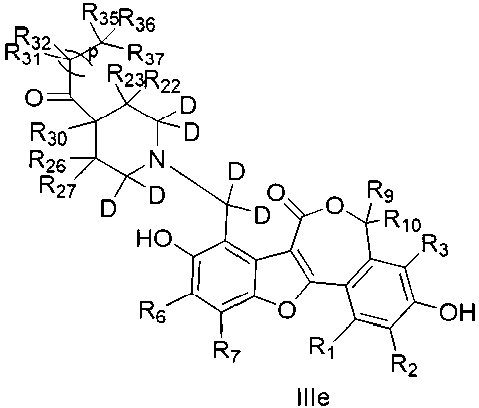

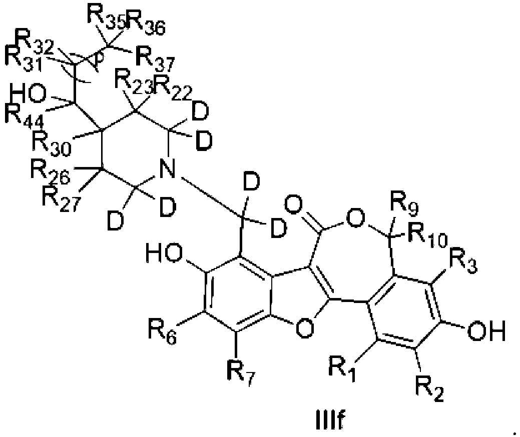

In a preferred embodiment, in the structure of formula III, Re, together with the piperidinyl attached thereto, forms a group selected from:

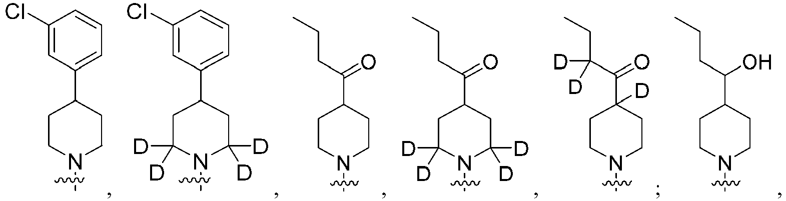

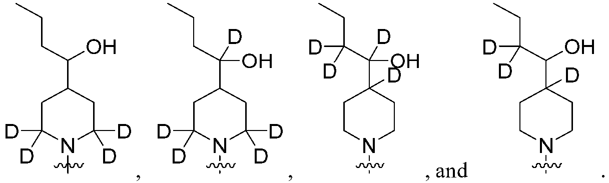

In some embodiments, the present disclosure provides a compound of formula IV, or a pharmaceutically acceptable salt, a stereoisomer, a tautomer, an N-oxide, a hydrate, a solvate, or a prodrug thereof, 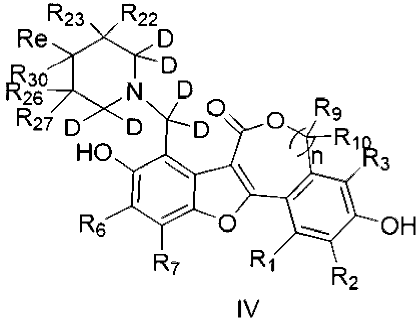
wherein n is 0 or 1;
Re is selected from: hydrogen, deuterium, C₁-C₆ alkyl optionally substituted with 1-5 substituents selected from halogen and hydroxy; C₁-C₆ alkoxy optionally substituted with 1-5 substituents selected from halogen and hydroxy; cyano; carboxyl; halogen; -NR^{c}R^{d}; hydroxy; C₆-C₁₄ aryl optionally substituted with 1-5 substituents selected from C₁-C₄ acyl, halogen, NR^{c}R^{d}, C₁-C₄ alkoxy, C₁-C₄ alkyl, cyano, hydroxy, C₁-C₄ alkoxycarbonyl, C₆-C₁₄ aryl, and halogenated C₁-C₄ alkyl; C₁-C₆ acyl; C₁-C₆ alkoxycarbonyl; C₂-C₈ alkenyloxycarbonyl; C₁-C₆ acyl substituted with NR^{c}R^{d}; C₁-C₄ acyl substituted with C₆-C₁₄ aryl optionally substituted with 1-5 substituents selected from halogen, C₁-C₄ alkyl, halogenated C₁-C₄ alkyl, and hydroxy; C₁-C₆ alkyl substituted with C₆-C₁₄ aryl optionally substituted with 1-5 substituents selected from halogen, C₁-C₄ alkyl, halogenated C₁-C₄ alkyl, and hydroxy; C₁-C₆ alkyl substituted with C₃-C₈ cycloalkyl optionally substituted with 1-5 substituents selected from halogen, C₁-C₄ alkyl, halogenated C₁-C₄ alkyl, and hydroxy; C₃-C₈ cycloalkyl optionally substituted with 1-5 substituents selected from halogen, C₁-C₄ alkyl, halogenated C₁-C₄ alkyl, and hydroxy; 4- to 7-membered heterocyclyl optionally substituted with 1-5 substituents selected from halogen, C₁-C₄ alkyl, halogenated C₁-C₄ alkyl, and hydroxy; C₁-C₆ alkyl substituted with 4- to 7-membered heterocyclyl optionally substituted with 1-5 substituents selected from halogen, C₁-C₄ alkyl, halogenated C₁-C₄ alkyl, and hydroxy; 5- to 10-membered heteroaryl optionally substituted with 1-5 substituents selected from halogen, C₁-C₄ alkyl, halogenated C₁-C₄ alkyl, and hydroxy; C₁-C₆ alkyl substituted with 5- to 10-membered heteroaryl optionally substituted with 1-5 substituents selected from halogen, C₁-C₄ alkyl, halogenated C₁-C₄ alkyl, and hydroxy; and respective deuterated variants thereof, wherein R^{c} and R^{d} are each independently selected from H, C₁-C₆ alkyl, C₂-C₈ alkenyl, and C₁-C₆ alkoxy;
R₇ is selected from H, C₁-C₆ alkyl optionally substituted with 1-5 substituents selected from halogen and hydroxy, C₂-C₆ alkenyl optionally substituted with 1-5 substituents selected from halogen and hydroxy, saturated or partially saturated C₃-C₈ carbocyclyl, halogen, hydroxy, C₁-C₄ alkoxy substituted with C₆-C₁₄ aryl, 5- to 10-membered heteroaryl, and C₆-C₁₄ aryl optionally substituted with 1-3 substituents selected from C₁-C₄ alkyl, halogenated C₁-C₄ alkyl, halogen, C₂-C₄ alkenyl, and hydroxy, as well as respective deuterated variants thereof; preferably, R₇ is selected from hydrogen, deuterium, and 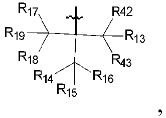 wherein R₁₃ to R₁₉, R₄₂, and R₄₃ are each independently selected from hydrogen and deuterium; more preferably, R₇ is selected from hydrogen, deuterium, and 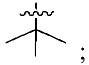
R₁, R₂, R₃, R₆, R₉, R₁₀, R₂₂, R₂₃, R₂₆, R₂₇, and R₃₀ are each independently selected from hydrogen and deuterium; preferably, R₁, R₂, and R₃ are all hydrogen, and R₆, R₉, R₁₀, R₂₂, R₂₃, R₂₆, R₂₇, and R₃₀ are each independently selected from hydrogen and deuterium.

In a preferred embodiment, in formula IV, Re, together with the piperidinyl attached thereto, forms a group selected from: 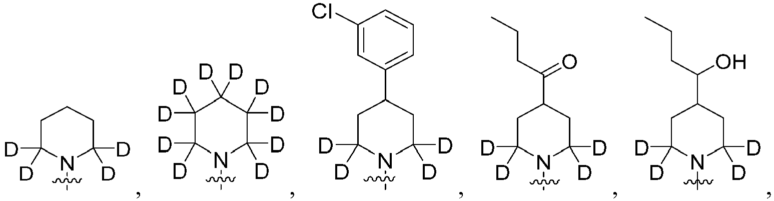 and

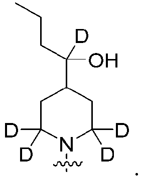

In a specific embodiment, the present disclosure provides compounds having structural formulas selected from the following structural formulas, or pharmaceutically acceptable salts, stereoisomers, tautomers, N-oxides, hydrates, solvates, or prodrugs thereof:

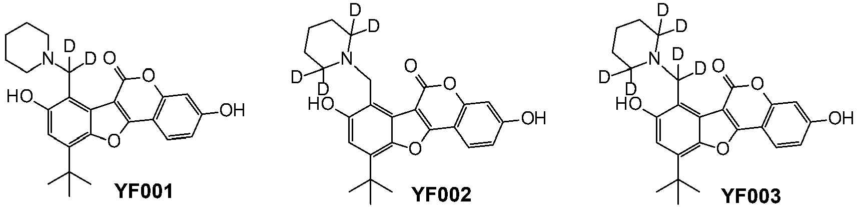

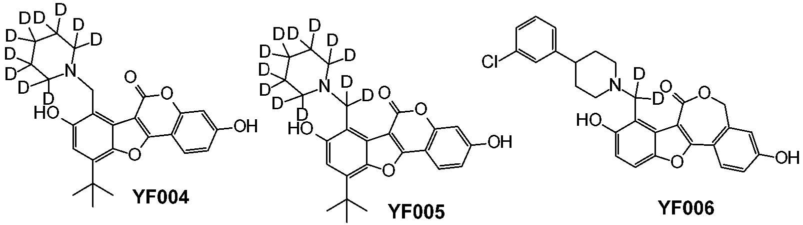

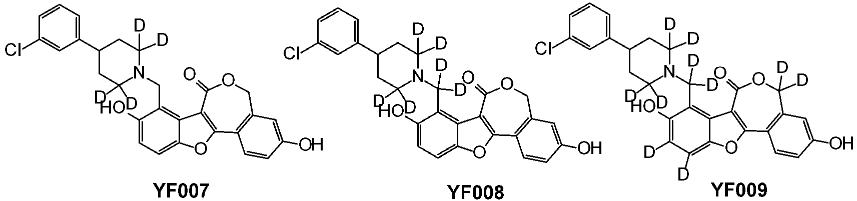

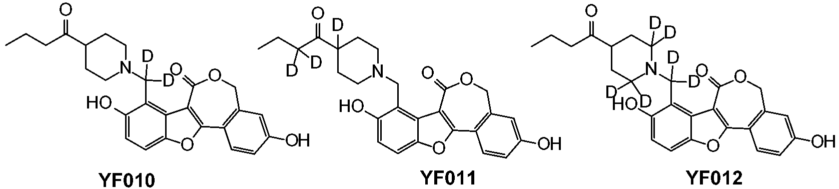

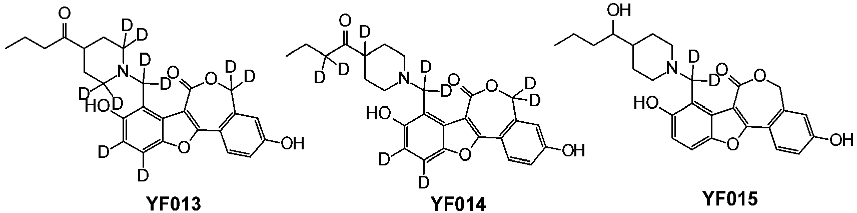

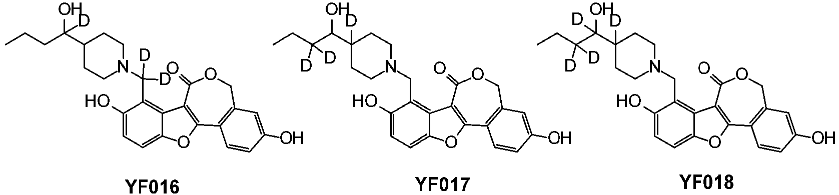

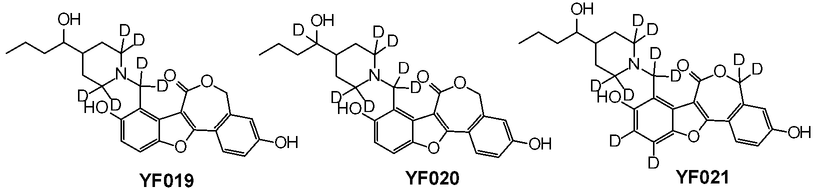

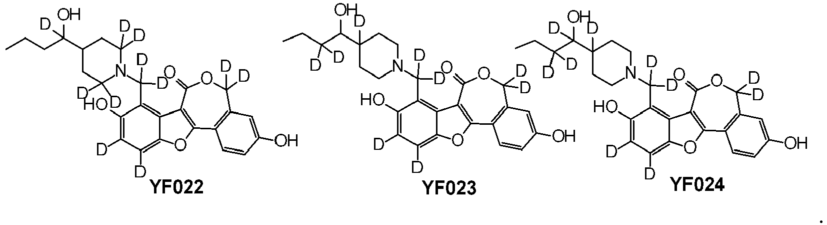

In a specific embodiment, the present disclosure provides hydrochlorides of the compounds described above:

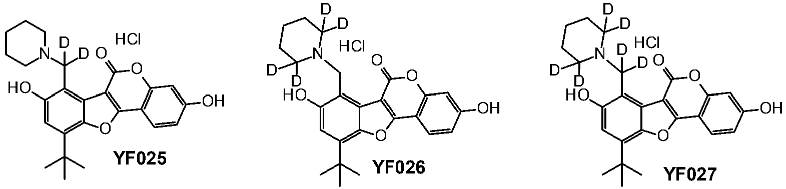

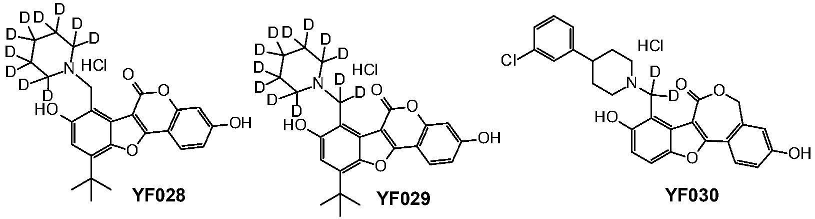

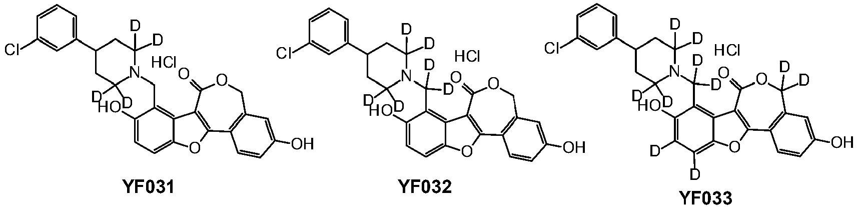

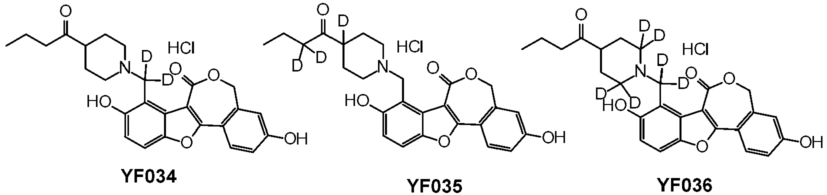

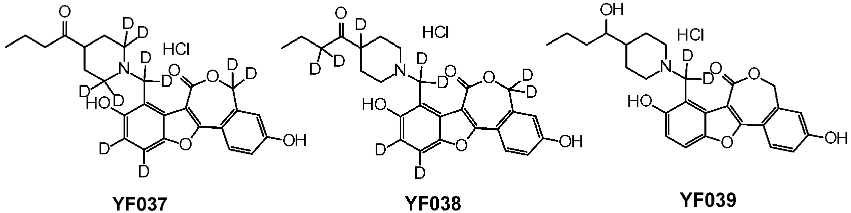

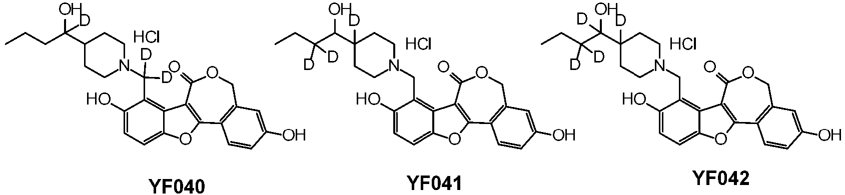

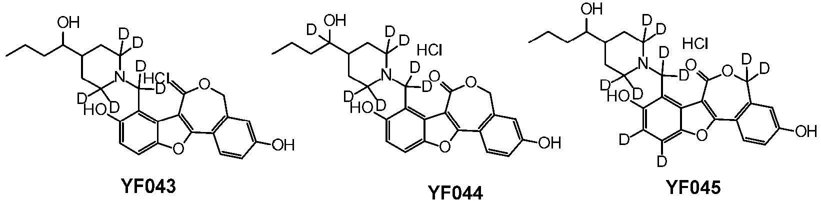

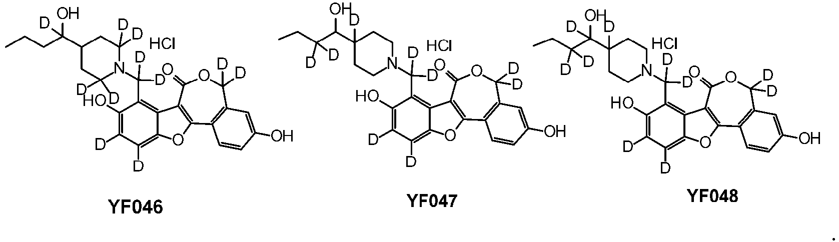

In another aspect, the present disclosure provides a pharmaceutical composition comprising a prophylactically or therapeutically effective amount of any one of the compounds, or the pharmaceutically acceptable salt, the stereoisomer, the tautomer, the N-oxide, the hydrate, the solvate, or the prodrug thereof provided by the present disclosure as an active ingredient, and a pharmaceutically acceptable carrier or excipient.

In one aspect, the present disclosure provides use of any one of the compounds, or the pharmaceutically acceptable salt, the stereoisomer, the tautomer, the N-oxide, the hydrate, the solvate, or the prodrug thereof provided by the present disclosure, or the pharmaceutical composition described above in the preparation of a medicament for preventing or treating a disease caused by bacterial infection.

In a preferred embodiment, the bacterium is selected from: *Mycobacterium tuberculosis,* drug-resistant *Mycobacterium tuberculosis, Mycobacterium smegmatis, Klebsiella pneumoniae, Staphylococcus aureus, Pseudomonas aeruginosa, Acinetobacter baumannii, Mycobacterium leprae, Mycobacterium bovis, Mycobacterium marinum, Corynebacterium diphtheriae, Bordetella pertussis, Haemophilus influenzae,* and *Streptococcus pneumoniae.* More preferably, the bacterium is *Mycobacterium tuberculosis* or drug-resistant *Mycobacterium tuberculosis.* Preferably, the disease is a pulmonary infectious disease, more preferably an infectious disease caused by pulmonary tubercle bacillus, and more preferably tuberculosis.

Other aspects and advantages of the present disclosure will be apparent from the following description of specific embodiments.

### BRIEF DESCRIPTION OF THE DRAWINGS

FIG. 1 is a schematic flow chart of step a to step g for synthesizing the compound or the intermediate thereof of the present disclosure according to some embodiments of the present disclosure.
FIG. 2 shows changes in pulmonary bacterial load in mice with acute infection of *Mycobacterium tuberculosis* after 4 weeks of treatment with compounds according to some embodiments of the present disclosure.

### DETAILED DESCRIPTION

### Definitions

In the present disclosure, "hydrogen" and "H" are used interchangeably in the present disclosure and refer to an atom having 1 proton in its nucleus, which has three isotopes, i.e., protium (P), deuterium (D), and tritium (T). "Protium" is one of the isotopes of hydrogen and denoted by the symbol P or ¹H. A protium atom consists of one proton and one electron, and it is the main form of hydrogen and accounts for about 99.98% of common hydrogen. "Deuterium", also known as heavy hydrogen, is another stable isotope of hydrogen and denoted by the element symbol of D or ²H. "Deuterium" has 1 proton and 1 neutron in its nucleus, with an abundance of 0.016%. "Tritium", denoted by the elemental symbol of T or ³H, is also known as superheavy hydrogen. "Tritium" has one proton and two neutrons in its nucleus. "Tritium" occurs in nature in an extremely small amount, accounting for only 0.004%. In the present disclosure, if a certain "hydrogen" atom is not specifically indicated to be in its specific isotopic form (e.g., "deuterium"), the "hydrogen" atom includes all isotopic forms thereof in its natural state.

"Deuteration" or "deuterated variant" described herein means that one or more hydrogens in the compound or the group thereof are ²H (deuterium, D). "Deuteration" may be mono-substituted, di-substituted, multi-substituted, or fully substituted.

"Heteroatom" described herein includes oxygen (O), sulfur (S), and nitrogen (N).

As used herein, "alkyl" refers to a linear or branched saturated hydrocarbon group, which may have 1-10 carbon atoms, preferably 1-6 carbon atoms. In certain embodiments, the alkyl is C₁-C₄ alkyl. In some embodiments, the alkyl is C₁-C₃ alkyl. Exemplary alkyl includes methyl, ethyl, propyl, isopropyl, butyl, sec-butyl, tert-butyl, pentyl, hexyl, and octyl.

As used herein, "alkenyl" refers to a linear or branched unsaturated hydrocarbon group having 2-10 carbon atoms and containing at least one carbon-carbon double bond in the chain. In some embodiments, the alkenyl is C₂-C₈ alkenyl. Exemplary alkenyl includes ethenyl, 1-propenyl, 2-propenyl, 2-methyl-1-propenyl, 1-butenyl, and 2-butenyl.

As used herein, "alkynyl" refers to a linear or branched unsaturated hydrocarbon group having 2-10 carbon atoms and containing at least one carbon-carbon triple bond in the chain. In some embodiments, the alkynyl is C₂-C₆ alkynyl. Exemplary alkynyl includes ethynyl, 1-propynyl, 1-methyl-2-propynyl, 2-propynyl, 1-butynyl, and 2-butynyl.

As used herein, "alkoxy" refers to an oxy group substituted with C₁-C₁₀ alkyl described above, preferably C₁-C₆ alkyl, or C₁-C₄ alkyl, or C₁-C₃ alkyl, e.g., methoxy or ethoxy.

As used herein, "aryl" is a monocyclic, bicyclic, or tricyclic aromatic group having 6 to 14 carbon atoms. In some embodiments, the aryl is C₆-C₁₀ aryl. Exemplary aryl includes phenyl, naphthyl, phenanthrenyl, anthracenyl, indenyl, azulenyl, biphenyl, biphenylene, and fluorenyl.

As used herein, "carbocyclyl" includes saturated and partially saturated carbocyclic groups. The carbocyclyl has 3-15 ring carbon atoms. Saturated carbocyclyl includes cycloalkyl, which is typically C₃-C₈ cycloalkyl, such as cyclopropyl, cyclobutyl, cyclopentyl, cyclohexyl, and cycloheptyl. Partially saturated carbocyclic group includes cycloalkenyl, such as C₃-C₈ cycloalkenyl, e.g., cyclopentenyl, cycloheptenyl, and cyclooctenyl. Herein, the carbocyclyl also includes bridged ring groups and spiro ring groups. Preferably, the bridged ring groups and spiro ring groups herein may have 4-12 ring carbon atoms.

Herein, the halogen includes fluorine, chlorine, bromine, and iodine.

Herein, the amido group is any C₁-C₁₀ acyl attached to the amino nitrogen and may be represented as R-NH-, wherein R is C₁-C₁₀ acyl. Exemplary amido groups include acetamido, propionamido, butyramido, pentanamido, and hexanamido.

Herein, the acyl may be represented as R-C(O)-, wherein R is H or alkyl as described herein. Exemplary acyl is C₁-C₁₀ acyl, such as acetyl.

Herein, the ester group may be represented as R-C(O)-O- or R-O-C(O)-, wherein R is the alkyl described herein, or R is a moiety other than the carboxyl in the amino acid molecule (i.e., the ester group is a monovalent group obtained by removing H from the carboxyl of the amino acid molecule).

As used herein, "heterocyclyl" refers to saturated or partially saturated 3- to 7-membered monocyclic groups, 7- to 10-membered bicyclic groups, spirocyclic groups, or bridged cyclic groups consisting of carbon atoms and 1-4 heteroatoms selected from O, N, and S. The spirocyclic group (also referred to herein as "spiro heterocyclyl") or bridged cyclic group (also referred to herein as "bridged heterocyclyl") may typically have 4-12 ring atoms. Exemplary heterocyclyl includes tetrahydrofuranyl, pyranyl, piperidinyl, piperazinyl, 1,4-diazepanyl, azetidinyl, oxetanyl, pyrrolidinyl, imidazolidinyl, imidazolinyl, indolinyl, isoindolinyl, quinuclidinyl, morpholinyl, isochromanyl, chromanyl, pyrazolidinyl, pyrazolinyl, tetrahydroisoquinolinyl, tetronoyl, and tetramoyl.

As used herein, "heteroaryl" refers to groups having 5-14, preferably 5-10 ring atoms with 6, 10, or 14 π electrons shared in the ring system. The ring atoms contained in heteroaryl are carbon atoms and 1-3 heteroatoms selected from oxygen, nitrogen, and sulfur. Exemplary heteroaryl includes thienyl, benzo[d]isothiazol-3-yl, benzo[b]thienyl, naphtho[2,3-b]thienyl, thianthrenyl, furyl, pyranyl, isobenzofuranyl, chromenyl, xanthenyl, thienooxazolyl, pyrrolyl, imidazolyl, pyrazolyl, pyridyl (including but not limited to 2-pyridyl, 3-pyridyl, and 4-pyridyl), pyrazinyl, pyrimidinyl, pyridazinyl, indolizinyl, isoindolyl, 3H-indolyl, indolyl, indazolyl, purinyl, 4H-quinolizinyl, isoquinolyl, quinolyl, phthalazinyl, naphthyridinyl, quinazolinyl, cinnolinyl, pteridinyl, carbazolyl, β-carbolinyl, phenanthridinyl, acridinyl, perimidinyl, phenanthrolinyl, phenazinyl, isothiazolyl, phenothiazinyl, isoxazolyl, furazanyl, phenoxazinyl, tetrahydropyridopyrimidinyl, tetrahydro-five-membered[c]pyrazol-3-yl, benzisoxazolyl such as 1,2-benzisoxazol-3-yl, benzimidazolyl, 2-hydroxyindolyl, thiadiazolyl, 2-oxobenzimidazolyl, imidazopyridazinyl, imidazopyridinyl, triazolopyridazinyl, tetrahydropyridopyrimidinyl, pyrazolopyrimidinyl, pyrrolopyrimidinyl, pyrrolopyridinyl, pyrrolopyrazinyl, triazolopyrazinyl, thienoquinolyl, furoquinolyl, thiazoloquinolyl, pyrazoloquinolyl, pyrroloquinolyl, imidazoquinolyl, oxazoloquinolyl, and the like.

Herein, unless otherwise stated, when substituted, the alkyl, carbocyclyl, alkoxy, alkenyl, alkynyl, heterocyclyl, aryl, or heteroaryl described in any one of the embodiments herein may be substituted with one or more (e.g., 1, 2, 3, or 4) substituents selected from the following groups: halogen, hydroxyl, sulfhydryl, carboxyl, amino, nitro, cyano, C₁-C₆ amido, C₁-C₆ acyloxy, C₁-C₆ alkoxy, aryloxy, alkylthio, C₁-C₆ alkyl, C₁-C₁₀ acyl, C₆-C₁₄ aryl, C₃-C₈ cycloalkyl, C₂-C₆ alkenyl, C₃-C₈ cycloalkenyl, C₂-C₆ alkynyl, heterocyclyl, heteroaryl, and the like. Among these substituents, amino, C₁-C₆ amido, C₁-C₆ acyloxy, C₁-C₆ alkoxy, aryloxy, alkylthio, C₁-C₆ alkyl, C₁-C₁₀ acyl, C₆-C₁₄ aryl, C₃-C₈ cycloalkyl, C₂-C₆ alkenyl, C₃-C₈ cycloalkenyl, C₂-C₆ alkynyl, heterocyclyl, or heteroaryl itself is optionally substituted; for example, each may optionally be substituted with 1, 2, 3, or 4 substituents selected from halogen, hydroxy, sulfhydryl, carboxyl, amino, nitro, cyano, C₁-C₆ amido, C₁-C₆ acyloxy, C₁-C₆ alkoxy, aryloxy, alkylthio, C₁-C₆ alkyl, C₁-C₆ acyl, C₆-C₁₄ aryl, C₃-C₈ cycloalkyl, C₂-C₆ alkenyl, C₂-C₆ alkynyl, heterocyclyl, and heteroaryl.

It should be understood that in the embodiments herein, when the substituent is carbocyclyl, heterocyclyl, aryl, or heteroaryl, the number of the carbocyclyl, heterocyclyl, aryl, or heteroaryl substituents is typically 1 or 2.

The term "arylalkyl", "heteroarylalkyl", or "heterocyclylalkyl" refers to alkyl substituted with aryl, heteroaryl, or heterocyclyl, wherein aryl, heteroaryl, heterocyclyl, and alkyl each have the definitions described above. In the present disclosure, the aryl, heteroaryl, heterocyclyl, and/or alkyl may have a specified number of carbon atoms. For example, "C₆-C₁₄ aryl C₁-C₁₀ alkyl" as an example of "arylalkyl" refers to C₁-C₁₀ alkyl substituted with C₆-C₁₄ aryl. In the present disclosure, the term "comprise" means that the pharmaceutical composition may also contain any other components, which can be present in any amount as long as the component present in the amount is acceptable in the human body and does not have an unacceptable effect on the activity of the active ingredient in the pharmaceutical composition of the present disclosure.

"Pharmaceutically acceptable salt" includes pharmaceutically acceptable acid addition salts and pharmaceutically acceptable base addition salts. "Pharmaceutically acceptable acid addition salt" refers to salts that are capable of retaining the biological effectiveness of free bases without having any undesirable effects and that are formed with inorganic or organic acids. Inorganic acid salts include, but are not limited to, hydrochlorides, hydrobromides, sulfates, nitrates, phosphates, and the like; organic acid salts include, but are not limited to, formates, acetates, 2,2-dichloroacetates, trifluoroacetates, propionates, caproates, caprylates, caprates, undecenates, glycolates, gluconates, lactates, sebacates, adipates, glutarates, malonates, oxalates, maleates, succinates, fumarates, tartrates, citrates, palmitates, stearates, oleates, cinnamates, laurates, malates, glutamates, pyroglutamates, aspartates, benzoates, mesylates, benzenesulfonates, p-toluenesulfonates, alginates, ascorbates, salicylates, 4-aminosalicylates, napadisylates, and the like. "Pharmaceutically acceptable base addition salt" refers to salts that are capable of retaining the biological effectiveness of free acids without having any undesirable effects and that are formed with inorganic bases or organic bases. Salts derived from inorganic bases include, but are not limited to, sodium salts, potassium salts, lithium salts, ammonium salts, calcium salts, magnesium salts, iron salts, zinc salts, copper salts, manganese salts, aluminum salts, and the like. Preferred inorganic salts are ammonium salts, sodium salts, potassium salts, calcium salts, and magnesium salts. Salts derived from organic bases include, but are not limited to, salts of the following: primary, secondary, and tertiary amines, substituted amines including naturally occurring substituted amines, cyclic amines, and basic ion exchange resins, such as ammonia, isopropylamine, trimethylamine, diethylamine, triethylamine, tripropylamine, ethanolamine, diethanolamine, triethanolamine, dimethylethanolamine, 2-dimethylaminoethanol, 2-diethylaminoethanol, dicyclohexylamine, lysine, arginine, histidine, caffeine, procaine, choline, betaine, ethylenediamine, glucosamine, methylglucamine, theobromine, purine, piperazine, piperidine, N-ethylpiperidine, and polyamine resins. Preferred organic bases include isopropylamine, diethylamine, ethanolamine, trimethylamine, dicyclohexylamine, choline, and caffeine. In some embodiments, the pharmaceutically acceptable salt of the compound of formula I of the present disclosure is a hydrochloride or a deuterated hydrochloride of the compound.

The term "stereoisomer" refers to a compound having the same chemical structure, but having a different arrangement of atoms or groups in space. Stereoisomers include enantiomers, diastereomers, conformers (rotamers), geometric isomers (cis/trans isomers), atropisomers, and the like. The term "enantiomer" refers to two isomers of a compound that are non-superposable onto each other but are mirror images of each other. The term "diastereoisomer" refers to stereoisomers with two or more chiral centers and whose molecules are not mirror images of each other. Diastereomers have different physical properties, such as melting points, boiling points, spectral properties, and reactivities. A mixture of diastereomers can be resolved through a high-resolution analytical process such as electrophoresis and chromatography, e.g., HPLC.

The term "tautomer" or "tautomeric form" refers to structural isomers with different energies that can be interconverted through a low energy barrier. If tautomerism is possible (e.g., in a solution), a chemical equilibrium of tautomers can be achieved. For example, proton tautomers (also known as prototropic tautomers) include interconversion via migration of a proton, such as keto-enol isomerization and imine-enamine isomerization. Valence tautomers include interconversions by reorganization of some of the bonding electrons. A specific example of keto-enol tautomerization is the interconversion of pentan-2,4-dione and 4-hydroxypent-3-en-2-one tautomers. Another example of tautomerization is phenol-keto tautomerization. One specific example of phenol-keto tautomerization is the interconversion of pyridin-4-ol and pyridin-4(1H)-one tautomers. Unless otherwise stated, all tautomeric forms of the compound of the present disclosure are within the scope of the present disclosure.

The term "nitrogen oxide" or "N-oxide" refers to an N-oxide formed by oxidizing one or more nitrogen atoms when the compound contains several amine functional groups. Specific examples of N-oxides are N-oxides of tertiary amines or N-oxides of a nitrogen atom of a nitrogen-containing heterocycle. The corresponding amines may be treated with an oxidizing agent such as hydrogen peroxide or a peracid (e.g., a peroxycarboxylic acid) to form N-oxides. In particular, N-oxides may be formed, for example, by reacting an amine compound with m-chloroperbenzoic acid (MCPBA) in an inert solvent such as dichloromethane.

The term "hydrate" refers to an association compound in which the solvent molecules are water molecules.

In the present disclosure, "solvate" refers to an association compound formed by one or more solvent molecules and the compound of the present disclosure. Solvents for forming the solvate include, but are not limited to, isopropanol, ethanol, methanol, dimethyl sulfoxide, ethyl acetate, acetic acid, and the like.

In the present disclosure, the term "prodrug" includes compounds that may themselves be biologically active or inactive and that, when administered by an appropriate method, are metabolized or chemically transformed in the human body into the compounds of Formula I to Formula IV and any subformulas thereof, or salts thereof.

Herein, "pharmaceutically acceptable" refers to substances (e.g., carriers or diluents) that do not substantially affect the biological activity or properties of the compound of the present disclosure and are relatively non-toxic. In the present application, "pharmaceutically acceptable carrier or excipient" includes, but is not limited to, any adjuvant, carrier, excipient, glidant, sweetener, diluent, preservative, dye/colorant, flavoring agent, surfactant, wetting agent, dispersant, suspending agent, stabilizer, isotonic agent, solvent, or emulsifier that is approved by the relevant governmental regulatory agency for human or livestock use.

Herein, "prevention" includes reducing the possibility of the occurrence or exacerbation of a disease or disorder in a patient.

Herein, "treat", "treating", or "treatment" includes the following meanings: inhibiting a disease or disorder, i.e., arresting its development; alleviating a disease or disorder, i.e., causing regression of the disease or disorder; and mitigating symptoms caused by the disease or disorder.

Herein, "effective amount" refers to an amount of at least one agent or compound that, after administration, is sufficient to alleviate, to some extent, one or more of the symptoms of the disease or disorder being treated. The result may be a reduction and/or alleviation of symptoms or causes, or any other desired change in a biological system. For example, an "effective amount" for treatment is an amount of a composition comprising the compounds disclosed herein required to provide a clinically significant effect in alleviating the disease or disorder. Techniques such as dose-escalation studies can be used to determine an effective amount appropriate for any individual case.

### Deuterated Tetracyclic Derivative

A first aspect of the present disclosure provides a deuterated tetracyclic derivative, or a pharmaceutically acceptable salt, a stereoisomer, a tautomer, an N-oxide, a hydrate, a solvate, or a prodrug thereof.

In some embodiments, the present disclosure provides a compound represented by formula I, or a pharmaceutically acceptable salt, a stereoisomer, a tautomer, an N-oxide, a hydrate, a solvate, or a prodrug thereof, 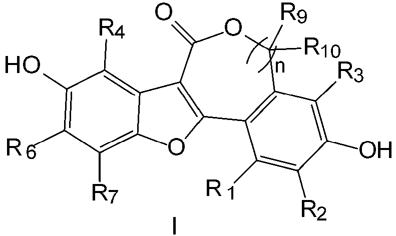
wherein n is 0 or 1;
R₄ is selected from H, halogen, an aldehyde group, hydroxy, sulfhydryl, - NR'R", cyano, nitro, optionally substituted C₁-C₁₀ alkyl, optionally substituted C₂-C₁₀ alkenyl, optionally substituted C₂-C₁₀ alkynyl, optionally substituted C₃-C₈ carbocyclyl, optionally substituted C₆-C₁₄ aryl, optionally substituted C₅-C₁₀ heteroaryl, optionally substituted C₄-C₁₀ heterocyclyl, and respective deuterated variants thereof; preferably, R₄ is selected from optionally substituted C₆-C₁₄ aryl C₁-C₁₀ alkyl, optionally substituted C₅-C₁₀ heteroaryl C₁-C₁₀ alkyl, optionally substituted C₄-C₁₀ heterocyclyl C₁-C₁₀ alkyl, and respective deuterated variants thereof;
R₇ is selected from H, hydroxy, sulfhydryl, cyano, carboxyl, nitro, -NR'R", optionally substituted C₁-C₁₀ alkyl, optionally substituted C₂-C₁₀ alkenyl, optionally substituted C₂-C₁₀ alkynyl, C₁-C₁₀ ester group, amido, halogen, optionally substituted C₃-C₈ carbocyclyl, optionally substituted C₁-C₁₀ alkoxy, optionally substituted C₆-C₁₄ aryl, optionally substituted C₅-C₁₀ heteroaryl, optionally substituted C₄-C₁₀ heterocyclyl, and respective deuterated variants thereof,
wherein R' and R" are each independently selected from: H, optionally substituted C₁-C₁₀ alkyl, optionally substituted C₂-C₈ alkenyl, and optionally substituted C₁-C₁₀ alkoxy;
R₁, R₂, R₃, R₆, R₉, and R₁₀ are independently selected from hydrogen and deuterium; and
the structure represented by formula I comprises at least one deuterium atom.

In some embodiments, R₄ is preferably optionally substituted C₆-C₁₄ aryl C₁-C₁₀ alkyl, optionally substituted C₅-C₁₀ heteroaryl C₁-C₁₀ alkyl, optionally substituted C₄-C₁₀ heterocyclyl C₁-C₁₀ alkyl, or respective deuterated variants thereof. More preferably, R₄ is optionally substituted C₄-C₈ heterocyclyl C₁-C₃ alkyl or a deuterated variant thereof. More preferably, R₄ is optionally substituted C₆ heterocyclyl C₁-C₃ alkyl or a deuterated variant thereof.

In some embodiments, R₄ is -(CR₁₁R₁₂)ₘ₋NRₐR_{b}, wherein:
m is 1, 2, or 3;
R₁₁ and R₁₂ are independently selected from hydrogen and deuterium; and
Rₐ and R_{b} are each independently selected from the following substituents and respective deuterated variants thereof: H; C₁-C₁₀ alkyl optionally substituted with 1-5 substituents selected from halogen and hydroxy; C₂-C₈ alkenyl optionally substituted with 1-5 substituents selected from halogen and hydroxy; 3- to 14-membered carbocyclyl optionally substituted with 1-5 substituents selected from halogen and C₁-C₄ alkyl; C₁-C₆ alkyl substituted with 3- to 14-membered carbocyclyl optionally substituted with 1-5 substituents selected from halogen and C₁-C₄ alkyl; C₁-C₆ alkyl substituted with 4- to 10-membered heterocyclyl optionally substituted with 1-5 substituents selected from halogen, hydroxy, and C₁-C₄ alkyl; C₆-C₁₄ aryl optionally substituted with 1-5 substituents selected from halogen, hydroxy, and C₁-C₄ alkyl; and C₆-C₁₄ aryl-C₁-C₆ alkyl optionally substituted with 1-5 substituents selected from halogen, hydroxy, and C₁-C₄ alkyl; or Rₐ and R_{b}, together with the nitrogen atom to which they are attached, form an optionally substituted 4- to 7-membered heterocyclyl or benzo 4- to 7-membered heterocyclyl or a deuterated variant thereof, wherein the 4-to 7-membered heterocyclyl or benzo 4- to 7-membered heterocyclyl is optionally substituted with 1-5 substituents selected from: C₁-C₆ alkyl optionally substituted with 1-5 substituents selected from halogen and hydroxy; C₁-C₆ alkoxy optionally substituted with 1-5 substituents selected from halogen and hydroxy; cyano; carboxyl; halogen; -NR^{c}R^{d}; hydroxy; C₆-C₁₄ aryl optionally substituted with 1-5 substituents selected from C₁-C₄ acyl, halogen, NR^{c}R^{d}, C₁-C₄ alkoxy, C₁-C₄ alkyl, cyano, hydroxy, C₁-C₄ alkoxycarbonyl, C₆-C₁₄ aryl, and halogenated C₁-C₄ alkyl; C₁-C₆ acyl; C₁-C₆ alkoxycarbonyl; C₂-C₈ alkenyloxycarbonyl; C₁-C₆ acyl substituted with NR^{c}R^{d}; C₁-C₄ acyl substituted with C₆-C₁₄ aryl optionally substituted with 1-5 substituents selected from halogen, C₁-C₄ alkyl, halogenated C₁-C₄ alkyl, and hydroxy; C₁-C₆ alkyl substituted with C₆-C₁₄ aryl optionally substituted with 1-5 substituents selected from halogen, C₁-C₄ alkyl, halogenated C₁-C₄ alkyl, and hydroxy; C₁-C₆ alkyl substituted with C₃-C₈ cycloalkyl optionally substituted with 1-5 substituents selected from halogen, C₁-C₄ alkyl, halogenated C₁-C₄ alkyl, and hydroxy; C₃-C₈ cycloalkyl optionally substituted with 1-5 substituents selected from halogen, C₁-C₄ alkyl, halogenated C₁-C₄ alkyl, and hydroxy; 4- to 7-membered heterocyclyl optionally substituted with 1-5 substituents selected from halogen, C₁-C₄ alkyl, halogenated C₁-C₄ alkyl, and hydroxy; C₁-C₆ alkyl substituted with 4- to 7-membered heterocyclyl optionally substituted with 1-5 substituents selected from halogen, C₁-C₄ alkyl, halogenated C₁-C₄ alkyl, and hydroxy; 5- to 10-membered heteroaryl optionally substituted with 1-5 substituents selected from halogen, C₁-C₄ alkyl, halogenated C₁-C₄ alkyl, and hydroxy; and C₁-C₆ alkyl substituted with 5- to 10-membered heteroaryl optionally substituted with 1-5 substituents selected from halogen, C₁-C₄ alkyl, halogenated C₁-C₄ alkyl, and hydroxy, wherein R^{c} and R^{d} are each independently selected from H, C₁-C₆ alkyl, C₂-C₈ alkenyl, and C₁-C₆ alkoxy.

In a preferred embodiment, R₄ is -(CR₁₁R₁₂)ₘ-NRₐR_{b}, wherein:
m is 1, 2, or 3;
R₁₁ and R₁₂ are independently selected from hydrogen and deuterium; and
Rₐ and R_{b}, together with the nitrogen atom to which they are attached, form optionally substituted 4- to 7-membered heterocyclyl, benzo 4- to 7-membered heterocyclyl, or a deuterated variant thereof, wherein the 4- to 7-membered heterocyclyl or benzo 4- to 7-membered heterocyclyl is optionally substituted with 1-5 substituents selected from: C₁-C₆ alkyl optionally substituted with 1-5 substituents selected from halogen and hydroxy; C₁-C₆ alkoxy optionally substituted with 1-5 substituents selected from halogen and hydroxy; cyano; carboxyl; halogen; -NR^{c}R^{d}; hydroxy; C₆-C₁₄ aryl optionally substituted with 1-5 substituents selected from C₁-C₄ acyl, halogen, NR^{c}R^{d}, C₁-C₄ alkoxy, C₁-C₄ alkyl, cyano, hydroxy, C₁-C₄ alkoxycarbonyl, C₆-C₁₄ aryl, and halogenated C₁-C₄ alkyl; C₁-C₆ acyl; C₁-C₆ alkoxycarbonyl; C₂-C₈ alkenyloxycarbonyl; C₁-C₆ acyl substituted with NR^{c}R^{d}; C₁-C₄ acyl substituted with C₆-C₁₄ aryl optionally substituted with 1-5 substituents selected from halogen, C₁-C₄ alkyl, halogenated C₁-C₄ alkyl, and hydroxy; C₁-C₆ alkyl substituted with C₆-C₁₄ aryl optionally substituted with 1-5 substituents selected from halogen, C₁-C₄ alkyl, halogenated C₁-C₄ alkyl, and hydroxy; C₁-C₆ alkyl substituted with C₃-C₈ cycloalkyl optionally substituted with 1-5 substituents selected from halogen, C₁-C₄ alkyl, halogenated C₁-C₄ alkyl, and hydroxy; C₃-C₈ cycloalkyl optionally substituted with 1-5 substituents selected from halogen, C₁-C₄ alkyl, halogenated C₁-C₄ alkyl, and hydroxy; 4- to 7-membered heterocyclyl optionally substituted with 1-5 substituents selected from halogen, C₁-C₄ alkyl, halogenated C₁-C₄ alkyl, and hydroxy; C₁-C₆ alkyl substituted with 4- to 7-membered heterocyclyl optionally substituted with 1-5 substituents selected from halogen, C₁-C₄ alkyl, halogenated C₁-C₄ alkyl, and hydroxy; 5- to 10-membered heteroaryl optionally substituted with 1-5 substituents selected from halogen, C₁-C₄ alkyl, halogenated C₁-C₄ alkyl, and hydroxy; and C₁-C₆ alkyl substituted with 5- to 10-membered heteroaryl optionally substituted with 1-5 substituents selected from halogen, C₁-C₄ alkyl, halogenated C₁-C₄ alkyl, and hydroxy, wherein R^{c} and R^{d} are each independently selected from H, C₁-C₆ alkyl, C₂-C₈ alkenyl, and C₁-C₆ alkoxy.

In a preferred embodiment, R₄ is -(CR₁₁R₁₂)ₘ-NRₐR_{b}, wherein:
m is 1, 2, or 3;
R₁₁ and R₁₂ are independently selected from hydrogen and deuterium; and - NRₐR_{b} is selected from: 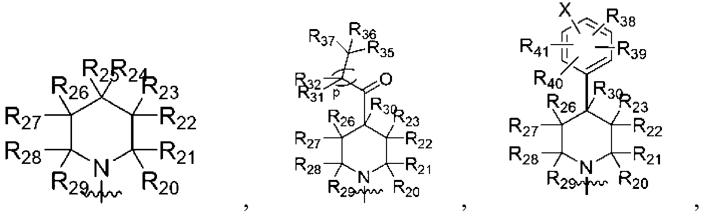 and 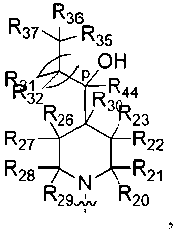 wherein p is independently 0, 1, 2, or 3; X is halogen; R₂₀ to R₃₂, R₃₅ to R₄₁, and R₄₄ are independently selected from: hydrogen and deuterium;
preferably, -NRₐR_{b} is selected from: 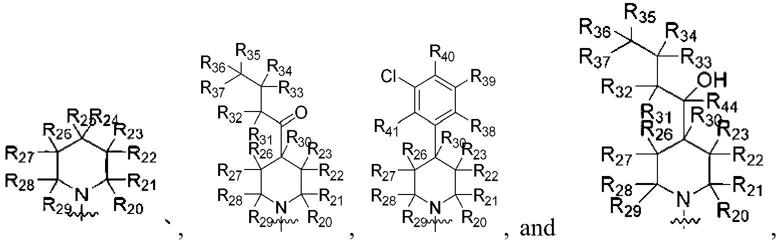 wherein R₂₀ to R₄₁ and R₄₄ are independently selected from hydrogen and deuterium.

In a preferred embodiment, R₄ is -(CR₁₁R₁₂)ₘ-NRₐR_{b}, wherein:
m is 1, 2, or 3;
R₁₁ and R₁₂ are independently selected from hydrogen and deuterium; and - NRₐR_{b} is selected from: 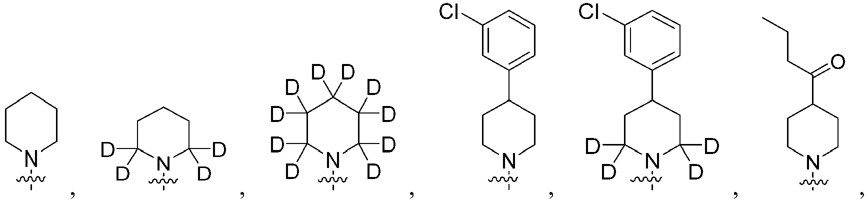 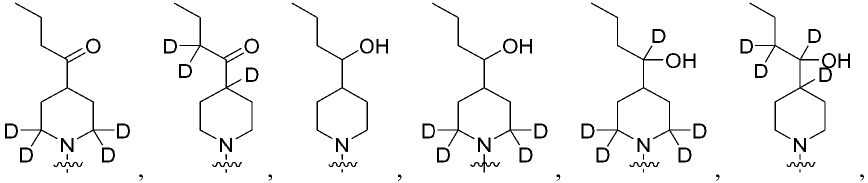 and

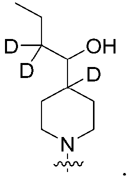

In any one of the above embodiments, R₇ is selected from H, C₁-C₆ alkyl optionally substituted with 1-5 substituents selected from halogen and hydroxy, C₂-C₆ alkenyl optionally substituted with 1-5 substituents selected from halogen and hydroxy, saturated or partially saturated C₃-C₈ carbocyclyl, halogen, hydroxy, C₁-C₄ alkoxy substituted with C₆-C₁₄ aryl, 5- to 10-membered heteroaryl, and C₆-C₁₄ aryl optionally substituted with 1-3 substituents selected from C₁-C₄ alkyl, halogenated C₁-C₄ alkyl, halogen, C₂-C₄ alkenyl, and hydroxy, as well as respective deuterated variants thereof.

Preferably, R₇ is selected from hydrogen, deuterium, and 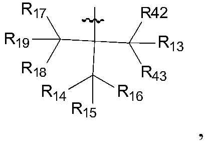 wherein R₁₃ to R₁₉, R₄₂, and R₄₃ are independently selected from hydrogen and deuterium.

In a preferred embodiment, R7 is selected from hydrogen, deuterium, and

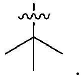

In a preferred embodiment, the compound represented by formula I comprises 2 to 20 deuterium atoms, e.g., 2, 3, 4, 5, 6, 7, 8, 9, 10, 11, 12, 13, 14, 15, 16, 17, 18, 19, or 20 deuterium atoms. In some embodiments, the compound represented by formula I comprises 2 to 20, 2 to 18, 2 to 16, 2 to 14, 2 to 12, 2 to 10, 2 to 8, 2 to 6, 2 to 4, 4 to 20, 6 to 20, 8 to 20, 10 to 20, 12 to 20, 14 to 20, 16 to 20, 18 to 20, 4 to 18, 6 to 16, 8 to 14, or 10 to 12 deuterium atoms.

In some embodiments, the present disclosure provides a compound represented by formula II, or a pharmaceutically acceptable salt, a stereoisomer, a tautomer, an N-oxide, a hydrate, a solvate, or a prodrug thereof, 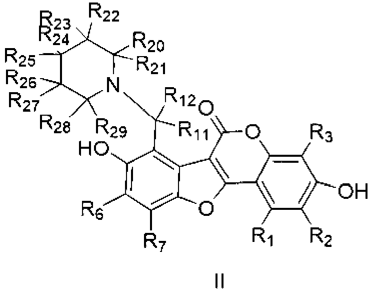 wherein,
R₇ is selected from hydrogen, deuterium, and 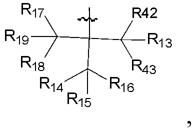 wherein R₁₃ to R₁₉, R₄₂, and R₄₃ are independently selected from hydrogen and deuterium.
R₁, R₂, R₃, R₆, R₁₁, R₁₂, and R₂₀ to R₂₉ are independently selected from hydrogen and deuterium; and
the structure represented by formula II comprises at least one deuterium atom.

In a preferred embodiment, R₇ is

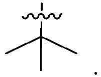

In a preferred embodiment, the compound represented by formula II comprises 2 to 20 deuterium atoms, e.g., 2, 3, 4, 5, 6, 7, 8, 9, 10, 11, 12, 13, 14, 15, 16, 17, 18, 19, or 20 deuterium atoms. In some embodiments, the compound represented by formula II comprises 2 to 20, 2 to 18, 2 to 16, 2 to 14, 2 to 12, 2 to 10, 2 to 8, 2 to 6, 2 to 4, 4 to 20, 6 to 20, 8 to 20, 10 to 20, 12 to 20, 14 to 20, 16 to 20, 18 to 20, 4 to 18, 6 to 16, 8 to 14, or 10 to 12 deuterium atoms.

In some embodiments, the present disclosure provides a compound having a structural formula shown as IIa below, or a pharmaceutically acceptable salt, a stereoisomer, a tautomer, an N-oxide, a hydrate, a solvate, or a prodrug thereof, wherein R₁, R₂, R₃, R₆, R₁₁, and R₁₂ are independently selected from hydrogen and deuterium, R₇ has any one of the definitions described above, and the structure represented by IIa comprises at least one deuterium atom:

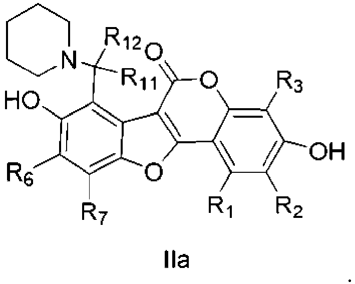

In some embodiments, the present disclosure provides a compound having a structural formula shown as IIb below, or a pharmaceutically acceptable salt, a stereoisomer, a tautomer, an N-oxide, a hydrate, a solvate, or a prodrug thereof, wherein R₁, R₂, R₃, R₆, R₁₁, and R₁₂ are independently selected from hydrogen and deuterium, R₇ has any one of the definitions described above, and the structure represented by IIb comprises at least one deuterium atom:

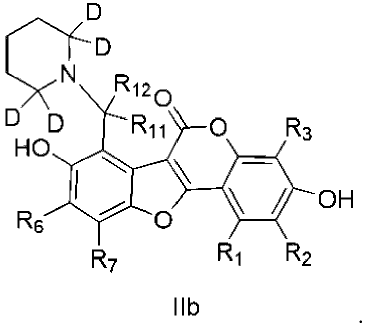

In some embodiments, the present disclosure provides a compound having a structural formula shown as IIc below, or a pharmaceutically acceptable salt, a stereoisomer, a tautomer, an N-oxide, a hydrate, a solvate, or a prodrug thereof, wherein R₁, R₂, R₃, R₆, R₁₁, and R₁₂ are independently selected from hydrogen and deuterium, R₇ has any one of the definitions described above, and the structure represented by IIc comprises at least one deuterium atom:

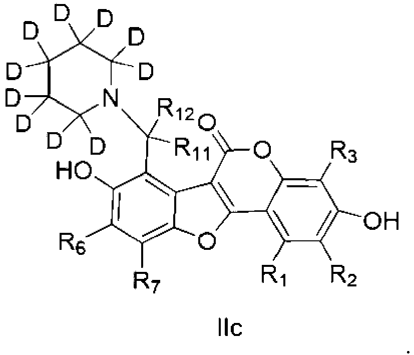

In some embodiments, the present disclosure provides a compound having a structural formula shown as IId below, or a pharmaceutically acceptable salt, a stereoisomer, a tautomer, an N-oxide, a hydrate, a solvate, or a prodrug thereof, wherein R₁, R₂, R₃, R₆, and R₂₂ to R₂₇ are independently selected from hydrogen and deuterium, R₇ has any one of the definitions described above, and the structure represented by IId comprises at least one deuterium atom:

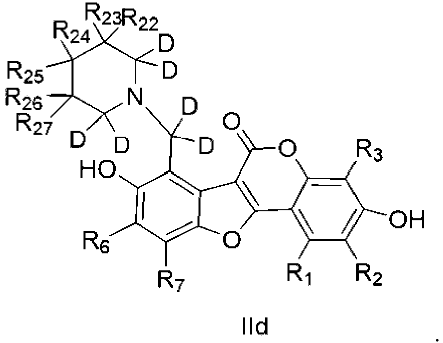

In some embodiments, the present disclosure provides a compound represented by formula III, or a pharmaceutically acceptable salt, a stereoisomer, a tautomer, an N-oxide, a hydrate, a solvate, or a prodrug thereof, 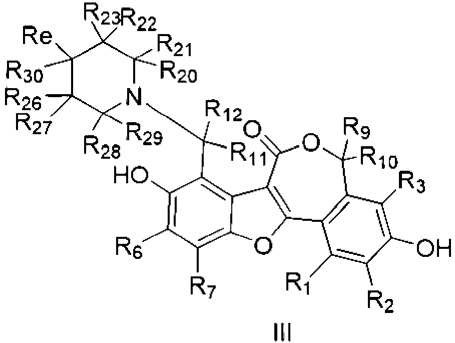
wherein Re is selected from: C₁-C₆ alkyl optionally substituted with 1-5 substituents selected from halogen and hydroxy; C₁-C₆ alkoxy optionally substituted with 1-5 substituents selected from halogen and hydroxy; cyano; carboxyl; halogen; - NR^{c}R^{d}; hydroxy; C₆-C₁₄ aryl optionally substituted with 1-5 substituents selected from C₁-C₄ acyl, halogen, NR^{c}R^{d}, C₁-C₄ alkoxy, C₁-C₄ alkyl, cyano, hydroxy, C₁-C₄ alkoxycarbonyl, C₆-C₁₄ aryl, and halogenated C₁-C₄ alkyl; C₁-C₆ acyl; C₁-C₆ alkoxycarbonyl; C₂-C₈ alkenyloxycarbonyl; C₁-C₆ acyl substituted with NR^{c}R^{d}; C₁-C₄ acyl substituted with C₆-C₁₄ aryl optionally substituted with 1-5 substituents selected from halogen, C₁-C₄ alkyl, halogenated C₁-C₄ alkyl, and hydroxy; C₁-C₆ alkyl substituted with C₆-C₁₄ aryl optionally substituted with 1-5 substituents selected from halogen, C₁-C₄ alkyl, halogenated C₁-C₄ alkyl, and hydroxy; C₁-C₆ alkyl substituted with C₃-C₈ cycloalkyl optionally substituted with 1-5 substituents selected from halogen, C₁-C₄ alkyl, halogenated C₁-C₄ alkyl, and hydroxy; C₃-C₈ cycloalkyl optionally substituted with 1-5 substituents selected from halogen, C₁-C₄ alkyl, halogenated C₁-C₄ alkyl, and hydroxy; 4- to 7-membered heterocyclyl optionally substituted with 1-5 substituents selected from halogen, C₁-C₄ alkyl, halogenated C₁-C₄ alkyl, and hydroxy; C₁-C₆ alkyl substituted with 4- to 7-membered heterocyclyl optionally substituted with 1-5 substituents selected from halogen, C₁-C₄ alkyl, halogenated C₁-C₄ alkyl, and hydroxy; 5- to 10-membered heteroaryl optionally substituted with 1-5 substituents selected from halogen, C₁-C₄ alkyl, halogenated C₁-C₄ alkyl, and hydroxy; and C₁-C₆ alkyl substituted with 5- to 10-membered heteroaryl optionally substituted with 1-5 substituents selected from halogen, C₁-C₄ alkyl, halogenated C₁-C₄ alkyl, and hydroxy; and respective deuterated variants thereof, wherein R^{c} and R^{d} are each independently selected from H, C₁-C₆ alkyl, C₂-C₈ alkenyl, and C₁-C₆ alkoxy;
R₇ is selected from H, C₁-C₆ alkyl optionally substituted with 1-5 substituents selected from halogen and hydroxy, C₂-C₆ alkenyl optionally substituted with 1-5 substituents selected from halogen and hydroxy, saturated or partially saturated C₃-C₈ carbocyclyl, halogen, hydroxy, C₁-C₄ alkoxy substituted with C₆-C₁₄ aryl, 5- to 10-membered heteroaryl, and C₆-C₁₄ aryl optionally substituted with 1-3 substituents selected from C₁-C₄ alkyl, halogenated C₁-C₄ alkyl, halogen, C₂-C₄ alkenyl, and hydroxy, as well as respective deuterated variants thereof;
R₁, R₂, R₃, R₆, R₉ to R₁₂, R₂₀ to R₂₃, and R₂₆ to R₃₀ are each independently selected from hydrogen and deuterium; and
the structure represented by formula III comprises at least one deuterium atom.

In a preferred embodiment, Re, together with the piperidinyl attached thereto, forms a group selected from:

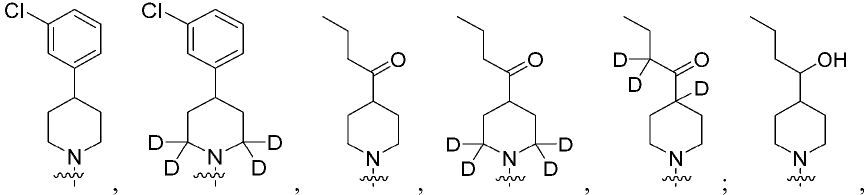

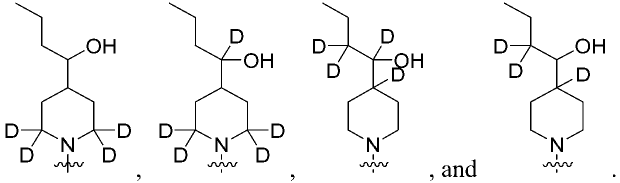

In a preferred embodiment, R₇ is selected from hydrogen, deuterium, and 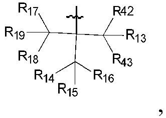 wherein R₁₃ to R₁₉, R₄₂, and R₄₃ are each independently selected from hydrogen and deuterium. In a more preferred embodiment, R₇ is hydrogen and deuterium.

In a preferred embodiment, the compound represented by formula III comprises 2 to 20 deuterium atoms, e.g., 2, 3, 4, 5, 6, 7, 8, 9, 10, 11, 12, 13, 14, 15, 16, 17, 18, 19, or 20 deuterium atoms. In some embodiments, the compound represented by formula III comprises 2 to 20, 2 to 18, 2 to 16, 2 to 14, 2 to 12, 2 to 10, 2 to 8, 2 to 6, 2 to 4, 4 to 20, 6 to 20, 8 to 20, 10 to 20, 12 to 20, 14 to 20, 16 to 20, 18 to 20, 4 to 18, 6 to 16, 8 to 14, or 10 to 12 deuterium atoms.

In a preferred embodiment, the present disclosure provides a compound having a structural formula shown as IIIa below, or a pharmaceutically acceptable salt, a stereoisomer, a tautomer, an N-oxide, a hydrate, a solvate, or a prodrug thereof, wherein R₁, R₂, R₃, R₆, R₉ to R₁₂, R₂₀ to R₂₃, R₂₆ to R₃₀, and R₃₈ to R₄₁ are independently selected from hydrogen and deuterium, X is halogen, R₇ has any one of the definitions described above, and the structure represented by formula IIIa comprises at least one deuterium atom: 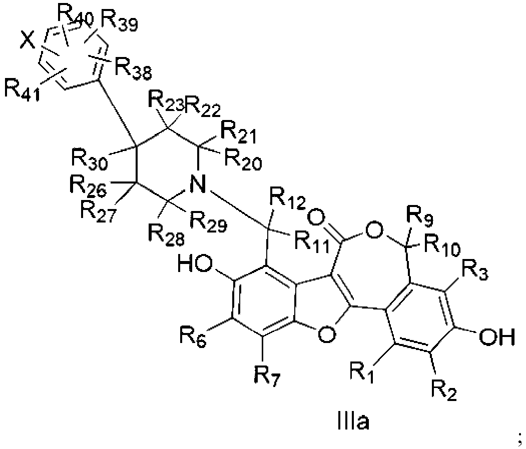 in a more preferred embodiment, R₁, R₂, and R₃ are all hydrogen.

In a preferred embodiment, the present disclosure provides a compound having a structural formula shown as IIIb below, or a pharmaceutically acceptable salt, a stereoisomer, a tautomer, an N-oxide, a hydrate, a solvate, or a prodrug thereof, wherein R₁, R₂, R₃, R₆, R₉ to R₁₂, R₂₀ to R₂₃, R₂₆ to R₃₂, and R₃₅ to R₃₇ are independently selected from hydrogen and deuterium, p is 0, 1, 2, or 3, R₇ has any one of the definitions described above, and the structure represented by formula IIIb comprises at least one deuterium atom: 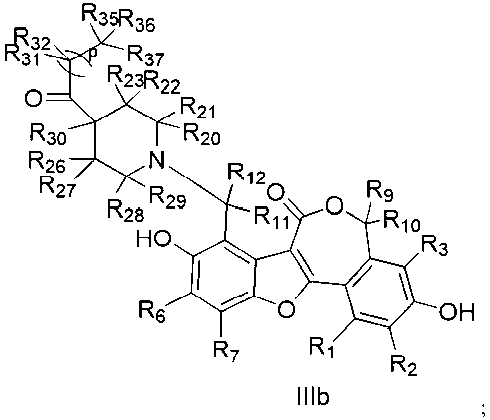 in a more preferred embodiment, R1, R2, and R3 are all hydrogen.

In a preferred embodiment, the present disclosure provides a compound having a structural formula shown as IIIc below, or a pharmaceutically acceptable salt, a stereoisomer, a tautomer, an N-oxide, a hydrate, a solvate, or a prodrug thereof, wherein R₁, R₂, R₃, R₆, R₉ to R₁₂, R₂₀ to R₂₃, R₂₆ to R₃₂, R₃₅ to R₃₇, and R₄₄ are independently selected from hydrogen and deuterium, p is 0, 1, 2, or 3, R₇ has any one of the definitions described above, and the structure represented by formula IIIc comprises at least one deuterium atom: 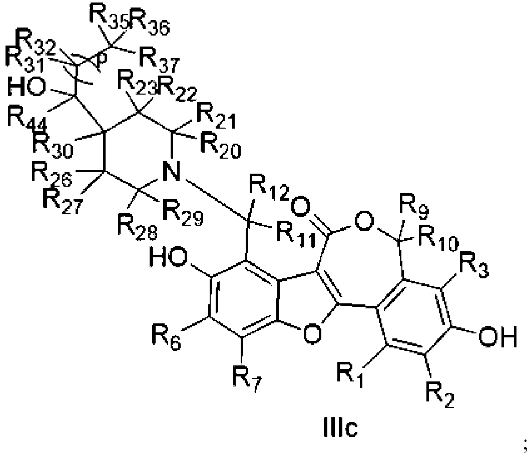 in a more preferred embodiment, R1, R2, and R3 are all hydrogen.

In a further preferred embodiment, the present disclosure provides a compound having a structural formula shown as IIId below, or a pharmaceutically acceptable salt, a stereoisomer, a tautomer, an N-oxide, a hydrate, a solvate, or a prodrug thereof, wherein R₁, R₂, R₃, R₆, R₉, R₁₀, R₂₂, R₂₃, R₂₆, R₂₇, R₃₀, and R₃₈ to R₄₁ are independently selected from hydrogen and deuterium, X is halogen, R₇ has any one of the definitions described above, and the structure represented by formula IIId comprises at least one deuterium atom:

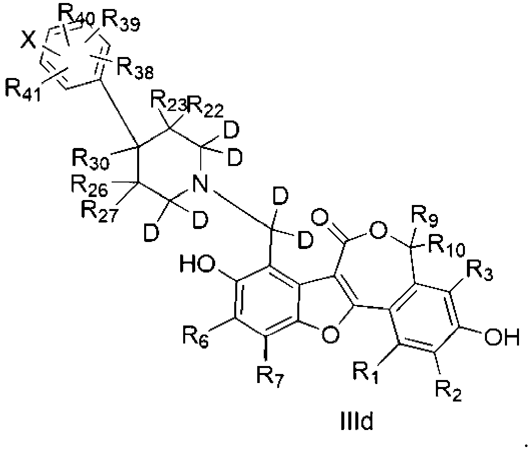

In a further preferred embodiment, the present disclosure provides a compound having a structural formula shown as IIIe below, or a pharmaceutically acceptable salt, a stereoisomer, a tautomer, an N-oxide, a hydrate, a solvate, or a prodrug thereof, wherein R₁, R₂, R₃, R₆, R₉, R₁₀, R₂₂, R₂₃, R₂₆, R₂₇, R₃₀ to R₃₂, and R₃₅ to R₃₇ are independently selected from hydrogen and deuterium, p is 0, 1, 2, or 3, R₇ has any one of the definitions described above, and the structure represented by formula IIIe comprises at least one deuterium atom:

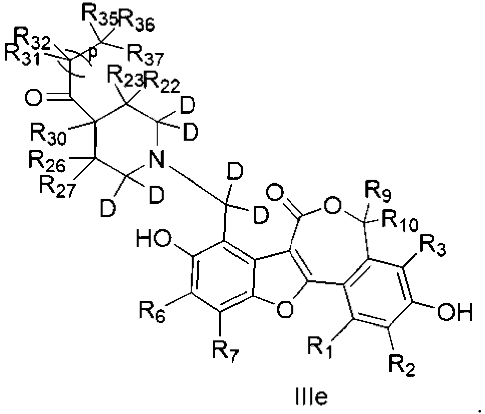

In a further preferred embodiment, the present disclosure provides a compound having a structural formula shown as IIIf below, or a pharmaceutically acceptable salt, a stereoisomer, a tautomer, an N-oxide, a hydrate, a solvate, or a prodrug thereof, wherein R₁, R₂, R₃, R₆, R₉, R₁₀, R₂₂, R₂₃, R₂₆, R₂₇, R₃₀ to R₃₂, R₃₅ to R₃₇, and R₄₄ are independently selected from hydrogen and deuterium, p is 0, 1, 2, or 3, R₇ has any one of the definitions described above, and the structure represented by formula IIIf comprises at least one deuterium atom:

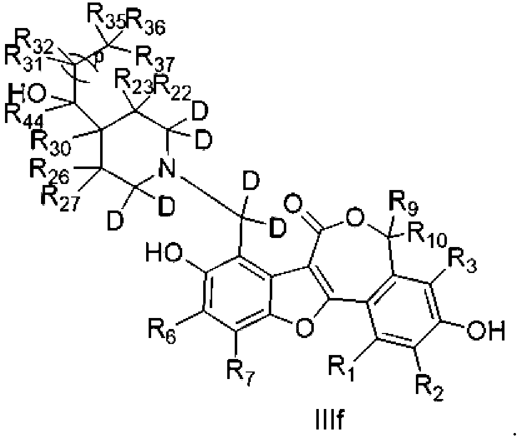

In some embodiments, the present disclosure provides a compound of formula IV, or a pharmaceutically acceptable salt, a stereoisomer, a tautomer, an N-oxide, a hydrate, a solvate, or a prodrug thereof, 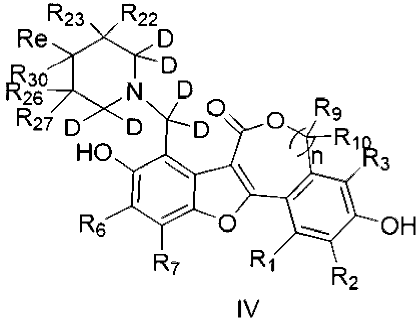
wherein n is 0 or 1;
Re is selected from: hydrogen, deuterium, C₁-C₆ alkyl optionally substituted with 1-5 substituents selected from halogen and hydroxy; C₁-C₆ alkoxy optionally substituted with 1-5 substituents selected from halogen and hydroxy; cyano; carboxyl; halogen; -NR^{c}R^{d}; hydroxy; C₆-C₁₄ aryl optionally substituted with 1-5 substituents selected from C₁-C₄ acyl, halogen, NR^{c}R^{d}, C₁-C₄ alkoxy, C₁-C₄ alkyl, cyano, hydroxy, C₁-C₄ alkoxycarbonyl, C₆-C₁₄ aryl, and halogenated C₁-C₄ alkyl; C₁-C₆ acyl; C₁-C₆ alkoxycarbonyl; C₂-C₈ alkenyloxycarbonyl; C₁-C₆ acyl substituted with NR^{c}R^{d}; C₁-C₄ acyl substituted with C₆-C₁₄ aryl optionally substituted with 1-5 substituents selected from halogen, C₁-C₄ alkyl, halogenated C₁-C₄ alkyl, and hydroxy; C₁-C₆ alkyl substituted with C₆-C₁₄ aryl optionally substituted with 1-5 substituents selected from halogen, C₁-C₄ alkyl, halogenated C₁-C₄ alkyl, and hydroxy; C₁-C₆ alkyl substituted with C₃-C₈ cycloalkyl optionally substituted with 1-5 substituents selected from halogen, C₁-C₄ alkyl, halogenated C₁-C₄ alkyl, and hydroxy; C₃-C₈ cycloalkyl optionally substituted with 1-5 substituents selected from halogen, C₁-C₄ alkyl, halogenated C₁-C₄ alkyl, and hydroxy; 4- to 7-membered heterocyclyl optionally substituted with 1-5 substituents selected from halogen, C₁-C₄ alkyl, halogenated C₁-C₄ alkyl, and hydroxy; C₁-C₆ alkyl substituted with 4- to 7-membered heterocyclyl optionally substituted with 1-5 substituents selected from halogen, C₁-C₄ alkyl, halogenated C₁-C₄ alkyl, and hydroxy; 5- to 10-membered heteroaryl optionally substituted with 1-5 substituents selected from halogen, C₁-C₄ alkyl, halogenated C₁-C₄ alkyl, and hydroxy; C₁-C₆ alkyl substituted with 5- to 10-membered heteroaryl optionally substituted with 1-5 substituents selected from halogen, C₁-C₄ alkyl, halogenated C₁-C₄ alkyl, and hydroxy; and respective deuterated variants thereof, wherein R^{c} and R^{d} are each independently selected from H, C₁-C₆ alkyl, C₂-C₈ alkenyl, and C₁-C₆ alkoxy;
R₇ is selected from H, C₁-C₆ alkyl optionally substituted with 1-5 substituents selected from halogen and hydroxy, C₂-C₆ alkenyl optionally substituted with 1-5 substituents selected from halogen and hydroxy, saturated or partially saturated C₃-C₈ carbocyclyl, halogen, hydroxy, C₁-C₄ alkoxy substituted with C₆-C₁₄ aryl, 5- to 10-membered heteroaryl, and C₆-C₁₄ aryl optionally substituted with 1-3 substituents selected from C₁-C₄ alkyl, halogenated C₁-C₄ alkyl, halogen, C₂-C₄ alkenyl, and hydroxy, as well as respective deuterated variants thereof;
R₁, R₂, R₃, R₆, R₉, R₁₀, R₂₂, R₂₃, R₂₆, R₂₇, and R₃₀ are each independently selected from hydrogen and deuterium; preferably, R₁, R₂, and R₃ are all hydrogen, and R₆, R₉, R₁₀, R₂₂, R₂₃, R₂₆, R₂₇, and R₃₀ are each independently selected from hydrogen and deuterium.

In a preferred embodiment, preferably, R₇ is selected from hydrogen, deuterium, and 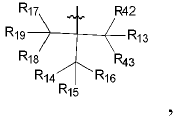 wherein R₁₃ to R₁₉, R₄₂, and R₄₃ are each independently selected from hydrogen and deuterium. In a more preferred embodiment, R₇ is selected from hydrogen, deuterium, and

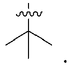

In a preferred embodiment, Re, together with the piperidinyl attached thereto, forms a group selected from: 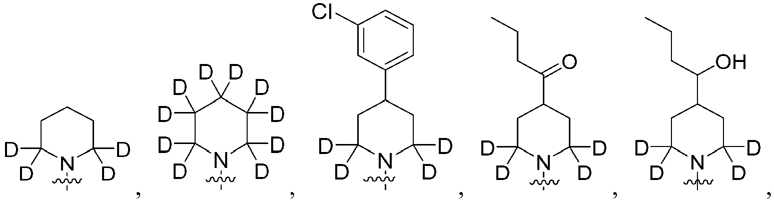 and

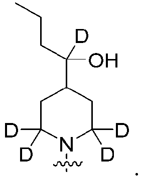

In a more preferred embodiment, Re, together with the piperidinyl attached thereto, forms a group selected from:

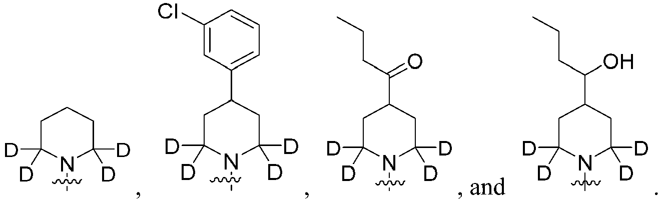

In a preferred embodiment, the compounds provided by the present disclosure, as compared with the corresponding non-deuterated compounds, exhibit a significant increase in one or more of the maximum *in vivo* plasma concentration Cₘₐₓ, the *in vivo* exposure AUC₀₋ₜ, and the maximum concentration in lung tissue distribution Cₘₐₓ. In some embodiments, the compounds provided by the present disclosure, as compared with the corresponding non-deuterated compounds, exhibit a significant increase in the maximum *in vivo* plasma concentration Cₘₐₓ. In some embodiments, the compounds provided by the present disclosure, as compared with the corresponding non-deuterated compounds, exhibit a significant increase in the *in vivo* exposure AUC₀₋ₜ. In some embodiments, the compounds provided by the present disclosure, as compared with the corresponding non-deuterated compounds, exhibit a significant increase in the maximum concentration in lung tissue distribution Cₘₐₓ. In some embodiments, the compounds provided by the present disclosure, as compared with the corresponding non-deuterated compounds, exhibit a significant increase in both the maximum *in vivo* plasma concentration Cₘₐₓ and the maximum concentration in lung tissue distribution Cₘₐₓ. In some embodiments, the compounds provided by the present disclosure, as compared with the corresponding non-deuterated compounds, exhibit a significant increase in both the maximum *in vivo* plasma concentration Cₘₐₓ and the *in vivo* exposure AUC₀₋ₜ. In some embodiments, the compounds provided by the present disclosure, as compared with the corresponding non-deuterated compounds, exhibit a significant increase in both the *in vivo* exposure AUC₀₋ₜ and the maximum concentration in lung tissue distribution Cₘₐₓ. In some embodiments, the compounds provided by the present disclosure, as compared with the corresponding non-deuterated compounds, exhibit a significant increase in the maximum *in vivo* plasma concentration Cₘₐₓ, the *in vivo* exposure AUC₀₋ₜ, and the maximum concentration in lung tissue distribution Cₘₐₓ.

In a preferred embodiment, the significant increase means that with respect to the parameter being compared, the deuterated compound exhibits a value that is more than 1-fold, e.g., 1.1- to about 100-fold, 1.1- to about 50-fold, 1.1- to about 30-fold, 1.1- to about 10-fold, 1.1- to about 8-fold, 1.1- to about 5-fold, 1.1- to about 4.5-fold, 1.1- to about 4-fold, 1.1- to about 3.5-fold, 1.1- to about 3-fold, 1.1- to about 2.5-fold, 1.1- to about 2-fold, 1.1- to about 1.9-fold, 1.1- to about 1.8-fold, 1.1- to about 1.7-fold, 1.1- to about 1.6-fold, 1.1- to about 1.5-fold, 1.1- to about 1.4-fold, 1.1- to about 1.3-fold, 1.1- to about 1.2-fold, about 2- to about 100-fold, about 2- to about 50-fold, about 2- to about 10-fold, about 2- to about 5-fold, about 2- to about 3-fold, about 3- to about 10-fold, about 3- to about 5-fold, about 4- to about 10-fold, about 5- to about 10-fold, about 6- to about 10-fold, about 7- to about 10-fold, or about 8- to about 10-fold, of the corresponding parameter of the corresponding non-deuterated compound. In a preferred embodiment, the significant increase means that with respect to the parameter being compared, the deuterated compound exhibits a value that is 1.1-fold, 1.2-fold, 1.3-fold, 1.4-fold, 1.5-fold, 1.6-fold, 1.7-fold, 1.8-fold, 1.9-fold, 2-fold, 2.5-fold, 3-fold, 3.5-fold, 4-fold, 4.5-fold, 5-fold, 5.5-fold, 6-fold, 6.5-fold, 7-fold, 7.5-fold, 8-fold, 8.5-fold, 9-fold, 9.5-fold, 10-fold, 15-fold, 20-fold, 50-fold, or 100-fold of the corresponding parameter of the corresponding non-deuterated compound.

In some other embodiments, the significant increase means that with respect to the parameter being compared, the deuterated compound provided by the present disclosure exhibits an increase of about 10% to about 500%, e.g., about 10% to about 400%, about 10% to about 300%, about 10% to about 200%, about 10% to about 100%, about 10% to about 90%, about 10% to about 80%, about 10% to about 70%, about 10% to about 60%, about 10% to about 50%, about 10% to about 40%, about 10% to about 30%, about 10% to about 20%, or about 10% to about 15%, relative to the corresponding parameter of the corresponding non-deuterated compound. In a preferred embodiment, the significant increase means that with respect to the parameter being compared, the deuterated compound provided by the present disclosure exhibits an increase of about 10%, about 15%, about 20%, about 25%, about 30%, about 35%, about 40%, about 45%, about 50%, about 55%, about 60%, about 65%, about 70%, about 75%, about 80%, about 85%, about 90%, about 95%, about 100%, about 150%, about 200%, about 250%, about 300%, about 350%, about 400%, about 450%, or about 500%, relative to the corresponding parameter of the corresponding non-deuterated compound.

In the present disclosure, when two or all of the maximum *in vivo* plasma concentration Cₘₐₓ, the *in vivo* exposure AUC₀₋ₜ, and the maximum concentration in lung tissue distribution Cₘₐₓ are significantly increased, the magnitudes of the increases are independent of one another. For example, for a certain deuterated compound, the maximum *in vivo* plasma concentration Cₘₐₓ is about 3-fold that of the corresponding non-deuterated compound, and the *in vivo* exposure AUC₀₋ₜ is about 1.5-fold that of the corresponding non-deuterated compound. For example, for a certain deuterated compound, the *in vivo* exposure AUC₀₋ₜ is increased by about 100%, while the maximum concentration in lung tissue distribution Cₘₐₓ is about 1.2-fold that of the corresponding non-deuterated compound.

In a preferred embodiment, the present disclosure provides any one of compounds having structural formulas selected from the following structural formulas, or a pharmaceutically acceptable salt, a stereoisomer, a tautomer, an N-oxide, a hydrate, a solvate, or a prodrug thereof:

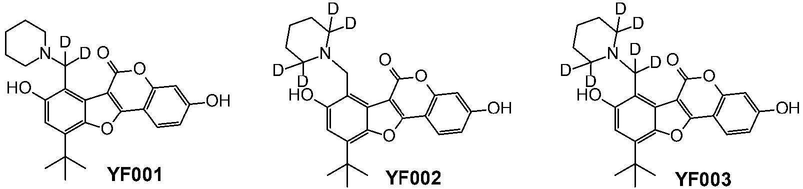

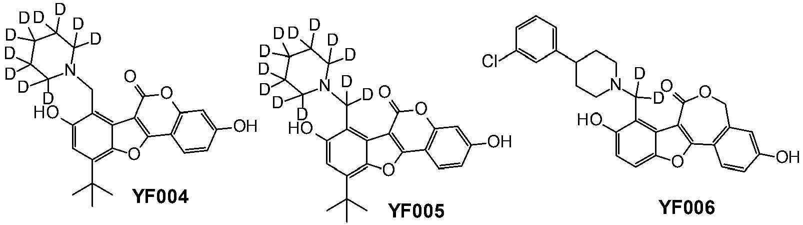

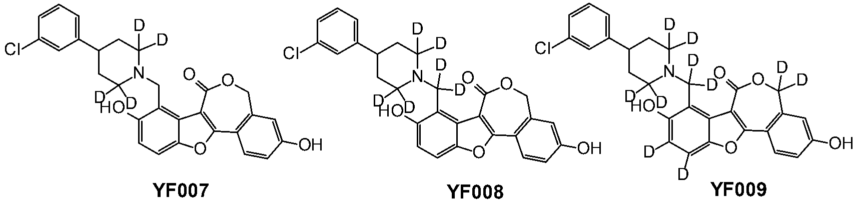

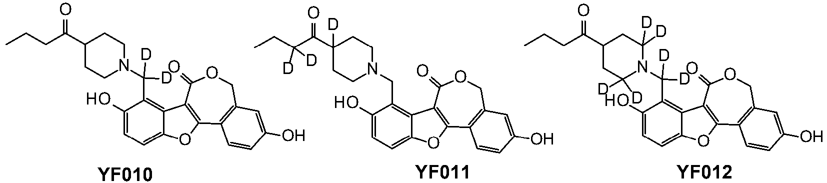

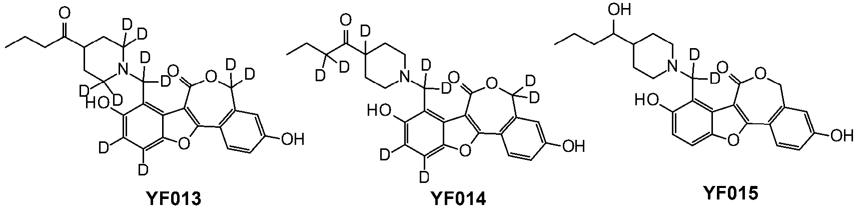

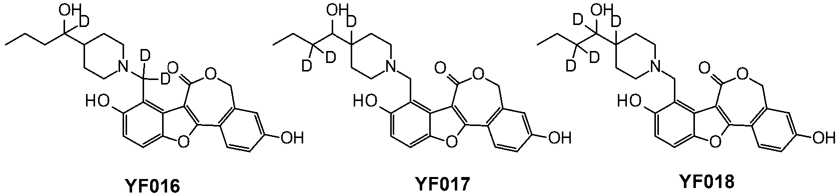

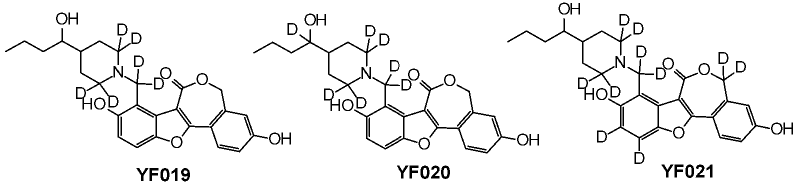

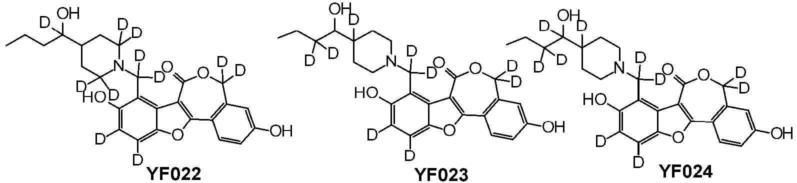

In a preferred embodiment, the present disclosure provides hydrochlorides YF025 to YF048 of any one of compounds YF001 to YF024 described above:

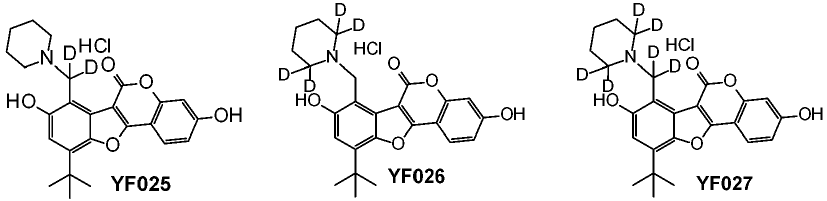

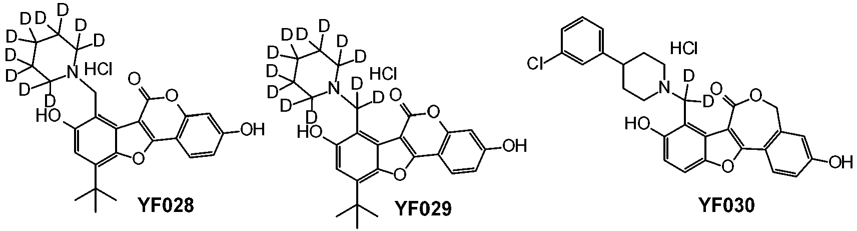

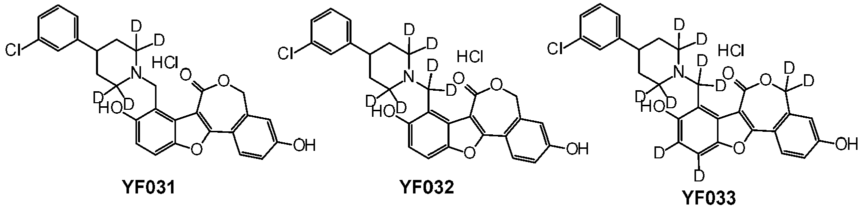

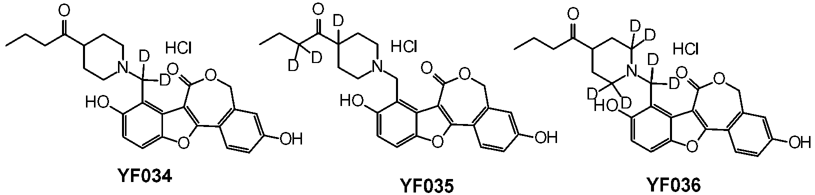

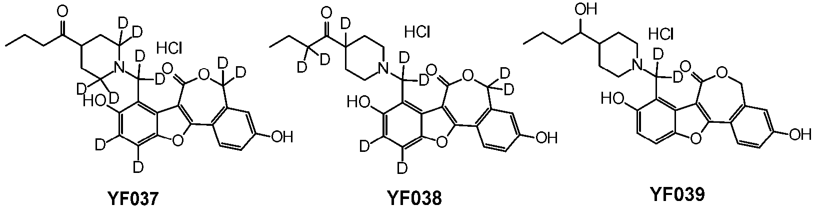

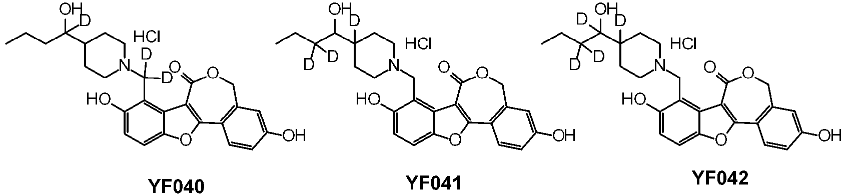

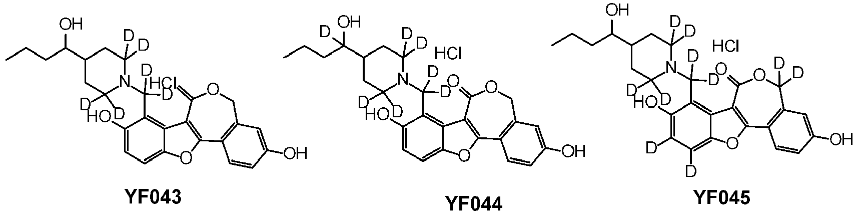

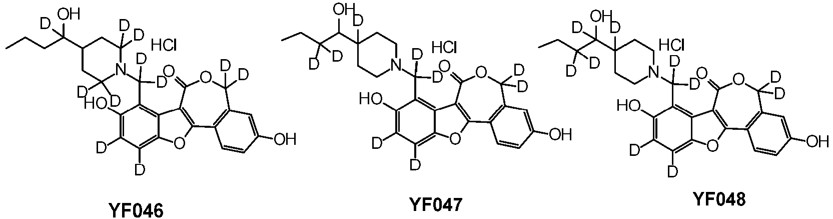

### Pharmaceutical Composition

In another aspect, the present disclosure provides a pharmaceutical composition comprising a prophylactically or therapeutically effective amount of any one of the compounds, or the pharmaceutically acceptable salt, the stereoisomer, the tautomer, the N-oxide, the hydrate, the solvate, or the prodrug thereof provided by the present disclosure as an active ingredient, and a pharmaceutically acceptable carrier or excipient.

In some embodiments, the active ingredient may account for 0.01-99 wt%, 0.05-80 wt%, 0.10-70 wt%, or 0.10-50 wt% of the total weight of the composition.

In some embodiments, the pharmaceutical composition of the present disclosure may further comprise a second active ingredient, and the second active ingredient may be an antibacterial agent, e.g., another anti-*Mycobacterium tuberculosis* agent.

In some embodiments, when administered in combination, the weight ratio of the compounds of the present disclosure to other antibacterial agents can be determined by those skilled in the art. The ratio and precise administration dose and dosing frequency depend on the particular compound of the present disclosure and other antibacterial agents used, the particular symptom to be treated, the severity of the symptom to be treated, the age, body weight, sex, diet, time of administration and general physical condition of the particular patient, the route of administration, as well as other medicaments taken by the individual, all of which are well known to those skilled in the art. In addition, it will be apparent that the effective daily dosage may be reduced or increased depending on the response of the subject being treated and/or the assessment of the compounds of the present disclosure by the prescribing physician.

The compounds of the present disclosure and one or more other antibacterial agents may be combined into a single formulation, or may be formulated as separate formulations such that they can be administered simultaneously, separately, or sequentially. Therefore, the present disclosure also relates to a product as a combined formulation for simultaneous, separate, or sequential use in the treatment of bacterial infections, which comprises the compounds of the present disclosure and one or more other antibacterial agents.

Other antibacterial agents may include β-lactam antibiotics, such as natural penicillins, semisynthetic penicillins, natural cephalosporins, semisynthetic cephalosporins, cephamycins, 1-oxacephems, clavulanic acids, penems, carbapenems, nocardicins, and monobactams; tetracyclines, anhydrotetracyclines, anthracyclines; aminoglycosides; nucleosides, such as N-nucleosides, C-nucleosides, carbocyclic nucleosides, and blasticidin S; macrolides, such as 12-membered ring macrolides, 14-membered ring macrolides, and 16-membered ring macrolides; ansamycins; peptides, such as bleomycins, gramicidins, polymyxins, bacitracins, macrocyclic peptide antibiotics containing lactone linkages, antinomycins, amphomycin, capreomycin, stallimycin, enramycins, mikamycin, neocarzinostatin, stendomycin, viomycin, and virginiamycin; cycloheximide; cycloserine; variotin; sarcomycin A; novobiocin; griseofulvin; chloramphenicol; mitomycins; fumagillin; monensin; pyrrolnitrin; fosfomycin; fusidic acid; D-(p-hydroxyphenyl)glycine; D-phenylglycine; and enediynes.

Specific antibiotics that may be combined with the compounds of the present disclosure are, for example, penicillins (penicillin potassium, procaine penicillin, and benzathine penicillin), phenoxymethylpenicillin (potassium), phenoxyethylpenicillin (potassium), propicillin, carbenicillin (disodium, phenyl sodium, and indanyl sodium), sulbenicillin, ticarcillin disodium, methicillin sodium, oxacillin sodium, o-cloxacillin sodium, dicloxacillin, flucloxacillin, ampicillin, mezlocillin, piperacillin sodium, amoxicillin, cyclacillin, hectacillin, sulbactam sodium, talampicillin hydrochloride, bacampicillin hydrochloride, pivmecillinam, cephalexin, cefaclor, cephaloglycin, cefadroxil, cephradine, cefroxadine, cephapirin sodium, cephalothin sodium, cephacetrile sodium, cefsulodin sodium, cephaloridine, cefatrizine, cefoperazone sodium, cefamandole, cefotiam hydrochloride, cefazolin sodium, ceftizoxime sodium, cefotaxime sodium, cefmenoxime hydrochloride, cefuroxime, ceftriaxone sodium, ceftazidime, cefoxitin, cefmetazole, cefatetan, latamoxef, clavulanic acid, imipenem, aztreonam, tetracycline, chlortetracycline hydrochloride, demeclocycline, oxytetracycline, methacycline, doxycycline, rolitetracycline, minocycline, daunorubicin hydrochloride, doxorubicin, aclarubicin, kanamycin sulfate, kanamycin B, tobramycin, gentamicin sulfate, dibekacin, amikacin, micronomicin, ribostamycin, neomycin sulfate, paromomycin sulfate, streptomycin sulfate, dihydrostrepomycin, destomycin A, hygromycin B, apramycin, sisomicin, netilmicin sulfate, spectinomycin hydrochloride, astromicin sulfate, validamycin, kasugamycin, polyoxin, bacitracin S, erythromycin, erythromycin estolate, oleandomycin phosphate, troleandomycin, kitasamycin, josamycin, spiramycin, tylosin, ivermectin, midecamycin, bleomycin sulfate, peplomycin sulfate, gramicidin S, polymyxin B, subtilisins, polymyxin sulfate, colistin methanesulfonate sodium, enramycin, mikamycin, virginiamycin, capreomycin sulfate, viomycin, enviomycin, vancomycin, actinomycin D, neocarzinostatin, betahistine, pepstatin, monensin antibiotics, lasalocid, salinomycin, amphotericin B, nystatin, natamycin, trichostatin, plicamycin, lincomycin, clindamycin, clindamycin palmitate hydrochloride, flavophospholipol, cycloserine, pecilocin, griseofulvin, chloramphenicol, chloramphenicol palmitate, mitomycins C, pyrrolnitrin, fosfomycin, fusidic acid, bicozamycin, tiamulin, and siccanin.

Other *Mycobacterial* drugs that may be combined with the compounds of the present disclosure are, for example, rifampicin, ethambutol, pyrazinamide, isoniazid, levofloxacin, moxifloxacin, gatifloxacin, ofloxacin, kanamycin, amikacin, capreomycin, streptomycin, ethionamide, prothionamide, cycloserine, terizidone, para-aminosalicylic acid, clofazimine, clarithromycin, amoxicillin-clavulanate, delamanid, pretomanid, bedaquiline, sutezolid, TB47, GSK 3036656, gepotidacin, thioacetazone, meropenem-clavulanate, TBA-7371, OPC-167832, Telacebec (Q203), BTZ-043, Contezolid (MRX-4/MRX-1), Delpazolid (LCB01-0371), Macozinone, Pretomanid, SPR720, SQ109, TBI-166, TBI-223, and thioridazine.

The pharmaceutical composition of the present disclosure may be administered topically (e.g., via the skin), and may be in the form of creams, solutions, suspensions, aerosols, or dry powder formulations; or may be administered systemically, e.g., by oral administration in the form of tablets, capsules, syrups, powders, or granules; or by gastrointestinal administration in the form of solutions or suspensions; or by subcutaneous (injection) administration; or by intravenous (injection) administration; or by rectal administration in the form of suppositories; or by transdermal administration. The composition of the present disclosure can be obtained by conventional methods using conventional pharmaceutical excipients well known in the art. These pharmaceutical compositions are preferably in single dosage forms particularly suitable for oral administration or parenteral injection. For example, in preparing compositions for oral dosage forms, in the case of oral liquid formulations such as suspensions, syrups, elixirs, emulsions, and solutions, any customary pharmaceutical medium may be used, such as water, glycols, oils, alcohols, and the like; or in the case of powders, pills, capsules, and tablets, solid carriers such as starches, sugars, kaolin, diluents, lubricants, binders, disintegrants, and the like may be used. Because of ease of administration, tablets and capsules represent the most advantageous oral dosage unit forms, in which case solid pharmaceutical carriers are obviously employed. For compositions for parenteral administration, the carrier typically includes at least a major proportion of sterile water, although other components may be included, for example to aid solubility. For example, injectable solutions may be prepared in which the carrier includes saline solutions, glucose solutions, or mixed solutions of saline and glucose. Injectable suspensions may also be prepared, in which case appropriate liquid carriers, suspending agents, and the like may be employed. Also included are solid form formulations designed to be converted into liquid form formulations shortly before use.

The pharmaceutical composition may additionally comprise various other components well known in the art, such as lubricants, stabilizers, buffers, emulsifiers, viscosity modifiers, surfactants, preservatives, flavoring agents, or colorants.

For ease of administration and uniformity of dosage, it is particularly preferred that the above pharmaceutical compositions be formulated as unit dosage forms. As used herein, a unit dosage form refers to a physically discrete unit suitable as a single dosage, each unit containing a predetermined amount of active ingredient calculated to produce the desired therapeutic effect in association with the required pharmaceutical carrier. Examples of such unit dosage forms include tablets (including scored tablets or coated tablets), capsules, pills, suppositories, powder packets, wafers, injection solutions or suspensions, and the like, as well as segregated multiples thereof. The daily dosage of the compound of the present disclosure will, of course, vary depending on the compound employed, the mode of administration, the therapy desired, and the mycobacterial disease identified.

### Use and Method

In another aspect, the present disclosure provides pharmaceutical use of any one of the compounds, or the pharmaceutically acceptable salt, the stereoisomer, the tautomer, the N-oxide, the hydrate, the solvate, or the prodrug thereof provided.

In some embodiments, the present disclosure provides use of any one of the compounds of formulas I, II, IIa, IIb, IIc, IId, III, IIIa, IIIb, IIIc, IIId, IIIe, IIIf, and IV, or the pharmaceutically acceptable salt, the stereoisomer, the tautomer, the N-oxide, the hydrate, the solvate, or the prodrug thereof in the preparation of a medicament for preventing or treating a disease caused by bacterial infection.

In some embodiments, the present disclosure provides any one of the compound of formulas I, II, IIa, IIb, IIc, IId, III, IIIa, IIIb, IIIc, IIId, IIIe, IIIf, and IV, or the pharmaceutically acceptable salt, the stereoisomer, the tautomer, the N-oxide, the hydrate, the solvate, or the prodrug thereof for use in preventing or treating a disease caused by bacterial infection.

In some embodiments, the present disclosure provides a method for preventing or treating a disease caused by bacterial infection, and the method includes administering to a subject in need thereof a prophylactically or therapeutically effective amount of any one of the compounds of formulas I, II, IIa, IIb, IIc, IId, III, IIIa, IIIb, IIIc, IIId, IIIe, IIIf, and IV, or the pharmaceutically acceptable salt, the stereoisomer, the tautomer, the N-oxide, the hydrate, the solvate, or the prodrug thereof.

In any one of the above embodiments, the bacterium is selected from: *Mycobacterium tuberculosis,* drug-resistant *Mycobacterium tuberculosis, Mycobacterium smegmatis, Klebsiella pneumoniae, Staphylococcus aureus, Pseudomonas aeruginosa, Acinetobacter baumannii, Mycobacterium leprae, Mycobacterium bovis, Mycobacterium marinum, Corynebacterium diphtheriae, Bordetella pertussis, Haemophilus influenzae, and Streptococcus pneumoniae.* In some embodiments, the bacterium is *Mycobacterium tuberculosis* or drug-resistant *Mycobacterium tuberculosis.* In some embodiments, the drug-resistant *Mycobacterium tuberculosis* is a rifampicin-resistant bacterium, an isoniazid-resistant bacterium, or a dual rifampicin- and isoniazid-resistant bacterium.

In some embodiments, the disease is a pulmonary infectious disease. In a preferred embodiment, the disease is an infectious disease caused by pulmonary tubercle bacillus. In a preferred embodiment, the disease is tuberculosis.

### Examples

The present disclosure provides a class of deuterated derivatives obtained by deuteration of tetracyclic derivatives having inhibitory activity against *Mycobacterium tuberculosis,* which exhibit favorable pharmacokinetic properties and significant *in vitro* and *in vivo* anti-tuberculosis efficacy.

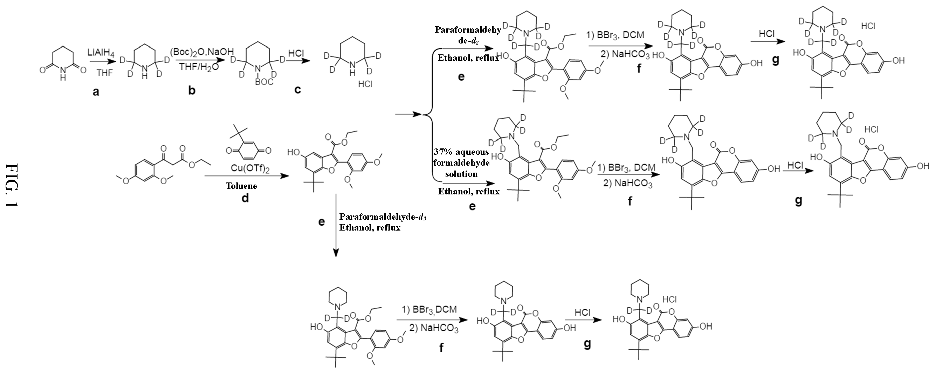

FIG. 1 and the following synthetic routes show the preparation routes of the compounds of the present disclosure or the intermediates thereof. Detailed procedures of step a to step g shown in FIG. 1 are as follows.

**Step a:** Under anhydrous and oxygen-free conditions and in an ice bath, extra-dry tetrahydrofuran (THF) was added to a reaction flask containing deuterated lithium aluminum hydride (LiAlD₄, 2.0 eq), and then an extra-dry THF solution containing glutarimide (1.0 eq) was added dropwise. The reaction temperature was controlled to be lower than 5 °C, and the mixture was reacted for 30 min. The cold bath was removed, and the mixture was allowed to naturally warm to room temperature and heated at reflux for 3 h. The mixture was allowed to naturally cool to room temperature. Purified water (10.0 eq) and a 15% aqueous NaOH solution (10.0 eq) were slowly added dropwise in sequence in an ice bath to quench the reaction, and the resulting mixture was filtered to obtain an aqueous THF solution containing 2,2,6,6-piperidine-*d*₄ having a pH of about 12-14. The solution was directly used in the next step.

**Step b:** Di-tert-butyl dicarbonate ((Boc)₂O, 0.7 eq) was added to the aqueous THF solution containing 2,2,6,6-piperidine-*d*₄, and the mixture was stirred at room temperature for 3 h, extracted with ethyl acetate, and washed three times with purified water. The organic phase was dried over anhydrous Na₂SO₄ and concentrated to obtain a crude product of tert-butyl 2,2,6,6-piperidine-*d*₄-1-carboxylate.

**Step c:** The crude product of tert-butyl 2,2,6,6-piperidine-*d*₄-1-carboxylate was dissolved in ethyl acetate, and a solution of HCl in ethyl acetate (3.5 M, 5.0 eq) was added. The mixture was reacted at room temperature for 3 h and concentrated to obtain 2,2,6,6-piperidine-*d*₄ hydrochloride.

**Step d:** Ethyl 3-(2,4-dimethoxyphenyl)-3-oxopropionate (1.05 eq), copper(II) trifluoromethanesulfonate (0.1 eq), and tert-butyl-p-benzoquinone (1.0 eq) were added to a reaction flask, and toluene (2 mL/mmol) was added. The mixture was reacted at 110 °C. After the reaction was completed as monitored by TLC, the reaction solution was concentrated to obtain a crude product, and the crude product was then slurried with methanol and filtered under vacuum to obtain ethyl 7-(tert-butyl)-2-(2,4-dimethoxyphenyl)-5-hydroxybenzofuran-3-carboxylate.

**Step e:** Ethyl 7-(tert-butyl)-2-(2,4-dimethoxyphenyl)-5-hydroxybenzofuran-3-carboxylate (1.0 eq), 2,2,6,6-piperidine-*d*₄ hydrochloride (2.0 eq), triethylamine (2.0 eq), and paraformaldehyde-*d*₂ were added to a reaction flask, and ethanol was added. The mixture was reacted at 80 °C. After the reaction was completed as monitored by TLC, the reaction mixture was concentrated to remove ethanol, and the residue was purified by column chromatography to obtain ethyl 7-(tert-butyl)-2-(2,4-dimethoxyphenyl)-5-hydroxy-4-((piperidin-1-yl-2,2,6,6-*d*₄)methyl-*d*₂)benzofuran-3-carboxylate.

The 2,2,6,6-piperidine-*d*₄ hydrochloride (2.0 eq) was replaced with piperidine hydrochloride to obtain ethyl 7-(tert-butyl)-2-(2,4-dimethoxyphenyl)-5-hydroxy-4-((piperidin-1-ylmethyl-*d*₂)benzofuran-3-carboxylate.

Paraformaldehyde-*d*₂ was replaced with a 37% aqueous formaldehyde solution to obtain ethyl 7-(tert-butyl)-2-(2,4-dimethoxyphenyl)-5-hydroxy-4-((piperidin-1-yl-2,2,6,6-*d*₄)methyl)benzofuran-3-carboxylate.

**Step f:** Ethyl 7-(tert-butyl)-2-(2,4-dimethoxyphenyl)-5-hydroxy-4-((piperidin-1-yl-2,2,6,6-*d*₄)methyl-*d*₂)benzofuran-3-carboxylate (2.0 eq) was dissolved in extra-dry dichloromethane. A solution of boron tribromide in dichloromethane (DCM) (2 M in DCM, 4.0 eq) was added dropwise at -20 °C under nitrogen atmosphere, and then the mixture was reacted at room temperature overnight. After the reaction was completed as monitored by TLC, ethanol was added to the reaction solution to quench the reaction. The mixture was refluxed for 1 h, and a solid was precipitated. The mixture was concentrated to obtain a crude product, and the crude product was slurried with dichloromethane and filtered under vacuum to obtain a solid. The solid was treated with a saturated sodium bicarbonate solution, filtered under vacuum, and washed with purified water to obtain 10-(tert-butyl)-3,8-dihydroxy-7-((piperidin-1-yl-2,2,6,6-*d*₄)methyl-*d*₂)-6H-benzofuran[3,2-c]chromen-6-one (compound YF003).

Ethyl 7-(tert-butyl)-2-(2,4-dimethoxyphenyl)-5-hydroxy-4-((piperidin-1-yl-2,2,6,6-*d*₄)methyl-*d*₂)benzofuran-3-carboxylate could be replaced with ethyl 7-(tert-butyl)-2-(2,4-dimethoxyphenyl)-5-hydroxy-4-((piperidin-1-ylmethyl-*d*₂)benzofuran-3-carboxylate to obtain 10-(tert-butyl)-3,8-dihydroxy-7-(piperidin-1-ylmethyl-*d*₂)-6H-benzofuran[3,2-c]chromen-6-one (compound YF001).

Ethyl 7-(tert-butyl)-2-(2,4-dimethoxyphenyl)-5-hydroxy-4-((piperidin-1-yl-2,2,6,6-*d*₄)methyl-*d*₂)benzofuran-3-carboxylate could be replaced with ethyl 7-(tert-butyl)-2-(2,4-dimethoxyphenyl)-5-hydroxy-4-((piperidin-1-yl-2,2,6,6-*d*₄)methyl)benzofuran-3-carboxylate to obtain 10-(tert-butyl)-3,8-dihydroxy-7-((piperidin-1-yl-2,2,6,6-*d*₄)methyl)-6H-benzofuran[3,2-c]chromen-6-one (compound YF002).

**Step g:** At room temperature, 10-(tert-butyl)-3,8-dihydroxy-7-((piperidin-1-yl-2,2,6,6-*d*₄)methyl-*d*₂)-6H-benzofuran[3,2-c]chromen-6-one (compound YF003) was dissolved in a mixed solvent of dichloromethane and methanol, and a solution of excess hydrogen chloride in ethyl acetate (3.5 M in ethyl acetate (EA)) was added. The mixture was reacted at room temperature for 1 h, concentrated, and then slurried with dichloromethane and filtered under vacuum to obtain a white solid, i.e., 10-(tert-butyl)-3,8-dihydroxy-7-((piperidin-1-yl-2,2,6,6-*d*₄)methyl-*d*₂)-6H-benzofuran[3,2-c]chromen-6-one hydrochloride (compound YF027).

Compound YF003 could be replaced with compound YF002 to obtain compound YF026.

Compound YF003 could be replaced with compound YF001 to obtain compound YF025.

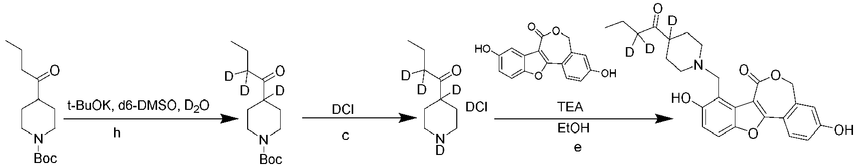

**Step h:** Tert-butyl 4-butyrylpiperidine-1-carboxylate (1.0 eq) and potassium tert-butoxide (*t*-BuOK, 3.6 eq) were dissolved in dimethyl sulfoxide-*d₆* (*d₆*-DMSO) under nitrogen atmosphere, and the mixture was reacted at room temperature for 0.5 h. Heavy water (D₂O, 10.0 eq) was added, and the mixture was reacted at 80 °C overnight. After the reaction was completed as detected by LC-MS, the reaction mixture was extracted with ethyl acetate and concentrated to obtain a crude product of tert-butyl 4-(butyryl-2,2-*d₂*)piperidine-1-carboxylate-4-*d*, which was directly used in the next step without purification.

**Step c:** 20% deuterated hydrochloric acid (DCl, 3.0 eq) was added to the crude product containing tert-butyl 4-(butyryl-2,2-*d₂*)piperidine-1-carboxylate-4-*d* (1.0 eq) under nitrogen atmosphere, and the mixture was reacted at room temperature overnight. The mixture was then concentrated to obtain a crude product of 4-(butyryl-2,2-*d₂*)piperidine-1,4-*d₂* deuterated hydrochloride, which was directly used in the next step without purification.

**Step e:** 4-(Butanoyl-2,2-*d₂*)piperidine-1,4-*d₂*deuterated hydrochloride (1.5 eq), 3,9-dihydroxybenzo[5,6]oxepino[4,3-b]benzofuran-7(5H)-one (1.0 eq) (prepared by referring to Example 10), a 37% aqueous formaldehyde solution (3.0 eq), and triethylamine (TEA, 3.0 eq) were dissolved in anhydrous ethanol (EtOH), and the mixture was reacted at 80 °C overnight. After the reaction was completed as monitored by TLC, the reaction mixture was concentrated and purified by column chromatography to obtain 8-((4-(butanoyl-2,2-*d₂*)piperidin-1-yl-4-*d*)methyl-3,9-dihydroxybenzo[5,6]oxepino[4,3-b]benzofuran-7(5H)-one (compound YF011).

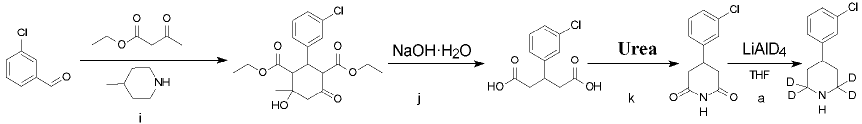

**Step i:** 3-Chlorobenzaldehyde (1.0 eq), ethyl acetoacetate (2.0 eq), and 4-methylpiperidine (0.3 eq) were mixed and placed in a reaction flask, and the mixture was reacted at room temperature overnight. A solid was precipitated, slurried with petroleum ether, and filtered to obtain white diethyl 2-(3-chlorophenyl)-4-hydroxy-4-methyl-6-oxocyclohexane-1,3-dicarboxylate.

**Step j:** A 30%-50% aqueous sodium hydroxide solution (1-2 mL/mmol) was added to diethyl 2-(3-chlorophenyl)-4-hydroxy-4-methyl-6-oxocyclohexane-1,3-dicarboxylate (1.0 eq), and the mixture was reacted at 100 °C. After the reaction was completed as monitored by TLC, the reaction solution was cooled to room temperature, purified water (1-5 times the aqueous sodium hydroxide solution) was added, and the pH value was adjusted to 5-6 with concentrated hydrochloric acid. The mixture was filtered to remove impurities, and the filtrate was collected. Hydrochloric acid was then added to the filtrate, and a white solid was precipitated, filtered, and dried to obtain white 3-(3-chlorophenyl)pentanedioic acid.

**Step k:** 3-(3-Chlorophenyl)pentanedioic acid (1.0 eq) and urea (4.0 eq) were mixed, and the mixture was reacted at 150 °C. After the reaction was completed as monitored by TLC, the reaction mixture was slurried with purified water and filtered to obtain 4-(3-chlorophenyl)piperidine-2,6-dione.

**Step a:** Deuterated lithium aluminum hydride (2.0 eq) was dissolved in extra-dry THF (10 mL/g) under anhydrous conditions and under nitrogen atmosphere, and extra-dry THF (1 mL/mmol) containing 4-(3-chlorophenyl)piperidine-2,6-dione (1.0 eq) was added dropwise in an ice bath. The reaction temperature was controlled to be lower than 5 °C, and the mixture was reacted for 30 min, and then refluxed. After the reaction was completed as monitored by TLC, purified water was added in an ice bath to quench the reaction, and EA was added. The resulting mixture was filtered, and the filtrate was collected, concentrated, and purified by column chromatography to obtain 4-(3-chlorophenyl)piperidine-2,2,6,6-*d₄*.

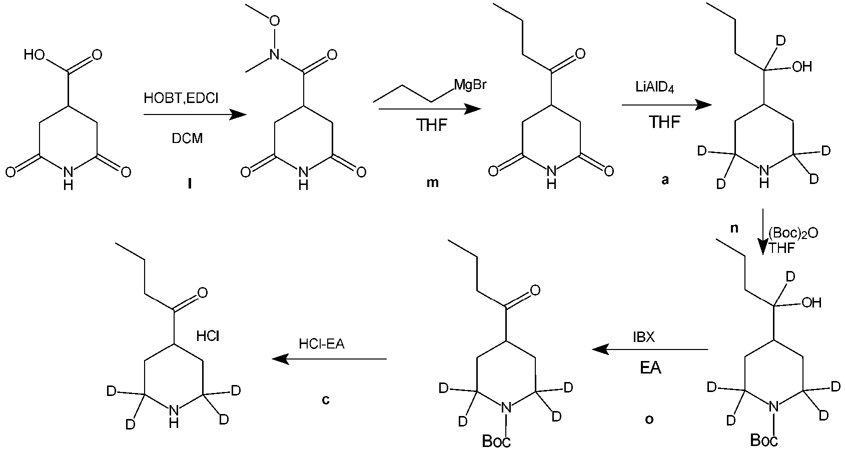

**Step l:** 2,6-Dioxopiperidine-4-carboxylic acid (1.0 eq), 1-hydroxybenzotriazole (HOBT) (1.5 eq), 1-ethyl-(3-dimethylaminopropyl)carbodiimide hydrochloride (EDCI) (1.3 eq), and methoxymethylamine hydrochloride (2.0 eq) were placed in a reaction flask, dichloromethane was added as a solvent, and triethylamine was added. The mixture was reacted at room temperature overnight. After the reaction was completed as monitored by TLC, the reaction mixture was quenched with purified water, and the resulting mixture was extracted with dichloromethane. The organic phase was concentrated and purified by column chromatography to obtain N-methoxy-N-methyl-2,6-dioxopiperidine-4-carboxamide.

**Step m:** A solution of propylmagnesium bromide in tetrahydrofuran (4.0 eq, 1 M in THF) was added dropwise to an extra-dry THF solution containing N-methoxy-N-methyl-2,6-dioxopiperidine-4-carboxamide (1.0 eq) under anhydrous and oxygen-free conditions and in an ice bath, and the mixture was then reacted at room temperature. After the reaction was completed as monitored by TLC, the reaction was quenched with a saturated ammonium chloride solution, and the resulting mixture was extracted with ethyl acetate. The organic phase was concentrated and purified by column chromatography to obtain 4-butyrylpiperidine-2,6-dione.

**Step a:** Extra-dry tetrahydrofuran was slowly added to a reaction flask containing LiAlD₄ (3.5 eq) in an ice bath, and then a solution of 4-butyrylpiperidine-2,6-dione in THF (1.0 eq) was added dropwise. The mixture was refluxed overnight. After the reaction was completed as monitored by TLC, the reaction was quenched with purified water and a 15% aqueous NaOH solution, and the reaction mixture was filtered to obtain an aqueous solution of 1-(piperidin-4-yl-2,2,6,6-*d₄*)butan-1-*d*-1-ol in THF, with pH controlled at about 12-14.

**Step n:** (Boc)₂O (1.1 eq) was added to the aqueous THF solution containing 1-(piperidin-4-yl-2,2,6,6-*d₄*)butan-1-*d*-1-ol, and the mixture was reacted at room temperature overnight, extracted with EA, and concentrated to obtain a crude product of tert-butyl 4-(1-hydroxybutyl-1-*d*)piperidine-1-carboxylate-2,2,6,6-*d₄*.

**Step o:** The crude product of tert-butyl 4-(1-hydroxybutyl-1-d)piperidine-1-carboxylate-2,2,6,6-*d₄* was dissolved in EA, and 2-iodoxybenzoic acid (IBX, 4.0 eq) was added. The mixture was heated to 77 °C and reacted. After the reaction was completed as monitored by·TLC, the reaction mixture was cooled, filtered, and concentrated to obtain a crude product of tert-butyl 4-butyrylpiperidine-1-carboxylate-2,2,6,6-*d₄*.

**Step c:** The crude product of tert-butyl 4-butyrylpiperidine-1-carboxylate-2,2,6,6-*d₄* was dissolved in EA, and a solution of hydrogen chloride in ethyl acetate (4-5.0 eq) was added. The mixture was reacted at room temperature for 2-3 h and concentrated to obtain 1-(piperidin-4-yl-2,2,6,6-*d₄*)butan-1-one hydrochloride.

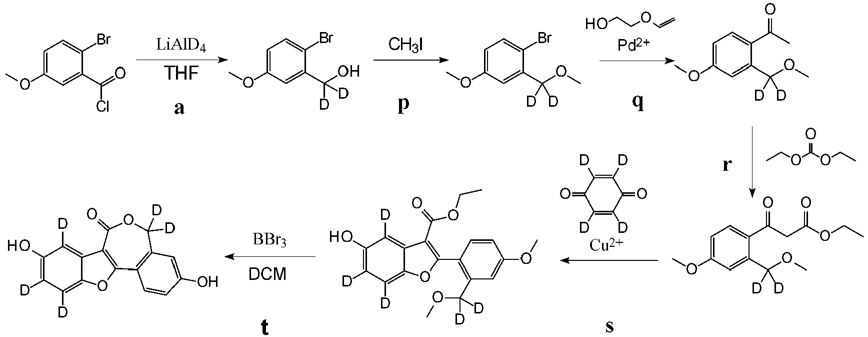

**Step a:** A solution of deuterated lithium aluminum hydride in tetrahydrofuran (3-4.0 eq) was added dropwise to an extra-dry THF solution containing 2-bromo-5-methoxybenzoyl chloride (1.0 eq) under anhydrous and oxygen-free conditions and in an ice bath, and the mixture was reacted at room temperature. After the reaction was completed as monitored by TLC, purified water was added to quench the reaction, and the resulting mixture was filtered. The filtrate was extracted with EA and purified by column chromatography to obtain (2-bromo-5-methoxyphenyl)methanol-*d₂*.

**Step p:** (2-Bromo-5-methoxyphenyl)methanol-*d₂* (1.0 eq) and sodium hydride (1.5 eq, 60% in oil) were dissolved in extra-dry THF, and iodomethane (1.5 eq) was added. The mixture was reacted at room temperature. After the reaction was completed as monitored by TLC, ice water was added to quench the reaction, and the resulting mixture was extracted with EA and purified by column chromatography to obtain 1-bromo-4-methoxy-2-(methoxymethyl-*d₂*)benzene.

**Step q:** 1-Bromo-4-methoxy-2-(methoxymethyl-*d₂*)benzene (1.0 eq), 2-vinylethanol (2.0 eq), and potassium carbonate (2.0 eq) were mixed and dispersed in water under nitrogen atmosphere, and then bis(diphenylphosphino)propane (dppp, 0.05 eq) and palladium acetate (0.025 eq) were sequentially added. The mixture was reacted at 80-90 °C overnight. When the starting materials were substantially completely consumed as monitored by TLC, and an intermediate product was produced, the reaction solution was cooled to room temperature and extracted with EA, followed by liquid separation. Concentrated hydrochloric acid was added dropwise to the organic phase, and the resulting mixture was reacted at room temperature for about 0.5 h. After the intermediate product was completely converted into the target product as monitored by TLC, water was added for liquid separation, and the organic phase was concentrated and purified by column chromatography to obtain 1-(4-methoxy-2-(methoxymethyl-*d₂*)phenyl)ethanone.

**Step r:** Diethyl carbonate (2.5 eq) was dissolved in THF, and potassium tert-butoxide (3.2 eq) was added. The mixture was stirred at room temperature for 0.5 h, and then a solution of 1-(4-methoxy-2-(methoxymethyl-*d₂*)phenyl)ethanone (1.0 eq) in THF was added dropwise. The mixture was reacted at 60-70 °C for about 2 h. After the reaction was completed as monitored by TLC, the reaction solution was poured into ice water to quench the reaction, and the pH value was adjusted to about 6 with concentrated hydrochloric acid. The resulting mixture was extracted with EA and purified by column chromatography to obtain ethyl 3-(4-methoxy-2-(methoxymethyl-*d₂*)phenyl)-3-oxopropionate.

**Step s:** Ethyl 3-(4-methoxy-2-(methoxymethyl-*d₂*)phenyl)-3-oxopropionate (1.0 eq), 1,4-benzoquinone-*d₄* (1.2 eq), and copper(II) trifluoromethanesulfonate (0.1 eq) were dissolved in xylene, and the mixture was reacted at 80-90 °C for about 2-4 h. After the reaction was completed as monitored by TLC, the reaction solution was directly purified by column chromatography to obtain ethyl 5-hydroxy-2-(4-methoxy-2(methoxymethyl-*d₂*)phenyl)benzofuran-3-carboxylate-4,6,7-*d₃*.

**Step t:** Ethyl 5-hydroxy-2-(4-methoxy-2-(methoxymethyl-*d₂*)phenyl)benzofuran-3-carboxylate-4,6,7-*d₃* was dissolved in extra-dry DCM under anhydrous and oxygen-free conditions. A solution of boron tribromide in DCM (4.0-6.0 eq, 1.0-4.0 M) was added dropwise in an ice bath, and the mixture was reacted at room temperature overnight. Ice-cold methanol was added to quench the reaction, and the reaction solution was directly concentrated. 1,4-Dioxane and purified water were added, and the resulting mixture was reacted at 100 °C for about 3 h. After the reaction was completed as monitored by·TLC, the reaction solution was concentrated. Water was added, and a solid was precipitated and then filtered under vacuum to obtain a crude product of 3,9-dihydroxybenzo[5,6]oxepino[4,3-b]benzofuran-7(5H)-one-5,5,8,10,11-*d₅.-*dihydroxybenzo[5,6]oxepino[4,3-b]benzofuran-7(5H)-one-5,5,8,10,11-*d₅*.

### Example 1

### 10-(tert-Butyl)-3,8-dihydroxy-7-(piperidin-1-ylmethyl-d₂)-6H-benzofuran[3,2-c]chromen-6-one (compound YF001)

Ethyl 3-(2,4-dimethoxyphenyl)-3-oxopropionate (1.05 eq), copper(II) trifluoromethanesulfonate (0.1 eq), and tert-butyl-p-benzoquinone (1.0 eq) were added to a reaction flask, and toluene (2 mL/mmol) was added. The mixture was reacted at 110 °C. After the reaction was completed as monitored by TLC, the reaction solution was concentrated to obtain a crude product of ethyl 7-(tert-butyl)-2-(2,4-dimethoxyphenyl)-5-hydroxybenzofuran-3-carboxylate, and the crude product was then slurried with methanol and filtered under vacuum to obtain ethyl 7-(tert-butyl)-2-(2,4-dimethoxyphenyl)-5-hydroxybenzofuran-3-carboxylate.

Ethyl 7-(tert-butyl)-2-(2,4-dimethoxyphenyl)-5-hydroxybenzofuran-3-carboxylate (1.0 eq), piperidine hydrochloride (2.0 eq), triethylamine (2.0 eq), and paraformaldehyde-*d*₂ were added to a reaction flask, and ethanol was added. The mixture was reacted at 80 °C. After the reaction was completed as monitored by TLC, the reaction mixture was concentrated to remove ethanol, and the residue was purified by column chromatography to obtain ethyl 7-(tert-butyl)-2-(2,4-dimethoxyphenyl)-5-hydroxy-4-(piperidin-1-ylmethyl-*d₂*)benzofuran-3-carboxylate.

Ethyl 7-(tert-butyl)-2-(2,4-dimethoxyphenyl)-5-hydroxy-4-(piperidin-1-ylmethyl-*d₂*)benzofuran-3-carboxylate (2.0 eq) was dissolved in extra-dry dichloromethane (DCM). A solution of boron tribromide in dichloromethane (2 M in DCM, 4.0 eq) was added dropwise at -20 °C under nitrogen atmosphere, and then the mixture was reacted at room temperature overnight. After the reaction was completed as monitored by TLC, ethanol was added to the reaction solution to quench the reaction. The mixture was refluxed for 1 h, and a solid was precipitated. The mixture was concentrated to obtain a crude product of 10-(tert-butyl)-3,8-dihydroxy-7-(piperidin-1-ylmethyl-*d₂*)-6H-benzofuran[3,2-c]chromen-6-one, and the crude product was slurried with DCM and filtered under vacuum to obtain a solid. The solid was treated with a saturated sodium bicarbonate solution, filtered under vacuum, and washed with purified water to obtain 10-(tert-butyl)-3,8-dihydroxy-7-(piperidin-1-ylmethyl-*d₂*)-6H-benzofuran[3,2-c]chromen-6-one (compound YF001) in the form of an off-white solid. ¹H NMR (600 MHz, DMSO-*d*₆) δ 10.89 (s, 1H), 10.33 (s, 1H), 7.99 (d, *J=* 8.6 Hz, 1H), 7.07 (s, 1H), 7.01 (dd, *J* = 8.6, 2.2 Hz, 1H), 6.95 (d, *J =* 2.2 Hz, 1H), 3.27 (m, 2H), 3.14-3.08 (m, 2H), 1.84-1.82 (m, 2H), 1.67-1.61 (m, 3H), 1.51 (s, 9H), 1.50-1.40 (m, 1H). HRMS(ESI) m/z: Calcd for C₂₅H₂₅D₂NO₅ (M+H)⁺ 424.21239; Found 424.20770.

### Example 2

10-(tert-Butyl)-3,8-dihydroxy-7-((piperidin-1-yl-2,2,6,6-*d*₄)methyl)-6H-benzofuran[3,2-c]chromen-6-one (compound YF002)

Under anhydrous and oxygen-free conditions and in an ice bath, extra-dry tetrahydrofuran (THF) was added to a reaction flask containing deuterated lithium aluminum hydride (LiAlD₄, 2.0 eq), and then an extra-dry THF solution containing glutarimide (1.0 eq) was added dropwise. The mixture was reacted at a low temperature (< 5 °C) for 30 min and heated under reflux for 3 h. Purified water (10.0 eq) and a 15% aqueous NaOH solution (10.0 eq) were slowly added dropwise in sequence in an ice bath to quench the reaction, and the mixture was filtered to obtain an aqueous THF solution containing 2,2,6,6-piperidine-*d*₄ having a pH of about 12-14. The solution was directly used in the next step.

(Boc)₂O (0.7 eq) was added to the aqueous THF solution containing 2,2,6,6-piperidine-*d*₄, and the mixture was reacted at room temperature for 3 h, extracted with ethyl acetate, and washed three times with purified water. The organic phase was concentrated to obtain a crude product of tert-butyl 2,2,6,6-piperidine-*d*₄-1-carboxylate.

The crude product of tert-butyl 2,2,6,6-piperidine-*d*₄-1-carboxylate was dissolved in ethyl acetate (EA), and a solution of HCl in EA (3.5 M, 5.0 eq) was added. The mixture was reacted at room temperature for 3 h and concentrated to obtain 2,2,6,6-piperidine-*d*₄ hydrochloride.

The subsequent preparation method was the same as that of Example 1, except that paraformaldehyde-*d*₂ was replaced with a 37% aqueous formaldehyde solution, and piperidine hydrochloride was replaced with 2,2,6,6-piperidine-*d*₄ hydrochloride to obtain a final product in the form of an off-white solid. ¹H NMR (600 MHz, DMSO-*d*₆) δ 10.92 (s, 1H), 10.36 (s, 1H), 7.99 (d, *J* = 8.6 Hz, 1H), 7.06 (s, 1H), 7.01 (dd, *J* = 8.6, 2.2 Hz, 1H), 6.95 (d, *J* = 2.2 Hz, 1H),4.32 (s, 2H), 1.85-1.78 (m, 2H), 1.67-1.60 (m, 3H), 1.52 (s, 9H), 1.49-1.38 (m, 1H). HRMS(ESI) m/z: Calcd for C₂₅H₂₃D₄NO₅ (M+H)⁺ 426.22804; Found 426.22012.

### Example 3

### 10-(tert-Butyl)-3,8-dihydroxy-7-((piperidin-1-yl-2,2,6,6-d₄)methyl-d₂)-6H-benzofuran[3,2-c]chromen-6-one (compound YF003)

The preparation method was the same as that of Example 2, except that the 37% aqueous formaldehyde solution was replaced with paraformaldehyde-*d*₂ to obtain a final product in the form of an off-white solid. ¹H NMR (600 MHz, DMSO-*d*₆) δ 10.90 (s, 1H), 10.33 (s, 1H), 8.00 (d, *J =* 8.6 Hz, 1H), 7.07 (s, 1H), 7.02 (dd, *J* = 8.6, 2.2 Hz, 1H), 6.96 (d, *J* = 2.2 Hz, 1H), 1.88-1.77 (m, 2H), 1.68-1.60 (m, 3H), 1.52 (s, 9H), 1.49-1.37 (m, 1H). HRMS(ESI) m/z: Calcd for C₂₅H₂₁D₆NO₅ (M+H)⁺ 428.24369; Found 428.23270.

### Example 4

### 10-(tert-Butyl)-3,8-dihydroxy-7-((piperidin-1-yl-d₁₀)methyl)-6H-benzofuran[3,2-c]chromen-6-one (compound YF004)

The preparation method was the same as that of Example 1, except that paraformaldehyde-*d*₂ was replaced with a 37% aqueous formaldehyde solution, and piperidine hydrochloride was replaced with piperidine-*d₁₁* deuterated hydrochloride to obtain a final product in the form of an off-white solid. ¹H NMR (600 MHz, DMSO-*d*₆) δ 10.95 (s, 1H), 10.33 (s, 1H), 7.96 (d, *J =* 8.7 Hz, 1H), 7.04 (s, 1H), 7.00 (dd, *J* = 8.7, 2.3 Hz, 1H), 6.93 (d, *J =* 2.3 Hz, 1H), 4.27 (s, 2H), 1.55(s, 9H). HRMS(ESI) m/z: Calcd for C₂₅H₁₇D₁₀NO₅ (M+H)⁺ 432.25201; Found 432.25012.

### Example 5

### 10-(tert-Butyl)-3,8-dihydroxy-7-((piperidin-1-yl-d₁₀)methyl-d₂)-6H-benzofuran[3,2-c]chromen-6-one (compound YF005)

The preparation method was the same as that of Example 1, except that piperidine hydrochloride was replaced with piperidine-*d₁₁* deuterated hydrochloride to obtain a final product in the form of an off-white solid. ¹H NMR (600 MHz, DMSO-*d*₆) δ 10.90 (s, 1H), 10.25 (s, 1H), 7.91 (d, *J*=8.7 Hz, 1H), 7.10 (s, 1H), 7.07 (dd, *J*=8.7, 2.4 Hz, 1H), 6.98 (d, *J=*2.4 Hz, 1H), 1.58(s, 9H). HRMS(ESI) m/z: Calcd for C₂₅H₁₅D₁₂NO₅ (M+H)⁺ 434.26400; Found 434.25962.

### Example 6

### 8-((4-(3-Chlorophenyl)piperidin-1-yl)methyl-d₂)-3,9-dihydroxybenzo[5,6]oxepino[4,3-b]benzofuran-7(5H)-one (compound YF006)

2-Bromo-5-hydroxybenzaldehyde (1.0 eq) was dissolved in methanol, and sodium borohydride (0.5 eq) was added in an ice bath. The mixture was reacted at room temperature for 0.5 h. After the reaction was completed as monitored by TLC, the reaction mixture was directly concentrated under reduced pressure to remove methanol and diluted with water. The pH value was adjusted to about 6 with 1 M hydrochloric acid, and a solid was precipitated. The mixture was filtered under vacuum to obtain a solid, and the solid was washed with water and dried to obtain 2-bromo-5-hydroxybenzyl alcohol.

In an ice bath, 2-bromo-5-hydroxybenzyl alcohol (1.0 eq) and sodium hydride (2.5 eq) were added to a reaction flask, extra-dry tetrahydrofuran was added, iodomethane (2.5 eq) was added dropwise, and the mixture was reacted at room temperature overnight. After the reaction was completed as monitored by TLC, the reaction was quenched with ice water, and the pH value was adjusted to about 6 with 1 M hydrochloric acid. The mixture was extracted with ethyl acetate, dried over anhydrous sodium sulfate, concentrated under reduced pressure, and purified by silica gel column chromatography to obtain 1-bromo-4-methoxy-2-(methoxymethyl)benzene.

1-Bromo-4-methoxy-2-(methoxymethyl)benzene (1.0 eq), palladium acetate (0.025 eq), 1,3-bis(diphenylphosphino)propane (0.05 eq), and potassium carbonate (2.0 eq) were added to a reaction flask, and water and 2-vinylethanol (2.5 eq) were added under nitrogen atmosphere. The mixture was reacted at 80 °C overnight. After the reaction was completed as monitored by TLC, an intermediate product was produced, and the reaction mixture was extracted with ethyl acetate. Concentrated hydrochloric acid was added to the ethyl acetate phase, and the mixture was further reacted at room temperature for about 1 h. After the intermediate product was completely consumed as monitored by TLC, a saturated aqueous sodium bicarbonate solution was added to quench the reaction, and the pH value was adjusted to about 7. The mixture was extracted with ethyl acetate and purified by silica gel column chromatography to obtain 2-(methoxymethyl)-4-methoxyacetophenone.

Sodium hydride (3.2 eq) and diethyl carbonate (2.5 eq) were dissolved in xylene at room temperature under nitrogen atmosphere, and the mixture was stirred at room temperature for 30 min. 2-(Methoxymethyl)-4-methoxyacetophenone (1.0 eq) was dissolved in xylene, and the resulting solution was added dropwise to the reaction system. The mixture was reacted at 110 °C for an additional 30 min. After the reaction was completed as monitored by TLC, the reaction solution was poured into ice water, and the pH was adjusted to neutral or slightly acidic with hydrochloric acid. The mixture was extracted with ethyl acetate and purified by silica gel column chromatography to obtain ethyl 3-[4-methoxy-2-(methoxymethyl)phenyl]-3-oxopropionate.

Ethyl 3-[4-methoxy-2-(methoxymethyl)phenyl]-3-oxopropionate (1.05 eq), copper(II) trifluoromethanesulfonate (0.1 eq), and benzoquinone (1.0 eq) were dissolved in xylene, and the mixture was reacted at 110 °C overnight. After the reaction was completed as monitored by TLC, the reaction mixture was concentrated under reduced pressure and purified by silica gel column chromatography to obtain 9-hydroxy-3-methoxybenzo[5,6]oxepino[4,3-b]benzofuran-7(5H)-one.

4-(3-Chlorophenyl)piperidine hydrochloride (2.0 eq), triethylamine (2.2 eq), paraformaldehyde-*d*₂ (3.3 eq), and 95% ethanol (3-5 mL/mmol) were mixed, and the reaction solution was stirred at 80 °C until the solution became completely clear and transparent. 3-Methoxy-9-hydroxybenzo[5,6]oxepino[4,3-b]benzofuran-7(5H)-one (1.0 eq) was added, and the mixture was reacted at 80 °C overnight. After the reaction was completed as monitored by TLC, the reaction mixture was concentrated and purified by column chromatography to obtain 8-((4-(3-chlorophenyl)piperidin-1-yl)methyl-*d*₂)-3-methoxy-9-hydroxybenzo[5,6]oxepino[4,3-b]benzofuran-7(5H)-one.

8-((4-(3-Chlorophenyl)piperidin-1-yl)methyl-*d*₂)-3-methoxy-9-hydroxybenzo[5,6]oxepino[4,3-b]benzofuran-7(5H)-one (1.0 eq) was dissolved in extra-dry dichloromethane (3-5 mL/mmol) under anhydrous and oxygen-free conditions, and a solution of boron tribromide in dichloromethane (1 M in DCM, 3.0 eq) was added dropwise at -20 °C. The mixture was reacted at room temperature overnight. After the reaction was completed as monitored by TLC, ethanol (the same volume as that of DCM) was added to quench the reaction. The mixture was refluxed at 80 °C for 1 h, concentrated, and purified by column chromatography. The target molecule was collected and concentrated. Subsequently, a saturated aqueous sodium bicarbonate solution was added, and the resulting mixture was stirred at room temperature for 1 h, filtered under vacuum, washed with purified water, and dried to obtain a final product in the form of a gray solid. ¹H NMR (600 MHz, DMSO-*d*₆) δ 10.65 (s, 1H), 10.46 (s, 1H), 7.89 (d, *J* = 8.4 Hz, 1H), 7.75 (d, *J* = 8.9 Hz, 1H), 7.36 (t, *J* = 7.8 Hz, 1H), 7.31-7.25 (m, 2H), 7.19 (d, *J =* 7.8 Hz, 1H), 7.16 -7.11 (m, 2H), 7.08 (dd, *J =* 8.5, 2.4 Hz, 1H), 5.27 (s, 2H), 3.49-3.39 (m, 2H), 3.20-3.15 (m, 2H), 2.93-2.90 (m, 1H), 2.07-1.86 (m, 4H). HRMS(ESI) m/z: Calcd for C₂₈H₂₂D₂ClNO₅ (M+H)⁺ 492.14995; Found (M + H)⁺ 492.15339.

### Example 7

### 8-((4-(3-Chlorophenyl)piperidin-1-yl-2,2,6,6-d₄)methyl)-3,9-dihydroxybenzo[5,6]oxepino[4,3-b]benzofuran-7(5H)-one (compound YF007)

3-Chlorobenzaldehyde (1.0 eq), ethyl acetoacetate (2.0 eq), and 4-methylpiperidine (0.3 eq) were mixed and placed in a reaction flask, and the mixture was reacted at room temperature overnight. A solid was precipitated, slurried with petroleum ether, and filtered to obtain white diethyl 2-(3-chlorophenyl)-4-hydroxy-4-methyl-6-oxocyclohexane-1,3-dicarboxylate.

A 30%-50% aqueous sodium hydroxide solution (1 mL/mmol) was added to diethyl 2-(3-chlorophenyl)-4-hydroxy-4-methyl-6-oxocyclohexane-1,3-dicarboxylate (1.0 eq), and the mixture was reacted at 100 °C. After the reaction was completed as monitored by TLC, purified water (4 times the aqueous sodium hydroxide solution) was added, and the pH value was adjusted to 5-6 with concentrated hydrochloric acid. The mixture was filtered, and the filtrate was collected. Hydrochloric acid was added to the filtrate, and a white solid was precipitated, filtered, and dried to obtain white 3-(3-chlorophenyl)pentanedioic acid.

3-(3-Chlorophenyl)pentanedioic acid (1.0 eq) and urea (4.0 eq) were mixed, and the mixture was reacted at 150 °C. After the reaction was completed as monitored by TLC, the reaction mixture was slurried with purified water and filtered to obtain 4-(3-chlorophenyl)piperidine-2,6-dione.

Deuterated lithium aluminum hydride (2.0 eq) was dissolved in extra-dry THF (10 mL/g) under anhydrous conditions and under nitrogen atmosphere, and extra-dry THF (1 mL/mmol) containing 4-(3-chlorophenyl)piperidine-2,6-dione (1.0 eq) was added dropwise in an ice bath. The reaction temperature was controlled to be lower than 5 °C, and the mixture was reacted for 30 min, and then refluxed. After the reaction was completed as monitored by TLC, purified water was added in an ice bath to quench the reaction, and EA was added. The resulting mixture was filtered, and the filtrate was collected, concentrated, and purified by column chromatography to obtain 4-(3-chlorophenyl)piperidine-2,2,6,6-*d₄*.

Ethyl 3-[4-methoxy-2-(methoxymethyl)phenyl]-3-oxopropionate (1.0 eq; prepared by referring to Example 6) and benzoquinone (1.2 eq) were dissolved in xylene, and copper(II) trifluoromethanesulfonate (0.1 eq) was added under nitrogen atmosphere. The mixture was reacted at 80-90 °C for 1-2 h. When the starting materials were completely consumed as monitored by TLC, and only a small amount of 9-hydroxy-3-methoxybenzo[5,6]oxepino[4,3-b]benzofuran-7(5H)-one was produced, the reaction was terminated, and the mixture was directly purified by column chromatography to obtain ethyl 5-hydroxy-2-(4-methoxy-2-(methoxymethyl)phenyl)benzofuran-3-carboxylate.

Ethyl 5-hydroxy-2-(4-methoxy-2-(methoxymethyl)phenyl)benzofuran-3-carboxylate (1.0 eq), 4-(3-chlorophenyl)piperidine-2,2,6,6-*d₄* (1.2 eq), and a 37% aqueous formaldehyde solution (3.0 eq) were added to ethanol (4 mL/mmol), and complete dissolution was achieved at 80 °C. The pH was adjusted to slightly basic with triethylamine, and the mixture was reacted at 80 °C overnight. After the reaction was completed as monitored by TLC, the reaction mixture was purified by column chromatography to obtain ethyl 4-(4-(3-chlorophenyl)piperidin-1-yl-2,2,6,6-*d*₄)methyl)-5-hydroxy-2-(4-methoxy-2-(methoxymethyl)phenyl)benzofuran-3-carboxylate.

Ethyl 4-(4-(3-chlorophenyl)piperidin-1-yl-2,2,6,6-*d*₄)methyl)-5-hydroxy-2-(4-methoxy-2-(methoxymethyl)phenyl)benzofuran-3-carboxylate (1.0 eq) was dissolved in extra-dry DCM (4 mL/mmol) under anhydrous and oxygen-free conditions, and a solution of boron tribromide in dichloromethane (2.0 M in DCM, 4.0 eq) was added dropwise at a low temperature (0 °C). The mixture was reacted at room temperature, and the reaction was completed as monitored by TLC. An intermediate product was produced, and methanol was added at a low temperature (0 °C) to quench the reaction, and the mixture was concentrated. 1,4-Dioxane and purified water were then added, and the resulting mixture was reacted at 100 °C. After the intermediate product was completely converted into the target molecule as monitored by TLC, the reaction was terminated. The pH was adjusted to slightly basic with a saturated sodium bicarbonate solution, followed by extraction with DCM. The organic phase was concentrated and purified by column chromatography to obtain a final product in the form of a yellow solid. ¹H NMR (600 MHz, DMSO-*d*₆) δ 10.60 (s, 1H), 10.53 (s, 1H), 7.85 (d, *J* = 8.5 Hz, 1H), 7.46 (d, *J* = 8.8 Hz, 1H), 7.38-7.29 (m, 2H), 7.28-7.20 (m, 2H), 7.12 (d, *J* = 2.4 Hz, 1H), 7.06 (dd, *J* = 8.5, 2.4 Hz, 1H), 6.89 (d, *J* = 8.8 Hz, 1H), 5.21 (s, 2H), 4.32 (s, 2H), 2.62-2.57 (m, 1H), 2.02-1.92 (m, 4H). HRMS(ESI) m/z: Calcd for C₂₈H₂₀D₄ClNO₅ (M+H)⁺ 494.17342; Found (M + H) ⁺ 494.26536.

### Example 8

### 8-((4-(3-Chlorophenyl)piperidin-1-yl-2,2,6,6-d₄)methyl-d₂)-3,9-dihydroxybenzo[5,6]oxepino[4,3-b]benzofuran-7(5H)-one (compound YF008)

The preparation method was the same as that of Example 7, except that the 37% aqueous formaldehyde solution was replaced with paraformaldehyde-*d*₂ to obtain a final product in the form of a yellow solid. ¹H NMR (400 MHz, DMSO-*d*₆) δ 10.66 (s, 1H), 10.45 (s, 1H), 7.87 (dd, *J* = 8.5, 1.8 Hz, 1H), 7.72 (dd, *J* = 8.9, 1.9 Hz, 1H), 7.35 (td, *J =* 8.0, 7.4, 1.8 Hz, 1H), 7.31-7.24 (m, 2H), 7.23-7.14 (m, 2H), 7.12 (d, *J* = 2.1 Hz, 1H), 7.08 (dt, *J* = 8.6, 2.1 Hz, 1H), 5.36 (s, 2H), 2.84-2.86 (m, 1H), 2.23-1.74 (m, 4H). HRMS(ESI) m/z: Calcd for C₂₈H₁₈D₆ClNO₅ (M+H)⁺ 496.18907; Found (M + H) ⁺ 496.18000.

### Example 9

### 8-((4-(3-Chlorophenyl)piperidin-1-yl-2,2,6,6-d₄)methyl-d₂)-3,9-dihydroxybenzo[5,6]oxepino[4,3-b]benzofuran-7(5H)-one-5,5,10,11-d₄ (compound YF009)

A solution of deuterated lithium aluminum hydride in tetrahydrofuran (3-4.0 eq) was added dropwise to an extra-dry THF solution containing 2-bromo-5-methoxybenzoyl chloride (1.0 eq) under anhydrous and oxygen-free conditions and in an ice bath, and the mixture was reacted at room temperature. After the reaction was completed as monitored by TLC, purified water was added to quench the reaction, and the resulting mixture was filtered. The filtrate was extracted with EA and purified by column chromatography to obtain (2-bromo-5-methoxyphenyl)methanol-*d₂*.

(2-Bromo-5-methoxyphenyl)methanol-*d₂* (1.0 eq) and sodium hydride (1.5 eq, 60% in oil) were dissolved in extra-dry THF, and iodomethane (1.5 eq) was added. The mixture was reacted at room temperature. After the reaction was completed as monitored by TLC, ice water was added to quench the reaction, and the resulting mixture was extracted with EA and purified by column chromatography to obtain 1-bromo-4-methoxy-2-(methoxymethyl-*d₂*)benzene.

1-Bromo-4-methoxy-2-(methoxymethyl-*d₂*)benzene (1.0 eq), 2-vinylethanol (2.0 eq), and potassium carbonate (2.0 eq) were mixed and dispersed in water under nitrogen atmosphere, and then 1,3-bis(diphenylphosphino)propane (dppp) (0.05 eq) and palladium acetate (0.025 eq) were sequentially added. The mixture was reacted at 80-90 °C overnight. When the starting materials were substantially completely consumed as monitored by TLC, and an intermediate product was produced, the reaction solution was cooled to room temperature and extracted with EA, followed by liquid separation. Concentrated hydrochloric acid was added dropwise to the organic phase, and the resulting mixture was reacted at room temperature for about 0.5-1 h. After the intermediate product was completely converted into the target product as monitored by TLC, water was added for liquid separation, and the organic phase was concentrated and purified by column chromatography to obtain 1-(4-methoxy-2-(methoxymethyl-*d₂*)phenyl)ethanone.

Diethyl carbonate (2.5 eq) was dissolved in THF, and potassium tert-butoxide (3.2 eq) was added. The mixture was stirred at room temperature for 0.5 h, and then a solution of 1-(4-methoxy-2-(methoxymethyl-*d₂*)phenyl)ethanone (1.0 eq) in THF was added dropwise. The mixture was reacted at 60-70 °C for about 2 h. After the reaction was completed as monitored by TLC, the reaction solution was poured into ice water to quench the reaction, and the pH value was adjusted to about 6 with concentrated hydrochloric acid. The resulting mixture was extracted with EA and purified by column chromatography to obtain ethyl 3-(4-methoxy-2-(methoxymethyl-*d₂*)phenyl)-3-oxopropionate.

Ethyl 3-(4-methoxy-2-(methoxymethyl-*d₂*)phenyl)-3-oxopropionate (1.0 eq), 1,4-benzoquinone-*d₄* (1.2 eq), and copper(II) trifluoromethanesulfonate (0.1 eq) were dissolved in xylene, and the mixture was reacted at 80-90 °C for about 2-4 h. After the reaction was completed as monitored by TLC, the reaction solution was directly purified by column chromatography to obtain ethyl 5-hydroxy-2-(4-methoxy-2(methoxymethyl-*d₂*)phenyl)benzofuran-3-carboxylate-4,6,7-*d₃*.

The subsequent preparation method was the same as that of Example 7, except that ethyl 5-hydroxy-2-(4-methoxy-2-(methoxymethyl)phenyl)benzofuran-3-carboxylate was replaced with ethyl 5-hydroxy-2-(4-methoxy-2-(methoxymethyl-*d₂*)phenyl)benzofuran-3-carboxylate-4,6,7-*d₃* to obtain a final product in the form of a yellow solid. ¹H NMR (600 MHz, Chloroform-d) δ 10.30 (s, 1H), 10.01 (s, 1H), 7.44 (d*, J =* 8.8 Hz, 1H), 7.36-7.27 (m, 2H), 7.21-7.19(m, 2H), 6.81 (dd*, J =* 8.7, 1.9 Hz, 1H), 6.78 (d*, J =* 1.9 Hz, 1H), 2.76-2.74 (m, 1H), 2.13-1.91 (m, 4H). HRMS(ESI) m/z: Calcd for C₂₈H₁₄D₁₀ClNO₅ (M+H)⁺ 500.20701; Found (M + H) ⁺ 500.19705.

### Example 10

### 8-((4-Butanoyl)piperidin-1-ylmethyl-d₂)-3,9-dihydroxybenzo[5,6]oxepino[4,3-b]benzofuran-7(5H)-one (compound YF010)

For the preparation method, reference may be made to Example 6, and this example differs in the following steps after the preparation of 9-hydroxy-3-methoxybenzo[5,6]oxepino[4,3-b]benzofuran-7(5H)-one.

9-Hydroxy-3-methoxybenzo[5,6]oxepino[4,3-b]benzofuran-7(5H)-one (1.0 eq) was dissolved in extra-dry dichloromethane, and 1 M BBr₃ (4.0 eq, 1 M in DCM) was added dropwise at -20 °C. The mixture was reacted at room temperature overnight. After the reaction was completed as monitored by TLC, the reaction was quenched with methanol, and the reaction mixture was concentrated under reduced pressure to obtain a crude product. The crude product was washed with methanol to obtain 3,9-dihydroxybenzo[5,6]oxepino[4,3-b]benzofuran-7(5H)-one.

4-Butanoylpiperidine hydrochloride (1.5 eq), 3,9-dihydroxybenzo[5,6]oxepino[4,3-b]benzofuran-7(5H)-one (1.0 eq), paraformaldehyde-*d*₂ (3.0 eq), and triethylamine (3.0 eq) were dissolved in anhydrous ethanol, and the mixture was reacted at 80 °C overnight. After the reaction was completed as monitored by TLC, the reaction mixture was concentrated and purified by column chromatography to obtain a final product in the form of a yellow solid. ¹H NMR (400 MHz, DMSO-*d*₆) δ 10.50 (s, 2H), 7.84 (d, *J =* 8.5 Hz, 1H), 7.45 (d, *J=* 8.8 Hz, 1H), 7.12 (d, *J =* 2.5 Hz, 1H), 7.05 (dd, *J* = 8.5, 2.4 Hz, 1H), 6.87 (d, *J =* 8.8 Hz, 1H), 5.15 (s, 2H), 2.77 (s, 2H), 2.45 (t, *J =* 7.1 Hz, 2H), 2.42-2.30 (m, 1H), 2.07 (t, *J =* 11.6 Hz, 2H), 1.76 (d, *J =* 12.8 Hz, 2H), 1.50-1.36 (m, 4H), 0.83 (t, *J =* 7.4 Hz, 3H). HRMS(ESI) m/z: Calcd for C₂₆H₂₅D₂NO₆ (M+H)⁺ 452.20731; Found (M + H-HCl) ⁺ 452.20303.

### Example 11

### 8-((4-(Butanoyl-2,2-d₂)piperidin-1-yl-4-d)methyl)-3,9-dihydroxybenzo[5,6]oxepino[4,3-b]benzofuran-7(5H)-one (YF011)

Tert-butyl 4-butyrylpiperidine-1-carboxylate (1.0 eq) and potassium tert-butoxide (3.6 eq) were dissolved in dimethyl sulfoxide-*d₆* (*d₆*-DMSO) under nitrogen atmosphere, and the mixture was reacted at room temperature for 0.5 h. Heavy water (10.0 eq) was added, and the mixture was reacted at 80 °C overnight. After the reaction was completed as detected by LC-MS, the reaction mixture was extracted with EA and concentrated to obtain a crude product of tert-butyl 4-(butyryl-2,2-*d₂*)piperidine-1-carboxylate-4-d, which was directly used in the next step without purification.

A 20% aqueous deuterated hydrochloric acid (DCl) solution (3.0 eq) was added to the crude product of tert-butyl 4-(butyryl-2,2-*d₂*)piperidine-1-carboxylate-4-d (1.0 eq) under nitrogen atmosphere, and the mixture was reacted at room temperature overnight. The mixture was then concentrated to obtain a crude product of 4-(butyryl-2,2-*d₂*)piperidine-1,4-*d₂* hydrochloride, which was directly used in the next step without purification.

4-(Butanoyl-2,2-*d₂*)piperidine-1,4-*d₂* hydrochloride (1.5 eq), 3,9-dihydroxybenzo[5,6]oxepino[4,3-b]benzofuran-7(5H)-one (prepared by referring to Example 10) (1.0 eq), a 37% aqueous formaldehyde solution (3.0 eq), and triethylamine (3.0 eq) were dissolved in anhydrous ethanol, and the mixture was reacted at 80 °C overnight. After the reaction was completed as monitored by TLC, the reaction mixture was concentrated and purified by column chromatography to obtain a final product in the form of a pale brownish-yellow solid. ¹H NMR (600 MHz, DMSO-*d*₆) δ 10.48 (s, 2H), 7.84 (d, *J* = 8.5 Hz, 1H), 7.47 (s, 1H), 7.12 (d, *J* = 2.5 Hz, 1H), 7.05 (dd, *J* = 8.5, 2.4 Hz, 1H), 6.89 (s, 1H), 5.16 (s, 2H), 3.95 (s, 2H), 2.99-2.62 (m, 2H), 2.08 (s, 2H), 1.77 (s, 2H), 1.47-1.40 (m, 4H), 0.83 (t, *J* = 7.4 Hz, 3H). HRMS(ESI) m/z: Calcd for C₂₆H₂₄D₃NO₆ (M+H)⁺ 453.21513; Found (M + H) ⁺ 453.20804.

### Example 12

### 8-((4-Butanoylpiperidin-1-yl-2,2,6,6-d₄)-methyl-d₂)-3,9-dihydroxybenzo[5,6]oxepino[4,3-b]benzofuran-7(5H)-one (YF012)

2,6-Dioxopiperidine-4-carboxylic acid (1.0 eq), 1-hydroxybenzotriazole (HOBT) (1.5 eq), 1-ethyl-(3-dimethylaminopropyl)carbodiimide hydrochloride (EDCI) (1.3 eq), and methoxymethylamine hydrochloride (2.0 eq) were placed in a reaction flask, DCM was added as a solvent, and triethylamine was added. The mixture was reacted at room temperature overnight. After the reaction was completed as monitored by TLC, the reaction was quenched with purified water, and the resulting mixture was extracted with DCM. The organic phase was concentrated and purified by column chromatography to obtain N-methoxy-N-methyl-2,6-dioxopiperidine-4-carboxamide.

A solution of propylmagnesium bromide in THF (4.0 eq, 1 M in THF) was added dropwise to an extra-dry THF solution containing N-methoxy-N-methyl-2,6-dioxopiperidine-4-carboxamide (1.0 eq) under anhydrous and oxygen-free conditions and in an ice bath, and the mixture was then reacted at room temperature. After the reaction was completed as monitored by TLC, the reaction was quenched with a saturated ammonium chloride solution, and the resulting mixture was extracted with EA. The organic phase was concentrated and purified by column chromatography to obtain 4-butyrylpiperidine-2,6-dione.

Extra-dry THF was slowly added to a reaction flask containing LiAlD₄ (3.5 eq) in an ice bath, and then a solution of 4-butyrylpiperidine-2,6-dione in THF (1.0 eq) was added dropwise. The mixture was refluxed overnight. After the reaction was completed as monitored by TLC, the reaction was quenched with purified water and a 15% aqueous NaOH solution, and the reaction mixture was filtered to obtain an aqueous solution of 1-(piperidin-4-yl-2,2,6,6-*d₄*)butan-1-*d*-1-ol in THF, with pH controlled at about 12-14.

(Boc)₂O (1.1 eq) was added to the aqueous THF solution containing 1-(piperidin-4-yl-2,2,6,6-*d₄*)butan-1-*d*-1-ol, and the mixture was reacted at room temperature overnight, extracted with EA, and concentrated to obtain a crude product of tert-butyl 4-(1-hydroxybutyl-1-*d*)piperidine-1-carboxylate-2,2,6,6-*d₄*.

The crude product of tert-butyl 4-(1-hydroxybutyl-1-d)piperidine-1-carboxylate-2,2,6,6-*d₄* was dissolved in EA, and 2-iodoxybenzoic acid (4.0 eq) was added. The mixture was heated to 77 °C and reacted. After the reaction was completed as monitored by·TLC, the reaction mixture was cooled, filtered, and concentrated to obtain a crude product of tert-butyl 4-butyrylpiperidine-1-carboxylate-2,2,6,6-*d₄*. The crude product of tert-butyl 4-butyrylpiperidine-1-carboxylate-2,2,6,6-*d₄* was dissolved in EA, and a solution of hydrogen chloride in ethyl acetate (4-5.0 eq) was added. The mixture was reacted at room temperature for 2-3 h and concentrated to obtain 1-(piperidin-4-yl-2,2,6,6-*d₄*)butan-1-one hydrochloride.

1-(Piperidin-4-yl-2,2,6,6-*d₄*)butan-1-one hydrochloride (1.5 eq), 3,9-dihydroxybenzo[5,6]oxepino[4,3-b]benzofuran-7(5H)-one (prepared by referring to Example 10) (1.0 eq), paraformaldehyde-*d*₂ (3.0 eq), and triethylamine (3.0 eq) were dissolved in anhydrous ethanol, and the mixture was reacted at 80 °C overnight. After the reaction was completed as monitored by·TLC, the reaction mixture was concentrated and purified by column chromatography to obtain a final product in the form of a yellow solid. ¹H NMR (400 MHz, DMSO-*d*₆) δ 10.65 (s, 1H), 10.42 (s, 1H), 7.49 (d, *J =* 8.6 Hz, 1H), 7.30 (d, *J =* 8.9 Hz, 1H), 7.10 (d, *J =* 8.9 Hz, 1H), 6.85-6.80 (m, 2H), 5.22 (s, 2H), 2.65-2.61 (m, 1H), 2.48 (t, *J* = 7.4 Hz, 2H), 1.86-1.72 (m, 4H), 1.50 (q, *J* = 7.6 Hz, 2H), 0.93 (t, *J* = 7.6 Hz, 3H). HRMS(ESI) m/z: Calcd for C₂₆H₂₁D₆NO₆ (M+H)⁺ 456.53960; Found (M + H) ⁺ 456.05103.

### Example 13

### 8-((4-Butanoylpiperidin-1-yl-2,2,6,6-d₄)-methyl-d₂)-3,9-dihydroxybenzo[5,6]oxepino[4,3-b]benzofuran-7(5H)-one-5,5,10,11-d₄ (YF013)

The preparation method was the same as that of Example 9, except for the following steps after the preparation of ethyl 5-hydroxy-2-(4-methoxy-2-(methoxymethyl-*d₂*)phenyl)benzofuran-3-carboxylate-4,6,7-*d₃*.

Ethyl 5-hydroxy-2-(4-methoxy-2-(methoxymethyl-*d₂*)phenyl)benzofuran-3-carboxylate-4,6,7-*d₃* was dissolved in extra-dry DCM under anhydrous and oxygen-free conditions. A solution of boron tribromide in DCM (4.0-6.0 eq, 1.0-4.0 M) was added dropwise in an ice bath, and the mixture was reacted at room temperature overnight. Ice-cold methanol was added to quench the reaction, and the reaction solution was directly concentrated. 1,4-Dioxane and purified water were added, and the resulting mixture was reacted at 100 °C for 3-12 h. After the reaction was completed as monitored by·TLC, the reaction solution was concentrated. Water was added, and a solid was precipitated and then filtered under vacuum to obtain a crude product of 3,9-dihydroxybenzo[5,6]oxepino[4,3-b]benzofuran-7(5H)-one-5,5,8,10,11-*d₅*. The crude product was slurried and purified to obtain 3,9-dihydroxybenzo[5,6]oxepino[4,3-b]benzofuran-7(5H)-one-5,5,8,10,11-*d₅*.

1-(Piperidin-4-yl-2,2,6,6-*d₄*)butan-1-one hydrochloride (1.5 eq), 3,9-dihydroxybenzo[5,6]oxepino[4,3-b]benzofuran-7(5H)-one-5,5,8,10,11-*d₅* (1.0 eq), paraformaldehyde-*d*₂ (3.0 eq), and triethylamine (3.0 eq) were dissolved in anhydrous ethanol, and the mixture was reacted at 80 °C overnight. After the reaction was completed as monitored by·TLC, the reaction mixture was concentrated and purified by column chromatography to obtain a final product in the form of a pale yellow solid. ¹H NMR (400 MHz, DMSO-*d*₆) δ 10.70 (s, 1H), 10.35 (s, 1H), 7.80 (d, *J =* 8.6 Hz, 1H), 7.13 (d, *J =* 2.4 Hz, 1H), 7.09 (dd, *J =* 8.6, 2.4 Hz, 1H), 2.60-2.54 (m, 1H), 2.48 (t, *J =* 7.1 Hz, 2H), 1.99-1.74 (m, 4H), 1.47 (q, *J =* 7.3 Hz, 2H), 0.89 (t, *J =* 7.3 Hz, 3H). HRMS(ESI) m/z: Calcd for C₂₆H₁₇D₁₀NO₆ (M+H)⁺ 460.24700;

### Example 14

### 8-((4-(Butanoyl-2,2-d₂)piperidin-1-yl-4-d)methyl-d₂)-3,9-dihydroxybenzo[5,6]oxepino[4,3-b]benzofuran-7(5H)-one-5,5, 10,11-d₄ (YF014)

The preparation method was the same as that of Example 13, except that 1-(piperidin-4-yl-2,2,6,6-*d₄*)butan-1-one hydrochloride was replaced with 4-(butyryl-2,2-*d₂*)piperidine-1,4-*d₂* hydrochloride to obtain a final product in the form of a pale yellow solid. ¹H NMR (400 MHz, DMSO-*d*₆) δ 10.54 (s, 1H), 10.46(s, 1H), 7.74 (d, *J* = 8.6 Hz, 1H), 7.14 (d, *J* = 2.4 Hz, 1H), 7.03 (dd, *J* = 8.6, 2.4 Hz, 1H), 3.01 -2.82 (m, 2H), 2.07 (s, 2H), 1.78-1.74 (m, 2H), 1.49-1.47 (m, 4H), 0.83 (t*, J =* 7.4 Hz, 3H). HRMS(ESI) m/z: Calcd for C₂₆H₁₈D₆NO₆ (M+H)⁺ 459.24000; Found (M + H) ⁺ 459.25780.

### Example 15

### 3,9-Dihydroxy-8-((4-(1-hydroxybutyl)piperidin-1-yl)methyl-d₂)benzo[5,6]oxepino[4,3-b]benzofuran-7(5H)-one (compound YF015)

Compound YF010 (1.0 eq) was dissolved in methanol, and sodium borohydride (1.0 eq) was added in an ice bath. The mixture was reacted at room temperature for 0.5-1 h. After the reaction was completed as monitored by TLC, the reaction solution was concentrated and purified by column chromatography to obtain a final product in the form of a pale yellow solid. ¹H NMR (600 MHz, DMSO-*d*₆) δ 7.98 (d, *J* = 8.6 Hz, 1H), 7.51 (d*, J =* 2.6 Hz, 1H), 7.48 (d, *J* = 8.8 Hz, 1H), 7.36 (dd, *J* = 8.6, 2.6 Hz, 1H), 6.91 (d, *J* = 8.8 Hz, 1H), 5.22 (s, 2H), 3.41 (t, *J* = 11.9 Hz, 2H), 3.22-3.12 (m, 1H), 3.09 (t, *J =* 11.3 Hz, 2H), 1.85 (d, *J =* 13.2 Hz, 1H), 1.68-1.23 (m, 8H), 0.85 (t, *J* = 6.9 Hz, 3H). HRMS(ESI) m/z: Calcd for C₂₆H₂₇D₂NO₆ (M+H)⁺ 454.21012; Found (M + H) ⁺ 454.22296.

### Example 16

### 3,9-Dihydroxy-8-((4-(1-hydroxybutyl-1-d)piperidin-1-yl)methyl-d₂)benzo[5,6]oxepino[4,3-b]benzofuran-7(5H)-one (compound YF016)

Compound YF010 (1.0 eq) was dissolved in methanol, and sodium borodeuteride (1.0 eq) was added in an ice bath. The mixture was reacted at room temperature for 0.5-1 h. After the reaction was completed as monitored by TLC, the reaction solution was concentrated and purified by column chromatography to obtain a final product in the form of a pale yellow solid. ¹H NMR (600 MHz, DMSO-*d*₆) δ 8.01 (d, *J =* 8.5 Hz, 1H), 7.59 (d, *J* = 2.4 Hz, 1H), 7.50 (d, *J =* 8.9 Hz, 1H), 7.45 (dd, *J* = 8.5, 2.4 Hz, 1H), 6.94 (d, *J* = 8.8 Hz, 1H), 5.24 (s, 2H), 3.34-3.10 (m, 4H), 1.80-1.78 (m, 1H), 1.68-1.10 (m, 8H), 0.80 (t, *J* = 6.8 Hz, 3H). HRMS(ESI) m/z: Calcd for C₂₆H₂₆D₃NO₆ (M+H)⁺ 455.22540; Found (M + H) ⁺ 455.23078.

### Example 17

### 3,9-Dihydroxy-8-((4-(1-hydroxybutyl-2,2-d₂)piperidin-1-yl-4-d)methyl)benzo[5,6]oxepino[4,3-b]benzofuran-7(5H)-one (compound YF017)

The preparation method was the same as that of Example 15, except that YF010 was replaced with YF011 to obtain a final product in the form of a creamy yellow solid. ¹H NMR (600 MHz, DMSO-*d*₆) δ 7.84 (d, *J* = 8.5 Hz, 1H), 7.47 (d, *J* = 8.8 Hz, 1H), 7.12 (d, *J* = 2.5 Hz, 1H), 7.05 (dd, *J* = 8.5, 2.4 Hz, 1H), 6.89 (d, *J* = 8.8 Hz, 1H), 5.16 (s, 2H), 3.95 (s, 2H), 3.41 (s, 1H), 2.81 (s, 2H), 2.08 (s, 2H), 1.77 (s, 2H), 1.46-1.41 (m, 4H), 0.83 (t, *J* = 7.4 Hz, 3H). HRMS(ESI) m/z: Calcd for C₂₆H₂₆D₃NO₆ (M+H)⁺ 455.24220; Found (M + H) ⁺ 455.23078.

### Example 18

### 3,9-Dihydroxy-8-((4-(1-hydroxybutyl-1,2,2-d₃)piperidin-1-yl-4-d)methyl)benzo[5,6]oxepino[4,3-b]benzofuran-7(5H)-one (compound YF018)

The preparation method was the same as that of Example 16, except that YF010 was replaced with YF011 to obtain a final product in the form of a yellowish-white solid. ¹H NMR (600 MHz, DMSO-*d*₆) δ 7.86 (d, *J =* 8.6 Hz, 1H), 7.46 (d, *J =* 8.9 Hz, 1H), 7.12 (d, *J =* 2.5 Hz, 1H), 7.05 (dd, *J* = 8.6, 2.5 Hz, 1H), 6.86 (d, *J =* 8.8 Hz, 1H), 5.14 (s, 2H), 4.00 (s, 2H), 2.82-2.79 (m, 2H), 2.10-2.08 (m, 2H), 1.80-1.77 (m, 2H), 1.40-1.35 (m, 4H), 0.86 (t, *J* = 7.3 Hz, 3H). HRMS(ESI) m/z: Calcd for C₂₆H₂₆D₃NO₆ (M+H)⁺ 455.24220; Found (M + H) ⁺ 455.23078. HRMS(ESI) m/z: Calcd for C₂₆H₂₅D₄NO₆ (M+H)⁺ 456.32400; Found (M + H) ⁺ 456.23861.

### Example 19

### 3,9-Dihydroxy-8-((4-(1-hydroxybutyl)piperidin-2,2,6,6-d₄-1-yl)methyl)benzo[5,6]oxepino[4,3-b]benzofuran-7(5H)-one (compound YF019)

The preparation method was the same as that of Example 15, except that **YF010** was replaced with YF012 to obtain a final product in the form of a yellow solid. ¹H NMR (400 MHz, DMSO-*d*₆) δ 10.62 (s, 1H), 10.37 (s, 1H), 7.80 (d, *J* = 8.5 Hz, 1H), 7.64 (d, *J* = 8.9 Hz, 1H), 7.10 (d, *J* = 8.9 Hz, 1H), 7.06 (d, *J* = 2.4 Hz, 1H), 7.02 (dd, *J* = 8.5, 2.4 Hz, 1H), 5.25 (s, 2H), 3.12-3.10 (m, 1H), 1.79 (d, *J =* 12.7 Hz, 1H), 1.61-1.15 (m, 8H), 0.79 (t, *J* = 6.7 Hz, 3H). HRMS(ESI) m/z: Calcd for C₂₆H₂₃D₆NO₆ (M+H)⁺ 458.23700; Found (M + H) ⁺ 458.53400.

### Example 20

### 3,9-Dihydroxy-8-((4-(1-hydroxybutyl-1-d)piperidin-2,2,6,6-d₄-1-yl)methyl)benzo[5,6]oxepino[4,3-b]benzofuran-7(5H)-one (compound YF020)

The preparation method was the same as that of Example 16, except that **YF010** was replaced with YF012 to obtain a final product in the form of a yellow solid. ¹H NMR (600 MHz, Methanol-*d₄*) δ 8.05 (d, *J* = 8.6 Hz, 1H), 7.59 (d, *J =* 2.4 Hz, 1H), 7.56-7.43 (m, 2H), 6.92 (d, *J* = 8.8 Hz, 1H), 5.25 (s, 2H), 1.86-1.20 (m, 9H), 0.79 (t, *J* = 7.0 Hz, 3H). HRMS(ESI) m/z: Calcd for C₂₆H₂₂D₇NO₆ (M+H)⁺ 459.24300; Found (M + H) ⁺459.25610.

### Example 21

### 3,9-Dihydroxy-8-((4-(1-hydroxybutyl)piperidin-2,2,6,6-d₄-1-yl)methyl-d₂)benzo[5,6]oxepino[4,3-b]benzofuran-7(5H)-one-5,5,10,11-d₄ (compound YF021)

The preparation method was the same as that of Example 15, except that YF010 was replaced with YF013 to obtain a final product in the form of a yellow solid. ¹H NMR (600 MHz, DMSO-*d*₆) δ 7.87 (d, *J* = 8.5 Hz, 1H), 7.11 (d, *J* = 2.4 Hz, 1H), 7.08 (dd, *J =* 8.5, 2.4 Hz, 1H), 3.22-3.19 (m, 1H), 2.10-1.87 (m, 4H), 1.40-1.25 (m, 5H), 0.83 (t, *J* = 7.4 Hz, 3H). HRMS(ESI) m/z: Calcd for C₂₆H₁₉D₁₀NO₆ (M+H)⁺ 462.16600; Found (M + H) ⁺ 462.28556.

### Example 22

### 3,9-Dihydroxy-8-((4-(1-hydroxybutyl-1-d)piperidin-2,2,6,6-d₄-1-yl)methyl-d₂)benzo[5,6]oxepino[4,3-b]benzofuran-7(5H)-one-5,5,10,11-d₄ (compound YF022)

The preparation method was the same as that of Example 16, except that YF010 was replaced with YF013 to obtain a final product in the form of a yellow solid. ¹H NMR (400 MHz, DMSO-*d*₆) δ 7.89 (d, *J=* 8.5 Hz, 1H), 7.12 (d, *J=* 2.4 Hz, 1H), 7.08 (dd, *J* = 8.5, 2.4 Hz, 1H), 2.05-1.74 (m, 4H), 1.37-1.19 (m, 5H), 0.83 (t, *J* = 7.4 Hz, 3H). HRMS(ESI) m/z: Calcd for C₂₆H₁₈D₁₁NO₆ (M+H)⁺ 463.27590; Found (M + H) ⁺ 463.29338.

### Example 23

### 3,9-Dihydroxy-8-((4-(1-hydroxybutyl-2,2-d₂)piperidin-1-yl-1-d)methyl-d₂)benzo[5,6]oxepino[4,3-b]benzofuran-7(5H)-one-5,5,10,11-d₄ (compound YF023)

The preparation method was the same as that of Example 15, except that YF010 was replaced with YF014 to obtain a final product in the form of a pale yellow solid. ¹H NMR (400 MHz, DMSO-*d*₆) δ 7.85 (d, *J* = 8.5 Hz, 1H), 7.12 (d, *J* = 2.5 Hz, 1H), 7.05 (dd, *J =* 8.5, 2.5 Hz, 1H), 3.41 (s, 1H), 2.94-2.89 (m, 2H), 2.11-2.10 (m, 1H), 1.85-1.80 (m, 2H), 1.40-1.37 (m, 4H), 0.83 (*t, J =* 7.4 Hz, 3H). HRMS(ESI) m/z: Calcd for C₂₆H₂₀D₉NO₆ (M+H)⁺ 461.26600; Found (M + H) ⁺ 461.27773.

### Example 24

### 3,9-Dihydroxy-8-((4-(1-hydroxybutyl-1,2,2-d₃)piperidin-1-yl-1-d)methyl-d₂)benzo[5,6]oxepino[4,3-b]benzofuran-7(5H)-one-5,5,10,11-d₄ (compound YF024)

The preparation method was the same as that of Example 16, except that YF010 was replaced with YF014 to obtain a final product in the form of a yellow solid. ¹H NMR (600 MHz, DMSO-*d*₆) δ 10.75 (s, 1H), 10.58 (s, 1H), 7.86(d, *J* = 8.5 Hz, 1H), 7.12 (d, *J* = 2.5 Hz, 1H), 7.05 (dd, *J* = 8.5, 2.5 Hz, 1H), 2.82-2.78 (m, 2H), 2.00-1.97 (m, 2H), 1.78-1.75 (m, 2H), 1.32-1.24 (m, 4H), 0.84 (t, *J* = 7.3 Hz, 3H). HRMS(ESI) m/z: Calcd for C₂₆H₁₉D₁₀NO₆ (M+H)⁺ 462.58260; Found (M + H) ⁺ 462.29338.

### Example 25

### 10-(tert-Butyl)-3,8-dihydroxy-7-(piperidin-1-ylmethyl-d₂)-6H-benzofuran[3,2-c]chromen-6-one hydrochloride (compound YF025)

At room temperature, 10-(tert-butyl)-3,8-dihydroxy-7-(piperidin-1-ylmethyl-*d₂*)-6H-benzofuran[3,2-c]chromen-6-one (compound YF001) was dissolved in a mixed solvent of DCM and methanol, and a solution of excess hydrogen chloride in ethyl acetate (3.5 M) was added. The mixture was reacted at room temperature for 1 h, concentrated, and then slurried with DCM and filtered under vacuum to obtain 10-(tert-butyl)-3,8-dihydroxy-7-((piperidin-1-yl-2,2,6,6-*d*₄)methyl-*d*₂)-6H-benzofuran[3,2-c]chromen-6-one hydrochloride in the form of a white solid. ¹H NMR (600 MHz, DMSO-*d*₆) δ 10.99 (s, 1H), 10.43 (s, 1H), 8.64 (s, 1H), 7.98 (d, *J* = 8.6 Hz, 1H), 7.13 (s, 1H), 7.02 (dd, *J* = 8.6, 2.2 Hz, 1H), 6.98 (d, *J* = 2.2 Hz, 1H), 3.44 (d, *J* = 11.9 Hz, 2H), 3.15-3.09 (m, 2H), 1.86-1.83 (m, 2H), 1.74-1.62 (m, 3H), 1.52 (s, 9H), 1.51-1.42 (s, 1H). HRMS(ESI) m/z: Calcd for C₂₅H₂₆D₂ClNO₅ (M+H)⁺ 460.18907; Found (M + H-HCl) ⁺ 424.20770.

### Example 26

### 10-(tert-Butyl)-3,8-dihydroxy-7-((piperidin-1-yl-2,2,6,6-d₄)methyl)-6H-benzofuran[3,2-c]chromen-6-one hydrochloride (compound YF026)

The preparation method was the same as that of Example 25, except that YF001 was replaced with YF002 to obtain a final product in the form of a white solid. ¹H NMR (600 MHz, DMSO-*d*₆) δ 11.03 (s, 1H), 10.47 (s, 1H), 8.64 (s, 1H), 7.97 (d, *J* = 8.6 Hz, 1H), 7.13 (s, 1H), 7.02 (dd, *J* = 8.6, 2.1 Hz, 1H), 6.98 (d, *J* = 2.1 Hz, 1H), 4.81 (d, *J* = 5.3 Hz, 2H), 1.87-1.77 (m, 2H), 1.67-1.62 (m, 3H), 1.51 (s, 9H), 1.49-1.40 (m, 1H). HRMS(ESI) m/z: Calcd for C₂₅H₂₄D₄ClNO₅ (M+H)⁺ 462.20472; Found (M + H-HCl) ⁺ 426.22074.

### Example 27

### 10-(tert-Butyl)-3,8-dihydroxy-7-((piperidin-1-yl-2,2,6,6-d₄)methyl-d₂)-6H-benzofuran[3,2-c]chromen-6-one hydrochloride (compound YF027)

The preparation method was the same as that of Example 25, except that YF001 was replaced with YF003 to obtain a final product in the form of a white solid. ¹H NMR (600 MHz, DMSO-*d*₆) δ 11.00 (s, 1H), 10.44 (s, 1H), 8.64 (s, 1H), 7.97 (d, *J* = 8.6 Hz, 1H), 7.13 (s, 1H), 7.02 (dd, *J* = 8.6, 2.2 Hz, 1H), 6.97 (d, *J* = 2.2 Hz, 1H), 1.82-1.79 (m, 2H), 1.67-1.63 (m, 3H), 1.51 (s, 9H), 1.48-1.40 (m, 1H). HRMS(ESI) m/z: Calcd for C₂₅H₂₂D₆ClNO₅ (M+H)⁺ 464.22037; Found (M + H-HCl) ⁺ 428.23172.

### Example 28

### 10-(tert-Butyl)-3,8-dihydroxy-7-((piperidin-1-yl-d₁₀)methyl)-6H-benzofuran[3,2-c]chromen-6-one hydrochloride (compound YF028)

The preparation method was the same as that of Example 25, **except that** YF001 was replaced with YF004 to obtain a final product in the form of a white solid. ¹H NMR (600 MHz, DMSO-*d*₆) δ 10.97 (s, 1H), 10.30 (s, 1H), 8.74 (s, 1H), 7.89 (d, *J* = 8.7Hz, 1H), 7.00 (s, 1H), 7.05 (dd, *J* = 8.7, 2.1 Hz, 1H), 6.96 (d, *J* = 2.1 Hz, 1H), 4.86 (d, *J =* 5.5 Hz, 2H), 1.51 (s, 9H). HRMS(ESI) m/z: Calcd for C₂₅H₁₈D₁₀ClNO₅ (M+H)⁺ 467.22801; Found (M + H-HCl) ⁺ 432.55100.

### Example 29

### 10-(tert-Butyl)-3,8-dihydroxy-7-((piperidin-1-yl-d₁₀)methyl-d₂)-6H-benzofuran[3,2-c]chromen-6-one hydrochloride (compound YF029)

The preparation method was the same as that of Example 25, except that YF001 was replaced with YF005 to obtain a final product in the form of a white solid. ¹H NMR (600 MHz, DMSO-*d*₆) δ 10.89 (s, 1H), 10.14 (s, 1H), 8.77 (s, 1H), 7.92 (d, *J*=8.7 Hz, 1H), 7.14 (s, 1H), 7.00 (dd, *J*=8.7, 2.3 Hz, 1H), 6.87 (d, *J*=2.3 Hz, 1H), 1.60(s, 9H). HRMS(ESI) m/z: Calcd for C₂₅H₁₆D₁₂ClNO₅ (M+H)⁺ 470.24096; Found (M + H-HCl) ⁺ 434.56620.

### Example 30

### 8-((4-(3-Chlorophenyl)piperidin-1-yl)methyl-d₂)-3,9-dihydroxybenzo[5,6]oxepino[4,3-b]benzofuran-7(5H)-one hydrochloride (compound YF030)

At room temperature, 8-((4-(3-chlorophenyl)piperidin-1-yl)methyl-*d*₂)-3,9-dihydroxybenzo[5,6]oxepino[4,3-b]benzofuran-7(5H)-one (YF006) was dissolved in ethyl acetate or dichloromethane, and a solution of excess hydrogen chloride in ethyl acetate was added. The mixture was reacted at room temperature for 1 h or overnight, and then either directly concentrated to dryness or filtered and then dried to obtain 8-((4-(3-chlorophenyl)piperidin-1-yl)methyl-*d*₂)-3,9-dihydroxybenzo[5,6]oxepino[4,3-b]benzofuran-7(5H)-one hydrochloride in the form of a white solid. ¹H NMR (600 MHz, DMSO-*d*₆) δ 10.64 (s, 1H), 10.43 (s, 1H), 9.57 (s, 1H), 7.88 (d, *J* = 8.5 Hz, 1H), 7.73 (d, *J* = 8.9 Hz, 1H), 7.35 (d, *J =* 7.7 Hz, 1H), 7.31-7.26 (m, 2H), 7.20 (d, *J =* 7.9 Hz, 1H), 7.17 (d, *J* = 8.8 Hz, 1H), 7.13 (d, *J* = 2.4 Hz, 1H), 7.08 (dd, *J* = 8.5, 2.4 Hz, 1H), 5.33 (s, 2H), 3.61 (d, *J* = 12.0 Hz, 2H), 3.34-3.28 (m, 2H), 2.96-2.86 (m,1H), 2.12-2.08 (m, 2H), 2.01-1.91 (m, 2H). HRMS(ESI) m/z: Calcd for C₂₈H₂₃D₂Cl₂NO₅ (M+H)⁺ 528.13445; Found (M + H-HCl) ⁺ 492.15334.

### Example 31

### 8-((4-(3-Chlorophenyl)piperidin-1-yl-2,2,6,6-d₄)methyl)-3,9-dihydroxybenzo[5,6]oxepino[4,3-b]benzofuran-7(5H)-one hydrochloride (compound YF031)

The preparation method was the same as that of Example 30, except that YF006 was replaced with YF007 to obtain a final product in the form of a white solid. ¹H NMR (600 MHz, DMSO-*d*₆) δ 10.66 (s, 1H), 10.46 (s, 1H), 9.78 (s, 1H), 7.87 (d, *J* = 8.5 Hz, 1H), 7.72 (d, *J* = 8.8 Hz, 1H), 7.35 (t, *J* = 7.6 Hz, 1H), 7.28 (d, *J* = 8.1 Hz, 2H), 7.19 (dd, *J* = 10.5, 8.1 Hz, 2H), 7.12 (d, *J* = 2.5 Hz, 1H), 7.08 (dd, *J* = 8.5, 2.3 Hz, 1H), 5.34 (s, 2H), 4.79 (s, 2H), 2.92-2.88 (m, 1H), 2.23-2.05 (m, 2H), 1.98-1.93 (m, 2H). HRMS(ESI) m/z: Calcd for C₂₈H₂₁D₄Cl₂NO₅ (M+H)⁺ 530.15010; Found (M + H-HCl) ⁺ 494.26536.

### Example 32

### 8-((4-(3-Chlorophenyl)piperidin-1-yl-2,2,6,6-d₄)methyl-d₂)-3,9-dihydroxybenzo[5,6]oxepino[4,3-b]benzofuran-7(5H)-one hydrochloride (compound YF032)

The preparation method was the same as that of Example 30, except that YF006 was replaced with **YF008** to obtain a final product in the form of a white solid. ¹H NMR (600 MHz, DMSO-*d*₆) δ 10.65 (s, 1H), 10.45 (s, 1H),9.76 (s, 1H), 7.87 (d, *J* = 8.5 Hz, 1H), 7.72 (d, *J* = 8.9 Hz, 1H), 7.35 (t, *J* = 7.6 Hz, 1H), 7.28 (dd, *J* = 8.4, 1.8 Hz, 2H), 7.19 (dd, *J =* 10.0, 8.0 Hz, 2H), 7.12 (d, *J =* 2.4 Hz, 1H), 7.09 (dd, *J* = 8.5*,* 2.5 Hz, 1H), 5.38 (s, 2H), 2.93-2.90 (m, 1H), 2.13-2.10 (m, 2H), 1.95-1.92 (m, 2H). HRMS(ESI) m/z: Calcd for C₂₈H₁₉D₆Cl₂NO₅ (M+H)⁺ 532.16575; Found (M ⁺ H-HCl) ⁺ 496.17764.

### Example 33

### 8-((4-(3-Chlorophenyl)piperidin-1-yl-2,2,6,6-d₄)methyl-d₂)-3,9-dihydroxybenzo[5,6]oxepino[4,3-b]benzofuran-7(5H)-one-5,5,10,11-d₄hydrochloride (compound YF033)

The preparation method was the same as that of Example 30, except that YF006 was replaced with YF009 to obtain a final product in the form of a white solid. ¹H NMR (600 MHz, Chloroform-d) δ 10.30 (s, 1H), 10.01 (s, 1H), 8.38 (s, 1H), 7.44 (d, *J* = 8.8 Hz, 1H), 7.35-7.27 (m, 2H), 7.24 (dd, *J* = 2.1, 1.5 Hz, 1H), 7.23-7.19 (m, 1H), 6.81 (dd, *J* = 8.7, 1.9 Hz, 1H), 6.78 (d, *J* = 2.0 Hz, 1H), 2.97-2.86 (m, 1H), 2.24-2.21 (m, 4H). HRMS(ESI) m/z: Calcd for C₂₈H₁₅D₁₀Cl₂NO₅ (M+H)⁺ 536.17404; Found (M + H-HCl)⁺ 500.18920.

### Example 34

### 8-((4-Butanoyl)piperidin-1-ylmethyl-d₂)-3,9-dihydroxybenzo[5,6]oxepino[4,3-b]benzofuran-7(5H)-one hydrochloride (compound YF034)

The preparation method was the same as that of Example **30,** except that YF006 was replaced with YF010 to obtain a final product in the form of a pale brown solid. ¹H NMR (600 MHz, DMSO-*d*₆) δ 10.66 (s, 1H), 10.45 (s, 1H), 9.60 (s, 1H), 7.86 (d, *J* = 8.5 Hz, 1H), 7.71 (d, *J =* 8.9 Hz, 1H), 7.17 (d, *J* = 8.9 Hz, 1H), 7.12 (d, *J* = 2.5 Hz, 1H), 7.08 (dd, *J* = 8.5, 2.3 Hz, 1H), 5.34 (s, 2H), 3.42 (d, *J* = 12.2 Hz, 2H), 3.21-3.10 (m, 2H), 2.70-2.66 (m, 1H), 2.47 (t, *J =* 7.1 Hz, 2H), 2.02-1.94 (m, 2H), 1.84-1.79 (m, 2H), 1.46 (q, *J =* 7.3 Hz, 2H), 0.83 (t, *J =* 7.4 Hz, 3H). HRMS(ESI) m/z: Calcd for C₂₆H₂₆D₂ClNO₆ (M+H)⁺ 488.18399; Found (M + H-HCl) ⁺ 452.20287.

### Example 35

### 8-((4-(Butanoyl-2,2-d₂)piperidin-1-yl-4-d)methyl)-3,9-dihydroxybenzo[5,6]oxepino[4,3-b]benzofuran-7(5H)-one hydrochloride (YF035)

The preparation method was the same as that of Example 30, except that YF006 was replaced with YF011 to obtain a final product in the form of a yellowish-white solid. ¹H NMR (400 MHz, DMSO-*d*₆) δ 10.66 (s, 1H), 10.46 (s, 1H), 9.51 (s, 1H), 7.86 (d, *J =* 8.5 Hz, 1H), 7.71 (d, *J =* 8.9 Hz, 1H), 7.17 (d, *J* = 8.9 Hz, 1H), 7.11 (s, 1H), 7.08 (dd, *J* = 8.5, 2.4 Hz, 1H), 5.33 (s, 2H), 4.72 (s, 2H), 3.42 (d, *J* = 11.7 Hz, 2H), 3.20-3.11 (m, 2H), 2.00-1.95 (m, 2H), 1.82-1.76 (m, 2H), 1.44 (q, *J* = 7.2 Hz, 2H), 0.82 (t, *J* = 7.4 Hz, 3H). HRMS(ESI) m/z: Calcd for C₂₆H₂₅D₃ClNO₆ (M+H)⁺ 489.19181; Found (M + H-HCl) ⁺ 453.56020.

### Example 36

### 8-((4-Butanoylpiperidin-1-yl-2,2,6,6-d₄)-methyl-d₂)-3,9-dihydroxybenzo[5,6]oxepino[4,3-b]benzofuran-7(5H)-one hydrochloride (YF036)

The preparation method was the same as that of Example 30, except that **YF006** was replaced with YF012 to obtain a final product in the form of a white solid. ¹H NMR (400 MHz, DMSO-*d*₆) δ 10.65 (s, 1H), 10.42 (s, 1H), 9.32 (s, 1H), 7.87 (d, *J* = 8.5 Hz, 1H), 7.72 (d, *J* = 8.9 Hz, 1H), 7.20-7.11 (m, 2H), 7.08 (dd, *J* = 8.5, 2.4 Hz, 1H), 5.33 (s, 2H), 2.72-2.70 (m, 1H), 2.47 (t, *J =* 7.2 Hz, 2H), 2.03-1.96 (m, 2H), 1.79-1.74 (m, 2H), 1.47 (q, *J* = 7.3 Hz, 2H), 0.83 (t*, J =* 7.4 Hz, 3H). HRMS(ESI) m/z: Calcd for C₂₆H₂₂D₆ClNO₆ (M+H)⁺ 492.99760; Found (M + H-HCl) ⁺ 456.05245.

### Example 37

### 8-((4-Butanoylpiperidin-1-yl-2,2,6,6-d₄)-methyl-d₂)-3,9-dihydroxybenzo[5,6]oxepino[4,3-b]benzofuran-7(5H)-one-5,5,10,11-d₄ hydrochloride (YF037)

The preparation method was the same as that of Example 30, except that YF006 was replaced with YF013 to obtain a final product in the form of a white solid. ¹H NMR (400 MHz, DMSO-*d*₆) δ 11.00 (s, 1H), **10**.64 (s, 1H), 9.03 (s, 1H), 7.80 (d, *J* = 8.5 Hz, 1H), 7.25 (d, *J* = 2.4 Hz, 1H), 7.10 (dd, *J* = 8.5, 2.4 Hz, 1H), 2.83-2.79 (m, 1H), 2.48 (t, *J* = 7.1 Hz, 2H), 2.10-2.00 (m, 2H), 1.87-1.78 (m, 2H), 1.47 (q, *J =* 7.3 Hz, 2H), 0.89 (t, *J* = 7.3 Hz, 3H). HRMS(ESI) m/z: Calcd for C₂₆H₁₈D₁₀ClNO₆ (M+H)⁺ 496.22300; Found (M + H-HCl) ⁺ 460.56400.

### Example 38

### 8-((4-(Butanoyl-2,2-d₂)piperidin-1-yl-4-d)methyl-d₂)-3,9-dihydroxybenzo[5,6]oxepino[4,3-b]benzofuran-7(5H)-one-5,5,10,11-d₄ hydrochloride (YF038)

The preparation method was the same as that of Example 30, except that YF006 was replaced with YF014 to obtain a final product in the form of a white solid. ¹H NMR (400 MHz, DMSO-*d*₆) δ 10.86 (s, 1H), 10.32 (s, 1H), 9.25 (s, 1H), 7.78 (d, *J* = 8.6 Hz, 1H), 7.13 (d, *J* = 2.4 Hz, 1H), 7.10 (dd, *J* = 8.6, 2.4 Hz, 1H), 3.43- 3.42(m, 2H), 3.19-3.11 (m, 2H), 1.99-1.96 (m, 2H), 1.81 (d*, J =* 13.1 Hz, 2H), 1.48 (q*, J* = 7.2 Hz, 2H), 0.89 (t, *J* = 7.3Hz, 3H). HRMS(ESI) m/z: Calcd for C₂₆H₁₉D₆ClNO₆ (M+H)⁺ 495.21700; Found (M+H-HCl) ⁺ 459.24700.

### Example 39

### 3,9-Dihydroxy-8-((4-(1-hydroxybutyl)piperidin-1-yl)methyl-d₂)benzo[5,6]oxepino[4,3-b]benzofuran-7(5H)-one hydrochloride (compound YF039)

The preparation method was the same as that of Example 30, except that YF006 was replaced with YF015 to obtain a final product in the form of a white solid. ¹H NMR (600 MHz, DMSO-*d*₆) δ 10.66 (s, 1H), 10.41 (s, 1H), 9.14 (s, 1H), 7.86 (d, *J* = 8.5 Hz, 1H), 7.70 (d, *J* = 8.9 Hz, 1H), 7.16 (d, *J* = 8.9 Hz, 1H), 7.12 (d, *J =* 2.5 Hz, 1H), 7.08 (dd, *J =* 8.5, 2.4 Hz, 1H), 5.31 (s, 2H), 3.43-3.39 (m, 2H), 3.20-3.07 (m, 3H), 1.88-1.83 (m, 1H), 1.74-1.37 (m, 5H), 1.36-1.19 (m, 3H), 0.87 (t, *J* = 6.9 Hz, 3H). HRMS(ESI) m/z: Calcd for C₂₆H₂₈D₂ClNO₆ (M+H)⁺ 490.19120; Found (M + H-HCl) ⁺ 454.22300.

### Example 40

### 3,9-Dihydroxy-8-((4-(1-hydroxybutyl-1-d)piperidin-1-yl)methyl-d₂)benzo[5,6]oxepino[4,3-b]benzofuran-7(5H)-one hydrochloride (compound YF040)

The preparation method was the same as that of Example 30, except that YF006 was replaced with YF016 to obtain a final product in the form of a white solid. ¹H NMR (400 MHz, DMSO-*d*₆) δ 10.62 (s, 1H), 10.37 (s, 1H), 9.10 (s, 1H), 7.80 (d, *J* = 8.5 Hz, 1H), 7.64 (d, *J =* 8.9 Hz, 1H), 7.10 (d, *J =* 8.9 Hz, 1H), 7.06 (d, *J =* 2.4 Hz, 1H), 7.02 (dd, *J* = 8.5, 2.4 Hz, 1H), 5.25 (s, 2H), 3.34 (s, 2H), 3.02 (s, 2H), 1.79-1.76 (m, 1H), 1.69-1.04 (m, 8H), 0.79 (t, *J* = 6.8 Hz, 3H). HRMS(ESI) m/z: Calcd for C₂₆H₂₇D₆ClNO₆ (M+H-HCl)⁺ 491.19000; Found (M + H) ⁺ 455.23081.

### Example 41

### 3,9-Dihydroxy-8-((4-(1-hydroxybutyl-2,2-d₂)piperidin-1-yl-4-d)methyl)benzo[5,6]oxepino[4,3-b]benzofuran-7(5H)-one hydrochloride (compound YF041)

The preparation method was the same as that of Example 30, except that YF006 was replaced with YF017 to obtain a final product in the form of a white solid. ¹H NMR (600 MHz, DMSO-*d*₆) δ 10.66 (s, 1H), 10.50 (s, 1H), 9.51 (s, 1H), 7.86 (d, *J* = 8.5 Hz, 1H), 7.70 (d, *J =* 8.9 Hz, 1H), 7.16 (d, *J =* 8.9 Hz, 1H), 7.11 (d, *J =* 2.4 Hz, 1H), 7.07 (dd, *J =* 8.5, 2.4 Hz, 1H), 5.32 (s, 2H), 4.71 (s, 2H), 3.43-3.40 (m, 3H), 3.17-3.12 (m, 2H), 1.98 (s, 2H), 1.81-1.78 (m, 2H), 1.38 (q*, J =* 7.2 Hz, 2H), 0.81 (*t, J =* 7.4 Hz, 3H). (ESI) m/z: Calcd for C₂₆H₂₇D₃ClNO₆ (M+H)⁺ 494.20746; Found (M + H-HCl) ⁺ 455.23100.

### Example 42

### 3,9-Dihydroxy-8-((4-(1-hydroxybutyl-1,2,2-d₃)piperidin-1-yl-4-d)methyl)benzo[5,6]oxepino[4,3-b]benzofuran-7(5H)-one hydrochloride (compound YF042)

The preparation method was the same as that of Example 30, except that YF006 was replaced with YF018 to obtain a final product in the form of a white solid. ¹H NMR (600 MHz, DMSO-*d*₆) δ 11.00 (s, 1H), 10.60 (s, 1H), 9.85 (s, 1H), 7.91 (d, *J* = 8.5 Hz, 1H), 7.71 (d, *J =* 8.8 Hz, 1H), 7.15 (d, *J =* 8.8 Hz, 1H), 7.11 (d, *J =* 2.4 Hz, 1H), 7.06 (dd, *J =* 8.5, 2.4 Hz, 1H), 5.33 (s, 2H), 4.68 (s, 2H), 3.50-3.45 (m, 2H), 3.17-3.12 (m, 2H), 1.90-1.80 (m, 4H), 1.35 (q, *J =* 7.3 Hz, 2H), 0.85 (t, *J =* 7.3 Hz, 3H). HRMS(ESI) m/z: Calcd for C₂₆H₂₆D₄ClNO₆ (M+H)⁺ 492.21529; Found (M + H-HCl) ⁺ 456.23541.

### Example 43

### 3,9-Dihydroxy-8-((4-(1-hydroxybutyl)piperidin-2,2,6,6-d₄-1-yl)methyl)benzo[5,6]oxepino[4,3-b]benzofuran-7(5H)-one hydrochloride (compound YF043)

The preparation method was the same as that of Example 30, except that YF006 was replaced with YF019 to obtain a final product in the form of a white solid. ¹H NMR (600 MHz, DMSO-*d*₆) δ 10.66 (s, 1H), 10.42 (s, 1H), 9.15 (s, 1H), 7.87 (d, *J* = 8.5 Hz, 1H), 7.70 (d, *J* = 8.9 Hz, 1H), 7.17 (d, *J* = 8.9 Hz, 1H), 7.13 (d, *J =* 2.5 Hz, 1H), 7.09 (dd, *J =* 8.5, 2.4 Hz, 1H), 5.37 (s, 2H), 3.23-3.17 (m, 1H), 1.88-1.86 (m, 1H), 1.73-1.19 (m, 8H), 0.86 (t*, J =* 6.9 Hz, 3H). HRMS(ESI) m/z: Calcd for C₂₆H₂₄D₆ClNO₆ (M+H)⁺ 493.21400; Found (M + H-HCl) ⁺ 458.55560.

### Example 44

### 3,9-Dihydroxy-8-((4-(1-hydroxybutyl-1-d)piperidin-2,2,6,6-d₄-1-yl)methyl)benzo[5,6]oxepino[4,3-b]benzofuran-7(5H)-one hydrochloride (compound YF044)

The preparation method was the same as that of Example 30, except that YF006 was replaced with YF020 to obtain a final product in the form of a white solid. ¹H NMR (600 **MHz,** DMSO-*d*₆) δ 10.64 (s, 1H), 10.41 (s, 1H), 9.35 (s, 1H), 7.87 (d, *J* = 8.5 Hz, 1H), 7.71 (d, *J* = 8.9 Hz, 1H), 7.16 (d, *J =* 8.9 Hz, 1H), 7.12 (d, *J =* 2.4 Hz, 1H), 7.08 (dd, *J* = 8.5, 2.4 Hz, 1H), 5.33 (s, 2H), 1.95-1.37 (m, 9H), 0.85 (t, *J* = 7.1, 3H). HRMS(ESI) m/z: Calcd for C₂₆H₂₃D₇ClNO₆ (M+H)⁺ 494.22000; Found (M + H-HCl) ⁺459.26617.

### Example 45

### 3,9-Dihydroxy-8-((4-(1-hydroxybutyl)piperidin-2,2,6,6-d₄-1-yl)methyl-d₂)benzo[5,6]oxepino[4,3-b]benzofuran-7(5H)-one-5,5,10,11-d₄ hydrochloride (compound YF045)

The preparation method was the same as that of Example 30, except that YF006 was replaced with YF021 to obtain a final product in the form of a white solid. ¹H NMR (600 MHz, DMSO-*d*₆) δ 10.70 (s, 1H), 10.52 (s, 1H), 9.00 (s, 1H), 7.89 (d, *J* = 8.6 Hz, 1H), 7.12 (d, *J* = 2.4 Hz, 1H), 7.09 (dd, *J* = 8.6, 2.4 Hz, 1H), 3.20-3.17 (m, 1H), 2.00-1.97 (m, 2H), 1.79-1.74 (m, 2H), 1.30-1.10 (m, 5H), 0.83 (t, *J* = 7.4 Hz, 3H). HRMS(ESI) m/z: Calcd for C₂₆H₂₀D₁₀ClNO₆ (M+H)⁺ 498.26224; Found (M + H-HCl) ⁺ 462.28600.

### Example 46

### 3,9-Dihydroxy-8-((4-(1-hydroxybutyl-1-d)piperidin-2,2,6,6-d₄-1-yl)methyl-d₂)benzo[5,6]oxepino[4,3-b]benzofuran-7(5H)-one-5,5,10,11-d₄ hydrochloride (compound YF046)

The preparation method was the same as that of Example 30, except that YF006 was replaced with YF022 to obtain a final product in the form of a white solid. ¹H NMR (400 MHz, DMSO-*d*₆) δ 11.00 (s, 1H), 10.62 (s, 1H), 8.87 (s, 1H), 7.88 (d, *J* = 8.5 Hz, 1H), 7.12 (d, *J* = 2.4 Hz, 1H), 7.08 (dd, *J* = 8.5, 2.4 Hz, 1H), 2.06-1.77 (m, 4H), 1.38-1.16 (m, 5H), 0.83 (t, *J* = 7.4 Hz, 3H). HRMS(ESI) m/z: Calcd for C₂₆H₁₉D₁₁ClNO₆ (M+H)⁺ 499.27006; Found (M + H-HCl)⁺ 463.30027.

### Example 47

### 3,9-Dihydroxy-8-((4-(1-hydroxybutyl-2,2-d₂)piperidin-1-yl-1-d)methyl-d₂)benzo[5,6]oxepino[4,3-b]benzofuran-7(5H)-one-5,5,10,11-d₄ hydrochloride (compound YF047)

The preparation method was the same as that of Example 30, except that YF006 was replaced with YF023 to obtain a final product in the form of an off-white solid. ¹H NMR (400 MHz, DMSO-*d*₆) δ 10.80 (s, 1H), 10.60 (s, 1H), 8.97 (s, 1H), 7.86 (d, *J =* 8.5 Hz, 1H), 7.11 (d, *J* = 2.4 Hz, 1H), 7.07 (dd, *J* = 8.5, 2.4 Hz, 1H), 3.45-3.17 (m, 5H), 2.00-1.95 (m, 2H), 1.79-1.76 (m, 2H), 1.18 (q, *J =* 7.2 Hz, 2H), 0.83 (t*, J =* 7.3 Hz, 3H). HRMS(ESI) m/z: Calcd for C₂₆H₂₁D₉ClNO₆ (M+H)⁺ 497.25441; Found (M + H-HCl) ⁺ 461.27560.

### Example 48

### 3,9-Dihydroxy-8-((4-(1-hydroxybutyl-1,2,2-d₃)piperidin-1-yl-1-d)methyl-d₂)benzo[5,6]oxepino[4,3-b]benzofuran-7(5H)-one-5,5,10,11-d₄ hydrochloride (compound YF048)

The preparation method was the same as that of Example 30, except that YF006 was replaced with YF024 to obtain a final product in the form of a white solid. ¹H NMR (600 MHz, DMSO-*d*₆) δ 10.82 (s, 1H), 10.71 (s, 1H), 9.12 (s, 1H), 7.90 (d, *J* = 8.5 Hz, 1H), 7.11 (d, *J* = 2.4 Hz, 1H), 7.06 (dd, *J =* 8.5, 2.4 Hz, 1H), 3.43-3.34 (m, 2H), 3.10-3.06 (m, 2H), 1.81-1.77 (m, 2H), 1.66-1.61 (m,2H), 1.19 (q, *J =* 7.3 Hz, 2H), 0.84 (t, *J* = 7.3 Hz, 3H). HRMS(ESI) m/z: Calcd for C₂₆H₂₀D₁₀ClNO₆ (M+H)⁺ 498.26224; Found (M + H-HCl)⁺ 462.28556.

### Example 49: In Vitro Pharmacodynamic Evaluation of Compounds of the Present Disclosure Against Mycobacterium Tuberculosis

In this example, the minimum inhibitory concentration (MIC) of the compounds of the present disclosure against the standard strain of *Mycobacterium tuberculosis* (*Mycobacterium tuberculosis* H37Rv) was determined.

### 1.1 Experimental materials

The compounds of the present disclosure and non-deuterated control compounds A, B, C, and D (their structures are shown below); *Mycobacterium tuberculosis* H37Rv (cultured in Middlebrook 7H9 culture medium), provided by Shanghai Pulmonary Hospital.

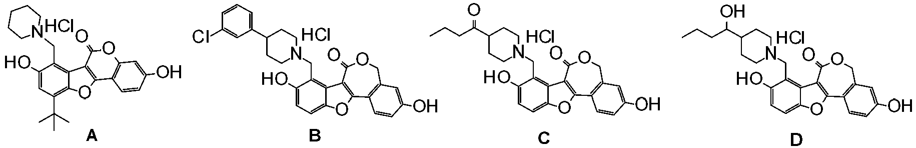

### 1.2 Preparation of test compounds

The test substance was completely dissolved in dimethyl sulfoxide (DMSO), and a stock solution at a concentration of 1 mg/mL was prepared and sterilized by filtration. The stock solution was serially diluted with Middlebrook 7H9 culture medium as needed to obtain test compound solutions at different concentrations, and the solutions were added to a 96-well culture plate at 100 µL/well.

### 1.3 Experimental methods

*Mycobacterium tuberculosis* H37Rv (standard strain) in the logarithmic growth phase was diluted with Middlebrook 7H9 culture medium to a specific turbidity OD600 = 1.0 (about 5 × 10⁶ CFU/mL). 200 µL of the dilution was diluted to 10 mL with the Middlebrook 7H9 culture medium to obtain a bacterial solution containing H37Rv. 100 µL of the bacterial solution containing H37Rv was added to the above culture plate containing the test compound to obtain an experimental group. The final concentrations of the test compounds were 4 µg/mL, 2 µg/mL, 1 µg/mL, 0.5 µg/mL, 0.25 µg/mL, 0.125 µg/mL, 0.0625 µg/mL, 0.03125 µg/mL, 0.0156 µg/mL, 0.0078 µg/mL, and 0.0039 µg/mL, respectively.

The following drug-free control groups were prepared respectively: a 100% bacterial inoculation amount group (100 µL diluted H37Rv bacterial solution + 100 µL Middlebrook 7H9 culture medium), a 10% bacterial inoculation amount group (10 µL diluted H37Rv bacterial solution + 190 µL Middlebrook 7H9 culture medium), and a non-inoculated group (200 µL Middlebrook 7H9 culture medium).

After 15 days of incubation, the size of the bacterial colonies was visually observed, and the minimum inhibitory concentration (MIC₉₀) was defined as the lowest concentration of the compound at which the colony size was not greater than that of the 10% bacterial inoculation amount drug-free control group, indicating 90% inhibition of the growth of *Mycobacterium tuberculosis* H37Rv. The activity results are shown in Table 1.

**Table 1. MIC₉₀ values of the compounds of the present disclosure against Mycobacterium tuberculosis H37Rv**

| Compound | MIC₉₀ (µg/mL) | Compound | MIC₉₀ (µg/mL) | Compound | MIC₉₀ (µg/mL) | Compound | MIC₉₀ (µg/mL) |
|---|---|---|---|---|---|---|---|
| A | ≤0.0039 | B | 0.125 | C | 0.0625 | D | 0.0156 |
| YF001 | ≤0.0039 | YF002 | ≤0.0039 | YF003 | ≤0.0039 | YF004 | ≤0.0039 |
| YF005 | ≤0.0039 | YF006 | 0.125 | YF007 | 0.125 | YF008 | 0.0625 |
| YF009 | 0.125 | YF010 | 0.0625 | YF011 | 0.0625 | YF012 | 0.03125 |
| YF013 | 0.0625 | YF014 | 0.0625 | YF015 | 0.0156 | YF016 | 0.0156 |
| YF017 | 0.0156 | YF018 | 0.0156 | YF019 | 0.0078 | YF020 | 0.0156 |
| YF021 | 0.0156 | YF022 | 0.0156 | YF023 | 0.0156 | YF024 | 0.0156 |
| YF025 | ≤0.0039 | YF026 | ≤0.0039 | YF027 | ≤0.0039 | YF028 | ≤0.0039 |
| YF029 | ≤0.0039 | YF030 | 0.125 | YF031 | 0.125 | YF032 | 0.0625 |
| YF033 | 0.125 | YF034 | 0.0625 | YF035 | 0.0625 | YF036 | 0.03125 |
| YF037 | 0.0625 | YF038 | 0.0625 | YF039 | 0.0156 | YF040 | 0.0156 |
| YF041 | 0.0156 | YF042 | 0.0156 | YF043 | 0.0156 | YF044 | 0.0078 |
| YF045 | 0.0156 | YF046 | 0.0156 | YF047 | 0.0156 | YF048 | 0.0156 |

As can be seen from Table 1, the compounds of the present disclosure show relatively good resistance against *Mycobacterium tuberculosis.*

### Example 50: Pharmacokinetic Evaluation of Deuterated Compounds in Mice

In this example, the pharmacokinetic parameters and lung tissue distribution of the deuterated compounds YF027, YF032, YF036, and YF044 and their respective non-deuterated control compounds (A, B, C, and D) were determined.

### 1.1 Experimental animals

Balb/C mice, male (1 group at each time point, 5 mice per group, 55 mice per compound in total), 7-8 weeks old, weighing about 20 g, animal source: Beijing Vital River Laboratory Animal Technology Co., Ltd.

### 1.2 Preparation of test samples

The test compound was completely dissolved in an 8% Tween-80 aqueous solution. A 10 mg/mL drug liquid was prepared for intraperitoneal injection and intragastric administration.

### 1.3 Experimental methods

Single intragastric administration was performed at a dose of 100 mg/kg. The pharmacokinetic differences between the deuterated compounds YF027, YF032, YF036, and YF044 and their respective non-deuterated controls A, B, C, and D were compared.

The mice were fasted overnight before administration. In the experiment, orbital venous blood and lung tissue were collected from the mice at time points of no administration group (0 min) and 10 min, 20 min, 30 min, 1 h, 2 h, 3 h, 4 h, 6 h, 8 h, and 24 h after intragastric administration, and about 300 µL of blood was collected from the eyeballs each time.

The collected blood sample was placed in an anticoagulation tube, and quickly inverted and mixed well. The tube was temporarily placed on ice after being labeled. The blood samples temporarily stored on ice were subjected to low-temperature centrifugation within 1 h at 4 °C at 3000 rpm for 10 min. The supernatant obtained after centrifugation was plasma. The plasma was transferred to a new centrifuge tube and stored in a refrigerator at -80 °C for subsequent detection.

The lung tissues of the mice were collected, washed with pre-cooled normal saline to remove the residual blood and tissue fluid, dried with absorbent paper, weighed with a balance, and placed into a centrifuge tube. The tubes were marked and stored in a refrigerator at -80 °C.

The concentrations of the compounds of the present disclosure and control compounds in plasma and lung tissue were determined using LC-MS/MS. The test results are shown in Tables 2-5.

**Table 2. Pharmacokinetic parameters of compound YF027 of the present disclosure and control compound A**

| Parameters | AUC₀₋ₜ | AUC_{0-∞} | t_{1/2} | CL | Tₘₐₓ | Cₘₐₓ | Lung tissue distribution Cₘₐₓ |
|---|---|---|---|---|---|---|---|
| | ng·h/mL | ng·h/mL | h | L/h/kg | h | ng/mL | ng/g |
| YF027 | 367.05 | 380.76 | 1.83 | 262.63 | 0.5 | 100.26 | 1406.73 |
| Control A | 117.00 | 121.25 | 4.35 | 824.72 | 0.167 | 120.1 | 947.88 |

The results show that the maximum *in vivo* plasma concentration Cₘₐₓ of the compound YF027 of the present disclosure (100.26 ng/mL) was close to that of the non-deuterated control compound A (120.1 ng/mL). However, the *in vivo* exposure AUC₀₋ₜ of YF027 (367.05 ng·h/mL) was significantly higher than that of the control compound A (117.00 ng·h/mL). In addition, the maximum concentration in lung tissue distribution Cₘₐₓ of YF027 (1406.73 ng/g) was also significantly higher than that of the control compound A in lung tissue (947.88 ng/g).

**Table 3. Pharmacokinetic parameters of compound YF032 of the present disclosure and control compound B**

| Parameters | AUC₀₋ₜ | AUC_{0-∞} | t_{1/2} | CL | Tₘₐₓ | Cₘₐₓ | Lung tissue distribution Cₘₐₓ |
|---|---|---|---|---|---|---|---|
| | ng·h/mL | ng·h/mL | h | L/h/kg | h | ng/mL | ng/g |
| YF032 | 14030.17 | 14032.28 | 1.85 | 7.13 | 1.00 | 4332.29 | 26338.0 |
| Control B | 10327.84 | 10367.88 | 1.74 | 9.65 | 1.00 | 3572.6 | 23434.77 |

The results show that the maximum *in vivo* plasma concentration Cₘₐₓ of the compound YF032 of the present disclosure (4332.29 ng/mL) was significantly higher than that of the non-deuterated control compound B (3572.6 ng/mL). The *in vivo* exposure AUC₀₋ₜ of YF032 (14030.17 ng·h/mL) was significantly higher than that of the control compound B (10327.84 ng·h/mL). In addition, the maximum concentration in lung tissue distribution Cₘₐₓ of YF032 (26338.0 ng/g) was also significantly higher than that of the control compound B in lung tissue (23434.77 ng/g).

**Table 4. Pharmacokinetic parameters of compound YF036 of the present disclosure and control compound C**

| Parameters | AUC₀₋ₜ | AUC_{0-∞} | t_{1/2} | CL | Tₘₐₓ | Cₘₐₓ | Lung tissue distribution Cₘₐₓ |
|---|---|---|---|---|---|---|---|
| | ng·h/mL | ng·h/mL | h | L/h/kg | h | ng/mL | ng/g |
| YF036 | 5655.66 | 5883.88 | 4.75 | 17.00 | 0.33 | 2084.39 | 13097.46 |
| Control C | 4960.45 | 5425.93 | 7.12 | 18.43 | 0.5 | 943.7 | 5418.41 |

The results show that the maximum *in vivo* plasma concentration Cₘₐₓ of the compound YF036 of the present disclosure (2084.39 ng/mL) was significantly higher than that of the non-deuterated control compound C (943.7 ng/mL). The *in vivo* exposure AUC₀₋ₜ of YF036 (5655.66 ng·h/mL) was significantly higher than that of the control compound C (4960.45 ng·h/mL). In addition, the maximum concentration in lung tissue distribution Cₘₐₓ of YF036 (13097.46 ng/g) was also significantly higher than that of the control compound C in lung tissue (5418.41 ng/g).

**Table 5. Pharmacokinetic parameters of compound YF044 of the present disclosure and control compound D**

| Parameters | AUC₀₋ₜ | AUC_{0-∞} | t_{1/2} | CL | Tₘₐₓ | Cₘₐₓ | Lung tissue distribution Cₘₐₓ |
|---|---|---|---|---|---|---|---|
| | ng·h/mL | ng·h/mL | h | L/h/kg | h | ng/mL | ng/g |
| YF044 | 11104.68 | 111067.0 | 2.03 | 9.00 | 0.50 | 4041.90 | 43437.42 |
| Control D | 4474.91 | 4498.51 | 2.86 | 22.28 | 91.94 | 2109.55 | 13533.72 |

The results show that the maximum *in vivo* plasma concentration Cₘₐₓ of the compound YF044 of the present disclosure (4041.90 ng/mL) was significantly higher than that of the non-deuterated control compound D (2109.55 ng/mL). The *in vivo* exposure AUC₀₋ₜ of YF044 (11104.68 ng·h/mL) was significantly higher than that of the control compound D (4474.91 ng·h/mL). In addition, the maximum concentration in lung tissue distribution Cₘₐₓ of YF044 (43437.42 ng/g) was also significantly higher than that of the control compound D in lung tissue (13533.72 ng/g).

As can be seen from the results, compared with the control compounds, the deuterated compounds YF027, YF032, YF036, and YF044 of the present disclosure had better pharmacokinetic and lung tissue distribution characteristics in animals and lung tissues (see Tables 2-5), and thus had better pharmacodynamic and therapeutic effects.

### Example 51: Enzyme Metabolic Phenotype Study of Compounds

This example was to detect the CYP450 metabolic enzyme phenotype of the deuterated compound YF027 and its non-deuterated control compound A (using a CYP450 enzyme metabolic phenotype research kit (recombinant enzymatic method/7 enzymes)-mouse liver microsome test).

### 1.1 Preparation of sample solution

a. The test compound was prepared into a 40 mM stock solution for later use. 2.5 µL of the stock solution was taken and diluted with DMSO to a test compound concentration of 200 µM, and the compound at this concentration was used in subsequent tests.
b. The working solution concentration of the mixed positive substrates, phenacetin and testosterone, was 200 µM.

### 1.2 Preparation of incubation system and initiation of reaction

1) The components of the kit were thawed in an ice bath. The mixture was well mixed by vortexing and then placed on ice for later use.
2) In addition to the microsomes, the other components of the incubation systems were mixed according to the ratios and well mixed by pipetting, and the ratios of the incubation systems are shown below;

**Table 6. Incubation system of positive control group**

| Name | Addition amount (µL) |
|---|---|
| Solution B of kit | 1.25 |
| Positive substrate | 0.625 |
| 0.1 M phosphate buffer solution | 120 |

**Table 7. Incubation system of experimental control group**

| Name | Addition amount (µL) |
|---|---|
| Solution B of kit | 1.25 |
| Compound YF027 | 0.625 |
| 0.1 M phosphate buffer solution | 120 |

**Table 8. Incubation system of experimental group**

| Name | Addition amount (µL) |
|---|---|
| Solution B of kit | 1.25 |
| Compound YF027 | 0.625 |
| Selective inhibitor (200×) | 0.625 |
| 0.1 M phosphate buffer solution | 119.375 |

The inhibitors were quinidine, naphthoflavone, pilocarpine, sodium diethyldithiocarbamate, ticlopidine, ketoconazole, and sulfaphenazole, which corresponded to the inhibition of CYP450 enzymes CYP2D6, CYP1A2, CYP2A6, CYP2E1, CYP2C19, CYP3A4, and CYP2C9, respectively.

**Table 9. Incubation system of negative control group**

| Name | Addition amount (µL) |
|---|---|
| Solution B of kit | 1.25 |
| Compound A | 0.625 |
| 0.1 M phosphate buffer solution | 123.125 |

3) Except for the negative control group (Table 9), 3.125 µL of liver microsomes was added to each of the above incubation systems to prepare 125 µL of reaction solution. The reaction solutions were well mixed by vortexing, and then placed in a water bath at 37 °C to initiate the reaction. A timer was set.
4) At time points of 0 min and 90 min, 50 µL of reaction solution was taken, and a pre-cooled acetonitrile solution was added to the reaction solution. The mixture was quickly well mixed by vortexing to stop the reaction.
5) Mass spectrometry detection pretreatment of reaction solution

The reaction solution was cryopreserved in a refrigerator at -80 °C overnight and then centrifuged for mass spectrometry detection.

### 1.3 Results

The experimental results show that the incubation system set in the experiment and the enzymes in the liver microsomes worked normally and had good activity. After 90 min of incubation, the positive substrates phenacetin and testosterone were almost completely metabolized, which was below the lower limit of mass spectrometry detection. The detection values after 90 min of incubation were processed as ratios relative to the 0-min detection values to determine the metabolic rate of the compounds in different experimental groups after 90 min. The metabolic inhibition rate was then calculated using the formula: Inhibition rate (%) = (1 - metabolic rate of sample with inhibitor/metabolic rate of sample without inhibitor) × 100%, as shown in Table 10 below. According to the content of the Technical Guidelines for Drug Interaction Studies (for Trial Implementation) issued by the Center for Drug Evaluation of the National Medical Products Administration, if the total elimination contribution of a specific metabolic enzyme to a drug is ≥ 25%, it can be considered that the enzyme significantly contributes to the clearance of the drug, that is, it is the major metabolic enzyme of the drug. The metabolic inhibition rate reflects the effect on the metabolic elimination of the drug after adding different selective inhibitors. Therefore, when the metabolic inhibition rate of a specific enzyme is ≥ 25%, the contribution of the specific enzyme to the clearance of the drug under study can be inferred to be ≥ 25% based on the results of the *in vitro* metabolism study.

The experimental results show that CYP3A4 was the major metabolic enzyme of compound A, but none of the above 7 CYP metabolic enzymes were major metabolic enzymes of YF027. Therefore, the main *in vivo* metabolic mode of deuterated YF027 was changed, and YF027 was no longer metabolized by the major metabolic enzyme subtype CYP3A4 of CYP450 enzyme, which provides the possibility of combining YF027 with other drugs clinically. For example, since isoniazid has a strong inhibitory effect on the enzymatic activity of CYP3A4, the compound A may cause drug interactions if used in combination with isoniazid.

**Table 10. Experimental results of CYP450 enzyme metabolic phenotype study**

| **CYP450 enzyme** | **Inhibition rate** | |
|---|---|---|
| | Compound A | YF027 |
| **CYP2A6** | 4.2% | 14.0% |
| **CYP2D6** | -8.7% | -1.3% |
| **CYP2C19** | -19.3% | -5.7% |
| **CYP1A2** | -11.7% | 0.4% |
| **CYP3A4** | 76.9% | 16.5% |
| **CYP2C9** | -2.8% | 3.7% |
| **CYP2E1** | -26.5% | -15.1% |

### Example 52: Anti-tuberculosis Activity of Compounds in Mice

### Experimental materials and reagents:

Deuterated compounds YF027, YF032, and YF036 and non-deuterated control compounds thereof (A, B, and C); 5- to 6-week-old BALB/c mice, from Zhejiang Vital River Laboratory Animal Technology Co., Ltd.; *Mycobacterium tuberculosis* H37Rv; vehicle: 10% tween 80-water.

### Experimental method:

Acute infection models were constructed by aerosol infection of mice with *Mycobacterium tuberculosis* H37Rv (about 100-1000 CFU/lung) for 2 days. Each group had 6 mice, the compounds were administered intragastrically for 4 weeks (5 days per week, once daily), and a 10% Tween 80 vehicle control group was also set. 3 days after the end of the administration, the mice were sacrificed, and the lungs were taken to determine the pulmonary bacterial load of the mice (plate counting method), thereby evaluating the *in vivo* bacteriostatic activity of the compounds. The specific doses are shown in Table 11.

The experimental results were analyzed using a Student's t-test bilateral test (unpaired, equal variance). When p ≤ 0.05, the difference was considered statistically significant. ns indicates p > 0.05, * indicates p < 0.05, ** indicates p < 0.01, *** indicates p < 0.001, and **** indicates p < 0.0001.

**Table 11. Specific doses**

| Compound | Dose (mg/kg) |
|---|---|
| YF027 | 100 |
| Compound A | 100 |
| YF032 | 100 |
| Compound B | 50 |
| YF036 | 100 |
| Compound C | 50 |

### Experimental results:

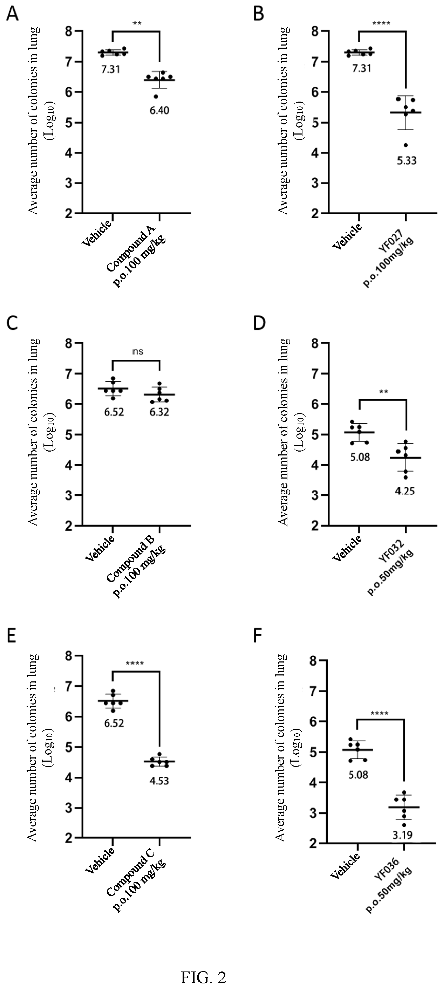

As shown in FIG. 2, the pulmonary bacterial load in the mice after 4 weeks of treatment with compound A was reduced by 0.91 log (FIG. 2A), and the pulmonary bacterial load in the mice after 4 weeks of treatment with deuterated YF027 was reduced by 1.98 log (FIG. 2B), that is, the pulmonary *Mycobacterium tuberculosis* clearance rate in the mice was increased from 87.7% to 99.0% after deuteration of the compound. Moreover, a similar effect was observed after deuteration of compound B. While compound B itself showed no bacteriostatic activity in mice (FIG. 2C), treatment with deuterated YF032 at half of the original dose for 4 weeks resulted in a 0.81 log reduction in bacterial load (FIG. 2D), indicating a significant increase in *in vivo* bacteriostatic activity. Meanwhile, YF036 (50 mg/kg, 1.87 log reduction, FIG. 2F) achieved a similar therapeutic effect with only half the dose of compound C (100 mg/kg, 1.99 log reduction, FIG. 2E). It can be seen that deuteration can significantly improve the *in vivo* bacteriostatic activity of the compound against *Mycobacterium tuberculosis.*

### Example 53: Repeat-Dose Toxicity of Compounds in Animals

### Experimental method:

Administration groups of the compound YF027 of the present disclosure and its non-deuterated control compound A were set for a 28-day repeat-dose toxicity study in mice. Each compound was administered to 10 Balb/c mice (5 females and 5 males, purchased from Beijing Vital River Laboratory Animal Technology Co., Ltd.) by intragastric administration. The administration was performed once daily for 6 consecutive days a week for 4 consecutive weeks, and an 8% Tween 80 vehicle control group (10 Balb/c mice, 5 females and 5 males) was set for administration at the same volume using the same method. The specific daily doses and administration regimens are shown in Table 12.

**Table 12. Administration information of repeat-dose toxicity assay**

| Administration group | Administration mode | Drug formulation concentration (mg/mL) | Dose (mg/kg) | Dose volume (µL/10g) |
|---|---|---|---|---|
| Vehicle control | Intragastric administration | 0 | 0 | 100 |
| YF027 | Intragastric administration | 10 | 100 | 100 |
| Compound A | Intragastric administration | 10 | 100 | 100 |

During the administration period, the mice were observed every day. The body weight, appearance signs, behavior and activity, fecal traits, and local reactions at the site of administration were recorded. After the end of the administration, the gross anatomy was performed on the mice in the administration group and the control group. The serum of the mice was collected, and the heart, liver, spleen, kidney, lung, and brain tissues and organs were weighed to calculate organ coefficients. The tissue samples of all groups were subjected to histopathological detection, and the serum samples were analyzed for biochemical parameters of liver and kidney functions. The serum biochemical parameters included: alanine aminotransferase (ALT), aspartate aminotransferase (AST), alkaline phosphatase (ALP), urea (UREA), creatinine (CREA), total protein (TP), and albumin (ALB). Statistical analysis was performed to evaluate potential toxic effects of the compounds.

### Experimental results:

During the administration period, no mice died in both compound YF027 and compound A administration groups. Compared with the vehicle control group, the body weight gain of the mice in the administration groups was normal. Male mice in the YF027 group and the compound A group exhibited piloerection during the administration period, while no significant abnormalities were found in other physical signs. After the administration was completed, gross necropsy was performed, and the organ-to-body weight ratios of major tissues and organs were calculated. The mice in the YF027 group and the compound A group showed no significant statistical difference compared with those of the vehicle control group. Although a slight decrease in the creatinine ratio was found in the YF027 group compared with the vehicle control group, this change was not considered pathologically significant. The mice in the compound A group showed no significant statistical difference compared with those of the vehicle control group. Histopathological examination of major tissues and organs also showed no significant treatment-related lesions.

The results show that the compound YF027 provided by the present disclosure did not produce a significant toxic response in mice after repeated administration at a dose of 100 mg/kg for 28 consecutive days. Under the same dosing conditions, compared with the non-deuterated compound A, no increased toxicity was observed in mice treated with the compound YF027.

## Claims

1. A compound represented by formula I, or a pharmaceutically acceptable salt, a stereoisomer, a tautomer, an N-oxide, a hydrate, a solvate, or a prodrug thereof: 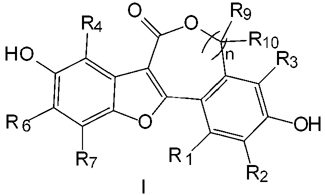
wherein n is 0 or 1;
R₄ is selected from H, halogen, an aldehyde group, hydroxy, sulfhydryl, -NR'R", cyano, nitro, optionally substituted C₁-C₁₀ alkyl, optionally substituted C₂-C₁₀ alkenyl, optionally substituted C₂-C₁₀ alkynyl, optionally substituted C₃-C₈ carbocyclyl, optionally substituted C₆-C₁₄ aryl, optionally substituted C₅-C₁₀ heteroaryl, optionally substituted C₄-C₁₀ heterocyclyl, and respective deuterated variants thereof; preferably, R₄ is selected from optionally substituted C₆-C₁₄ aryl C₁-C₁₀ alkyl, optionally substituted C₅-C₁₀ heteroaryl C₁-C₁₀ alkyl, optionally substituted C₄-C₁₀ heterocyclyl C₁-C₁₀ alkyl, and respective deuterated variants thereof;
R₇ is selected from H, hydroxy, sulfhydryl, cyano, carboxyl, nitro, -NR'R", optionally substituted C₁-C₁₀ alkyl, optionally substituted C₂-C₁₀ alkenyl, optionally substituted C₂-C₁₀ alkynyl, C₁-C₁₀ ester group, amido, halogen, optionally substituted C₃-C₈ carbocyclyl, optionally substituted C₁-C₁₀ alkoxy, optionally substituted C₆-C₁₄ aryl, optionally substituted C₅-C₁₀ heteroaryl, optionally substituted C₄-C₁₀ heterocyclyl, and respective deuterated variants thereof, wherein R' and R" are each independently selected from: H, optionally substituted C₁-C₁₀ alkyl, optionally substituted C₂-C₈ alkenyl, and optionally substituted C₁-C₁₀ alkoxy;
R₁, R₂, R₃, R₆, R₉, and R₁₀ are independently selected from hydrogen and deuterium; and
the structure represented by formula I comprises at least one deuterium atom.

2. The compound, or the pharmaceutically acceptable salt, the stereoisomer, the tautomer, the N-oxide, the hydrate, the solvate, or the prodrug thereof according to claim 1, wherein
R₄ is -(CR₁₁R₁₂)ₘ-NRₐR_{b},
wherein m is 1, 2, or 3;
R₁₁ and R₁₂ are independently selected from hydrogen and deuterium; and
Rₐ and R_{b} are each independently selected from the following substituents and respective deuterated variants thereof: H; C₁-C₁₀ alkyl optionally substituted with 1-5 substituents selected from halogen and hydroxy; C₂-C₈ alkenyl optionally substituted with 1-5 substituents selected from halogen and hydroxy; 3- to 14-membered carbocyclyl optionally substituted with 1-5 substituents selected from halogen and C₁-C₄ alkyl; C₁-C₆ alkyl substituted with 3- to 14-membered carbocyclyl optionally substituted with 1-5 substituents selected from halogen and C₁-C₄ alkyl; C₁-C₆ alkyl substituted with 4- to 10-membered heterocyclyl optionally substituted with 1-5 substituents selected from halogen, hydroxy, and C₁-C₄ alkyl; C₆-C₁₄ aryl optionally substituted with 1-5 substituents selected from halogen, hydroxy, and C₁-C₄ alkyl; and C₆-C₁₄ aryl-C₁-C₆ alkyl optionally substituted with 1-5 substituents selected from halogen, hydroxy, and C₁-C₄ alkyl; or Rₐ and R_{b}, together with the nitrogen atom to which they are attached, form an optionally substituted 4- to 7-membered heterocyclyl or benzo 4- to 7-membered heterocyclyl or a deuterated variant thereof, wherein the 4-to 7-membered heterocyclyl or benzo 4- to 7-membered heterocyclyl is optionally substituted with 1-5 substituents selected from: C₁-C₆ alkyl optionally substituted with 1-5 substituents selected from halogen and hydroxy; C₁-C₆ alkoxy optionally substituted with 1-5 substituents selected from halogen and hydroxy; cyano; carboxyl; halogen; -NR^{c}R^{d}; hydroxy; C₆-C₁₄ aryl optionally substituted with 1-5 substituents selected from C₁-C₄ acyl, halogen, NR^{c}R^{d}, C₁-C₄ alkoxy, C₁-C₄ alkyl, cyano, hydroxy, C₁-C₄ alkoxycarbonyl, C₆-C₁₄ aryl, and halogenated C₁-C₄ alkyl; C₁-C₆ acyl; C₁-C₆ alkoxycarbonyl; C₂-C₈ alkenyloxycarbonyl; C₁-C₆ acyl substituted with NR^{c}R^{d}; C₁-C₄ acyl substituted with C₆-C₁₄ aryl optionally substituted with 1-5 substituents selected from halogen, C₁-C₄ alkyl, halogenated C₁-C₄ alkyl, and hydroxy; C₁-C₆ alkyl substituted with C₆-C₁₄ aryl optionally substituted with 1-5 substituents selected from halogen, C₁-C₄ alkyl, halogenated C₁-C₄ alkyl, and hydroxy; C₁-C₆ alkyl substituted with C₃-C₈ cycloalkyl optionally substituted with 1-5 substituents selected from halogen, C₁-C₄ alkyl, halogenated C₁-C₄ alkyl, and hydroxy; C₃-C₈ cycloalkyl optionally substituted with 1-5 substituents selected from halogen, C₁-C₄ alkyl, halogenated C₁-C₄ alkyl, and hydroxy; 4- to 7-membered heterocyclyl optionally substituted with 1-5 substituents selected from halogen, C₁-C₄ alkyl, halogenated C₁-C₄ alkyl, and hydroxy; C₁-C₆ alkyl substituted with 4- to 7-membered heterocyclyl optionally substituted with 1-5 substituents selected from halogen, C₁-C₄ alkyl, halogenated C₁-C₄ alkyl, and hydroxy; 5- to 10-membered heteroaryl optionally substituted with 1-5 substituents selected from halogen, C₁-C₄ alkyl, halogenated C₁-C₄ alkyl, and hydroxy; and C₁-C₆ alkyl substituted with 5- to 10-membered heteroaryl optionally substituted with 1-5 substituents selected from halogen, C₁-C₄ alkyl, halogenated C₁-C₄ alkyl, and hydroxy, wherein R^{c} and R^{d} are each independently selected from H, C₁-C₆ alkyl, C₂-C₈ alkenyl, and C₁-C₆ alkoxy.

3. The compound, or the pharmaceutically acceptable salt, the stereoisomer, the tautomer, the N-oxide, the hydrate, the solvate, or the prodrug thereof according to claim 2, wherein
-NRₐR_{b} is selected from: 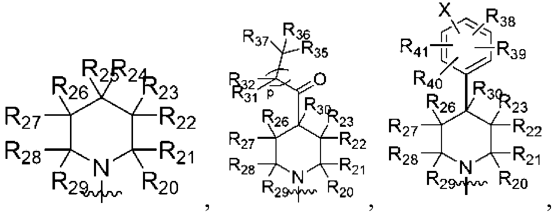 and 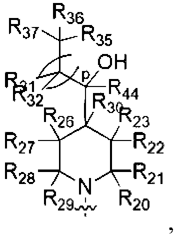 wherein p is independently 0, 1, 2, or 3, X is halogen, and R₂₀ to R₃₂, R₃₅ to R₄₁, and R₄₄ are independently selected from hydrogen and deuterium;
preferably, -NRₐR_{b} is selected from: 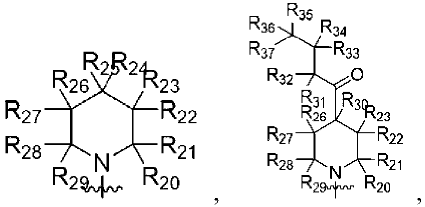 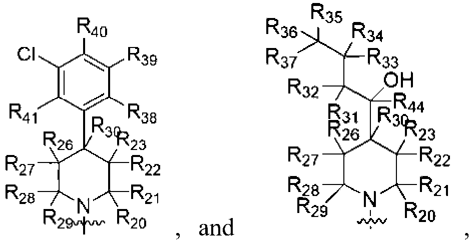 wherein R₂₀ to R₄₁, and R₄₄ are independently selected from hydrogen and deuterium;
more preferably, -NRₐR_{b} is selected from:

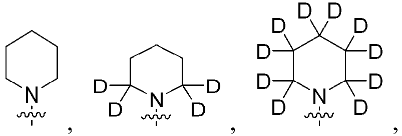

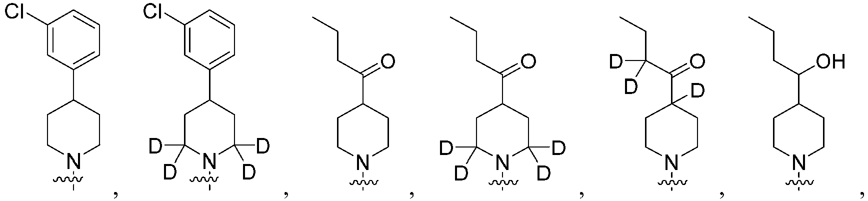

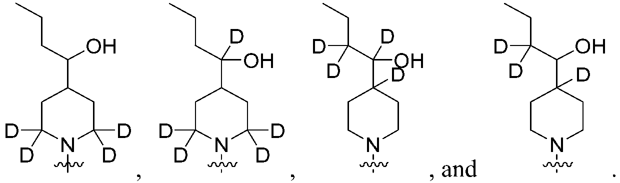

4. The compound, or the pharmaceutically acceptable salt, the stereoisomer, the tautomer, the N-oxide, the hydrate, the solvate, or the prodrug thereof according to any one of claims 1 to 3, wherein
R₇ is selected from H, C₁-C₆ alkyl optionally substituted with 1-5 substituents selected from halogen and hydroxy, C₂-C₆ alkenyl optionally substituted with 1-5 substituents selected from halogen and hydroxy, saturated or partially saturated C₃-C₈ carbocyclyl, halogen, hydroxy, C₁-C₄ alkoxy substituted with C₆-C₁₄ aryl, 5- to 10-membered heteroaryl, and C₆-C₁₄ aryl optionally substituted with 1-3 substituents selected from C₁-C₄ alkyl, halogenated C₁-C₄ alkyl, halogen, C₂-C₄ alkenyl, and hydroxy, as well as respective deuterated variants thereof;
preferably, R₇ is selected from hydrogen, deuterium, and 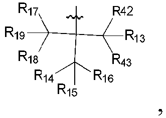 wherein R₁₃ to R₁₉, R₄₂, and R₄₃ are independently selected from hydrogen and deuterium;
preferably, R₇ is selected from hydrogen, deuterium, and

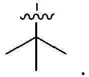

5. The compound, or the pharmaceutically acceptable salt, the stereoisomer, the tautomer, the N-oxide, the hydrate, the solvate, or the prodrug thereof according to any one of claims 1 to 4, wherein the structure represented by formula I comprises 2, 3, 4, 5, 6, 7, 8, 9, 10, 11, 12, 13, 14, 15, 16, 17, 18, 19, or 20 deuterium atoms.

6. A compound represented by formula II, or a pharmaceutically acceptable salt, a stereoisomer, a tautomer, an N-oxide, a hydrate, a solvate, or a prodrug thereof, 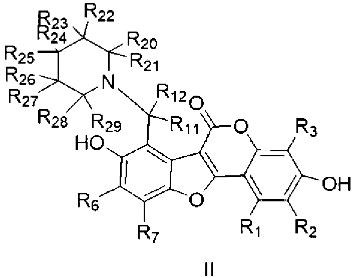 wherein,
R₇ is selected from hydrogen, deuterium, and 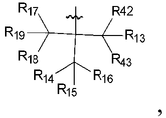 wherein R₁₃ to R₁₉, R₄₂, and R₄₃ are independently selected from hydrogen and deuterium; preferably, R₇ is 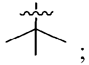
R₁, R₂, R₃, R₆, R₁₁, R₁₂, and R₂₀ to R₂₉ are independently selected from hydrogen and deuterium; and
the structure represented by formula II comprises at least one deuterium atom, preferably 2, 3, 4, 5, 6, 7, 8, 9, 10, 11, 12, 13, 14, 15, 16, 17, 18, 19, or 20 deuterium atoms.

7. The compound, or the pharmaceutically acceptable salt, the stereoisomer, the tautomer, the N-oxide, the hydrate, the solvate, or the prodrug thereof according to claim 6, wherein the compound has a structural formula shown as IIa, IIb, IIc, or IId below:

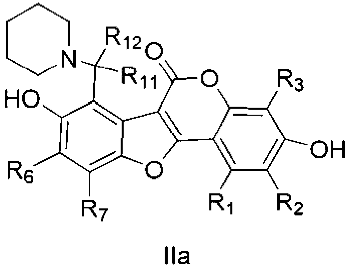

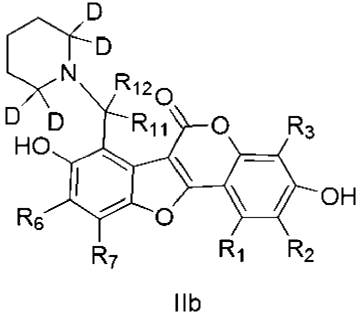

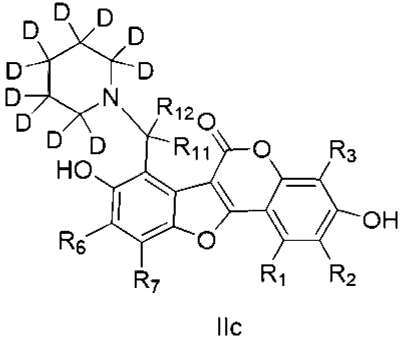

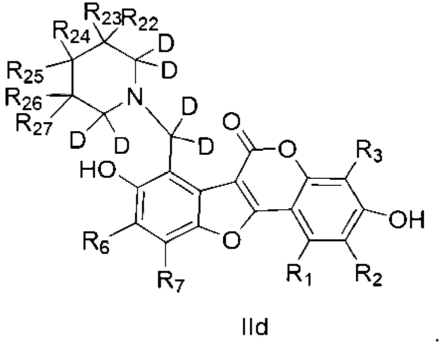

8. A compound represented by formula III, or a pharmaceutically acceptable salt, a stereoisomer, a tautomer, an N-oxide, a hydrate, a solvate, or a prodrug thereof, 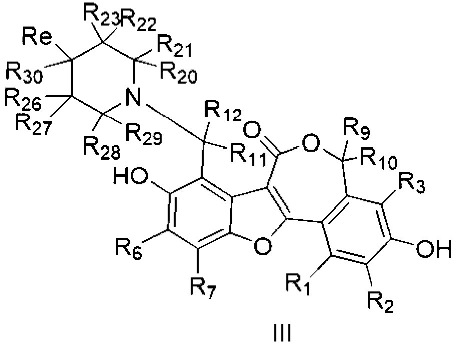
wherein Re is selected from: C₁-C₆ alkyl optionally substituted with 1-5 substituents selected from halogen and hydroxy; C₁-C₆ alkoxy optionally substituted with 1-5 substituents selected from halogen and hydroxy; cyano; carboxyl; halogen; -NR^{c}R^{d}; hydroxy; C₆-C₁₄ aryl optionally substituted with 1-5 substituents selected from C₁-C₄ acyl, halogen, NR^{c}R^{d}, C₁-C₄ alkoxy, C₁-C₄ alkyl, cyano, hydroxy, C₁-C₄ alkoxycarbonyl, C₆-C₁₄ aryl, and halogenated C₁-C₄ alkyl; C₁-C₆ acyl; C₁-C₆ alkoxycarbonyl; C₂-C₈ alkenyloxycarbonyl; C₁-C₆ acyl substituted with NR^{c}R^{d}; C₁-C₄ acyl substituted with C₆-C₁₄ aryl optionally substituted with 1-5 substituents selected from halogen, C₁-C₄ alkyl, halogenated C₁-C₄ alkyl, and hydroxy; C₁-C₆ alkyl substituted with C₆-C₁₄ aryl optionally substituted with 1-5 substituents selected from halogen, C₁-C₄ alkyl, halogenated C₁-C₄ alkyl, and hydroxy; C₁-C₆ alkyl substituted with C₃-C₈ cycloalkyl optionally substituted with 1-5 substituents selected from halogen, C₁-C₄ alkyl, halogenated C₁-C₄ alkyl, and hydroxy; C₃-C₈ cycloalkyl optionally substituted with 1-5 substituents selected from halogen, C₁-C₄ alkyl, halogenated C₁-C₄ alkyl, and hydroxy; 4- to 7-membered heterocyclyl optionally substituted with 1-5 substituents selected from halogen, C₁-C₄ alkyl, halogenated C₁-C₄ alkyl, and hydroxy; C₁-C₆ alkyl substituted with 4- to 7-membered heterocyclyl optionally substituted with 1-5 substituents selected from halogen, C₁-C₄ alkyl, halogenated C₁-C₄ alkyl, and hydroxy; 5- to 10-membered heteroaryl optionally substituted with 1-5 substituents selected from halogen, C₁-C₄ alkyl, halogenated C₁-C₄ alkyl, and hydroxy; and C₁-C₆ alkyl substituted with 5- to 10-membered heteroaryl optionally substituted with 1-5 substituents selected from halogen, C₁-C₄ alkyl, halogenated C₁-C₄ alkyl, and hydroxy; and respective deuterated variants thereof, wherein R^{c} and R^{d} are each independently selected from H, C₁-C₆ alkyl, C₂-C₈ alkenyl, and C₁-C₆ alkoxy;
R₇ is selected from H, C₁-C₆ alkyl optionally substituted with 1-5 substituents selected from halogen and hydroxy, C₂-C₆ alkenyl optionally substituted with 1-5 substituents selected from halogen and hydroxy, saturated or partially saturated C₃-C₈ carbocyclyl, halogen, hydroxy, C₁-C₄ alkoxy substituted with C₆-C₁₄ aryl, 5- to 10-membered heteroaryl, and C₆-C₁₄ aryl optionally substituted with 1-3 substituents selected from C₁-C₄ alkyl, halogenated C₁-C₄ alkyl, halogen, C₂-C₄ alkenyl, and hydroxy, as well as respective deuterated variants thereof; preferably, R₇ is selected from hydrogen,
deuterium, and 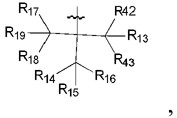 wherein R₁₃ to R₁₉, R₄₂, and R₄₃ are each independently selected from hydrogen and deuterium; more preferably, R₇ is hydrogen or deuterium;
R₁, R₂, R₃, R₆, R₉ to R₁₂, R₂₀ to R₂₃, and R₂₆ to R₃₀ are each independently selected from hydrogen and deuterium; and
the structure represented by formula III comprises at least one deuterium atom, preferably 2, 3, 4, 5, 6, 7, 8, 9, 10, 11, 12, 13, 14, 15, 16, 17, 18, 19, or 20 deuterium atoms.

9. The compound, or the pharmaceutically acceptable salt, the stereoisomer, the tautomer, the N-oxide, the hydrate, the solvate, or the prodrug thereof according to claim 8, wherein the compound has a structural formula shown as IIIa, IIIb, or IIIc below: 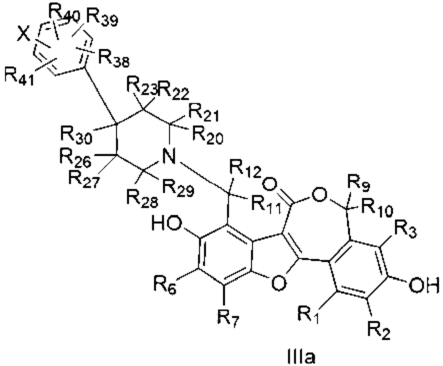 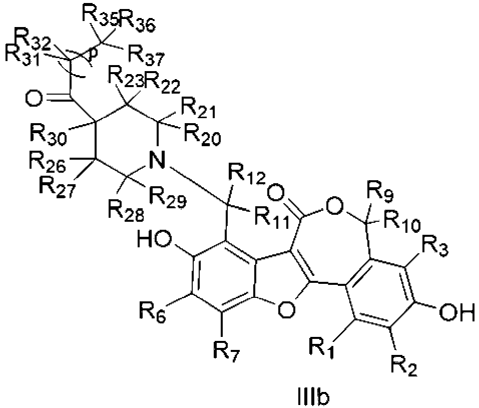 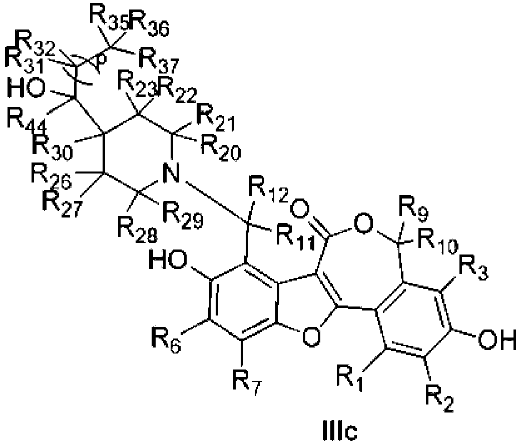 wherein p is independently 0, 1, 2, or 3, X is halogen, and R₃₁, R₃₂, R₃₅ to R₄₁, and R₄₄ are independently selected from hydrogen and deuterium; preferably, R₁, R₂, and R₃ are all hydrogen.

10. The compound, or the pharmaceutically acceptable salt, the stereoisomer, the tautomer, the N-oxide, the hydrate, the solvate, or the prodrug thereof according to claim 9, wherein the compound has a structural formula shown as IIId, IIIe, or IIIf below:

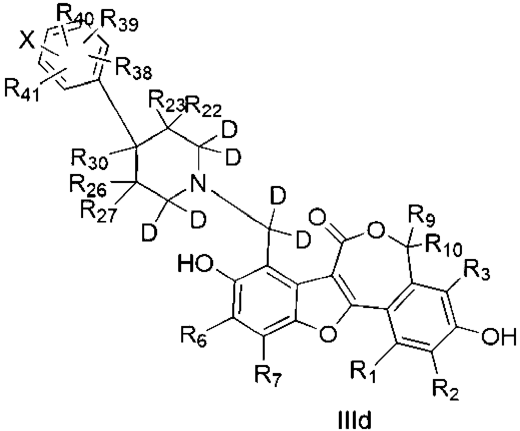

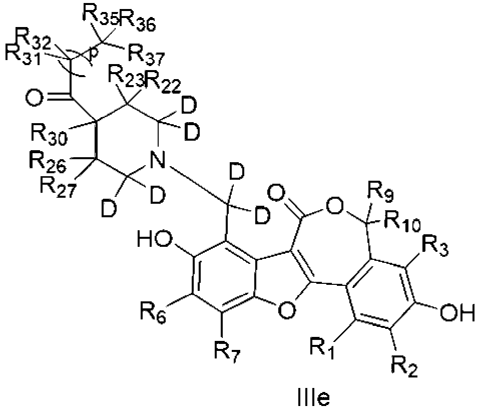

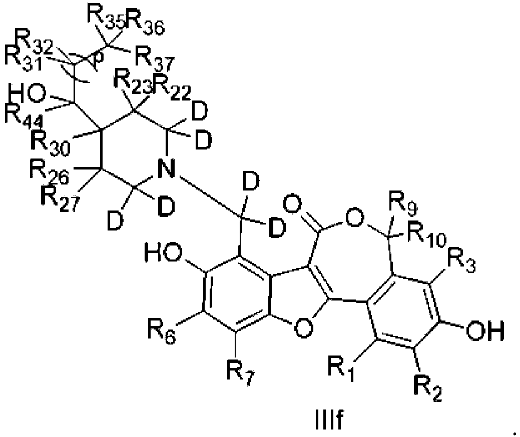

11. The compound, or the pharmaceutically acceptable salt, the stereoisomer, the tautomer, the N-oxide, the hydrate, the solvate, or the prodrug thereof according to claim 8, wherein Re, together with the piperidinyl attached thereto, forms a group selected from:

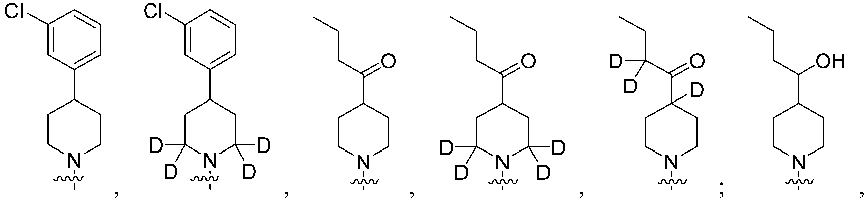

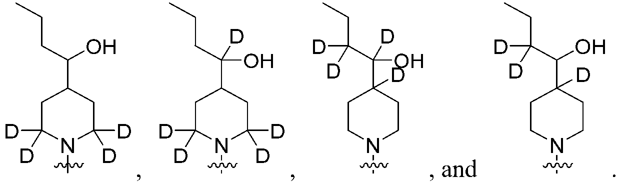

12. A compound of formula IV, or a pharmaceutically acceptable salt, a stereoisomer, a tautomer, an N-oxide, a hydrate, a solvate, or a prodrug thereof, 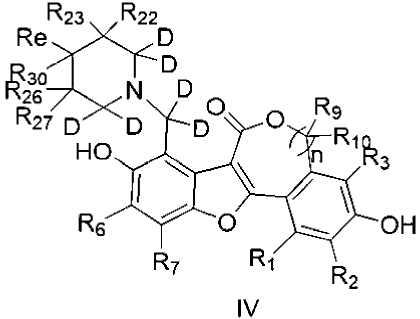
wherein n is 0 or 1;
Re is selected from: hydrogen, deuterium, C₁-C₆ alkyl optionally substituted with 1-5 substituents selected from halogen and hydroxy; C₁-C₆ alkoxy optionally substituted with 1-5 substituents selected from halogen and hydroxy; cyano; carboxyl; halogen; - NR^{c}R^{d}; hydroxy; C₆-C₁₄ aryl optionally substituted with 1-5 substituents selected from C₁-C₄ acyl, halogen, NR^{c}R^{d}, C₁-C₄ alkoxy, C₁-C₄ alkyl, cyano, hydroxy, C₁-C₄ alkoxycarbonyl, C₆-C₁₄ aryl, and halogenated C₁-C₄ alkyl; C₁-C₆ acyl; C₁-C₆ alkoxycarbonyl; C₂-C₈ alkenyloxycarbonyl; C₁-C₆ acyl substituted with NR^{c}R^{d}; C₁-C₄ acyl substituted with C₆-C₁₄ aryl optionally substituted with 1-5 substituents selected from halogen, C₁-C₄ alkyl, halogenated C₁-C₄ alkyl, and hydroxy; C₁-C₆ alkyl substituted with C₆-C₁₄ aryl optionally substituted with 1-5 substituents selected from halogen, C₁-C₄ alkyl, halogenated C₁-C₄ alkyl, and hydroxy; C₁-C₆ alkyl substituted with C₃-C₈ cycloalkyl optionally substituted with 1-5 substituents selected from halogen, C₁-C₄ alkyl, halogenated C₁-C₄ alkyl, and hydroxy; C₃-C₈ cycloalkyl optionally substituted with 1-5 substituents selected from halogen, C₁-C₄ alkyl, halogenated C₁-C₄ alkyl, and hydroxy; 4- to 7-membered heterocyclyl optionally substituted with 1-5 substituents selected from halogen, C₁-C₄ alkyl, halogenated C₁-C₄ alkyl, and hydroxy; C₁-C₆ alkyl substituted with 4- to 7-membered heterocyclyl optionally substituted with 1-5 substituents selected from halogen, C₁-C₄ alkyl, halogenated C₁-C₄ alkyl, and hydroxy; 5- to 10-membered heteroaryl optionally substituted with 1-5 substituents selected from halogen, C₁-C₄ alkyl, halogenated C₁-C₄ alkyl, and hydroxy; C₁-C₆ alkyl substituted with 5- to 10-membered heteroaryl optionally substituted with 1-5 substituents selected from halogen, C₁-C₄ alkyl, halogenated C₁-C₄ alkyl, and hydroxy; and respective deuterated variants thereof, wherein R^{c} and R^{d} are each independently selected from H, C₁-C₆ alkyl, C₂-C₈ alkenyl, and C₁-C₆ alkoxy;
R₇ is selected from H, C₁-C₆ alkyl optionally substituted with 1-5 substituents selected from halogen and hydroxy, C₂-C₆ alkenyl optionally substituted with 1-5 substituents selected from halogen and hydroxy, saturated or partially saturated C₃-C₈ carbocyclyl, halogen, hydroxy, C₁-C₄ alkoxy substituted with C₆-C₁₄ aryl, 5- to 10-membered heteroaryl, and C₆-C₁₄ aryl optionally substituted with 1-3 substituents selected from C₁-C₄ alkyl, halogenated C₁-C₄ alkyl, halogen, C₂-C₄ alkenyl, and hydroxy, as well as respective deuterated variants thereof; preferably, R₇ is selected from hydrogen,
deuterium, and 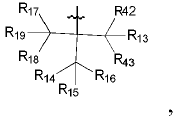 wherein R₁₃ to R₁₉, R₄₂, and R₄₃ are each independently selected from hydrogen and deuterium; more preferably, R₇ is selected from hydrogen, deuterium, and 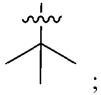
R₁, R₂, R₃, R₆, R₉, R₁₀, R₂₂, R₂₃, R₂₆, R₂₇, and R₃₀ are each independently selected from hydrogen and deuterium; preferably, R₁, R₂, and R₃ are all hydrogen, and R₆, R₉, R₁₀, R₂₂, R₂₃, R₂₆, R₂₇, and R₃₀ are each independently selected from hydrogen and deuterium.

13. The compound, or the pharmaceutically acceptable salt, the stereoisomer, the tautomer, the N-oxide, the hydrate, the solvate, or the prodrug thereof according to claim 12, wherein Re, together with the piperidinyl attached thereto, forms a group selected from: 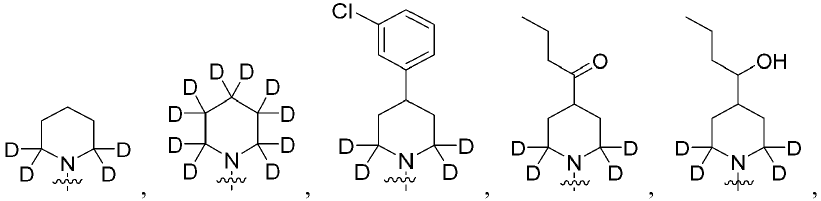 and

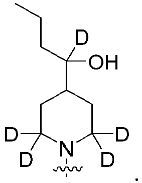

14. A compound having a structural formula selected from the following structural formulas, or a pharmaceutically acceptable salt, a stereoisomer, a tautomer, an N-oxide, a hydrate, a solvate, or a prodrug thereof:

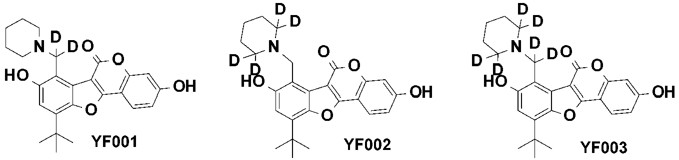

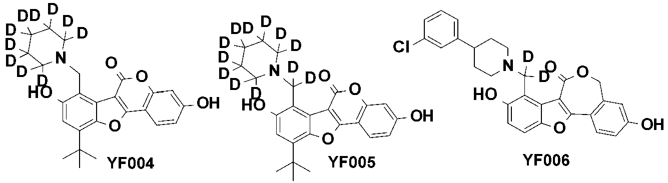

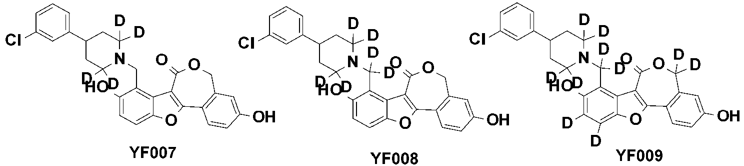

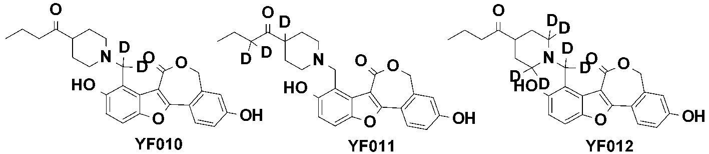

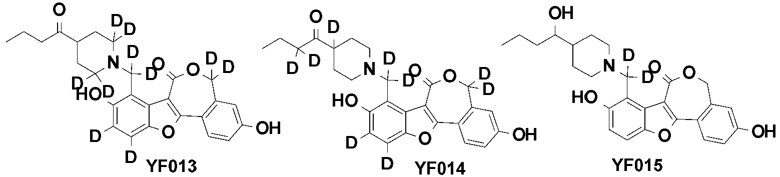

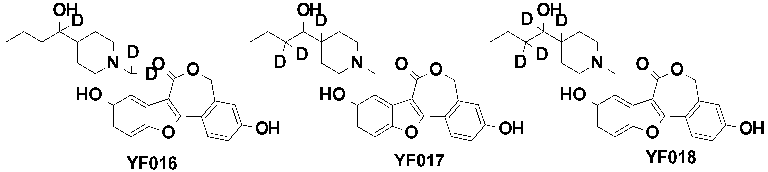

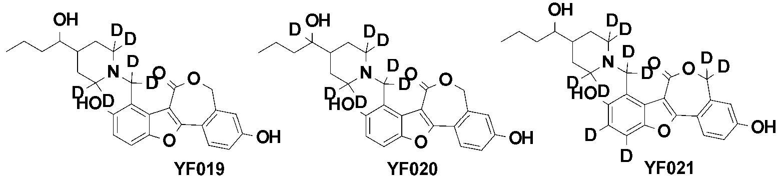

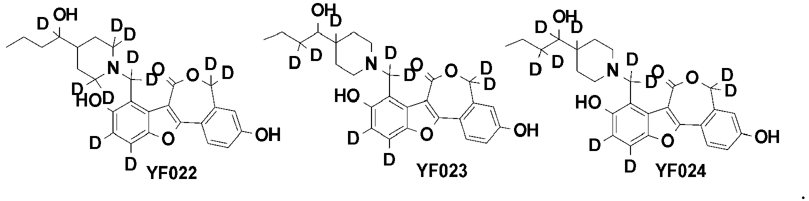

15. The compound, or the pharmaceutically acceptable salt, the stereoisomer, the tautomer, the N-oxide, the hydrate, the solvate, or the prodrug thereof according to any one of claims 1 to 14, wherein the pharmaceutically acceptable salt is a hydrochloride.

16. A pharmaceutical composition, comprising:
(i) a prophylactically or therapeutically effective amount of the compound, or the pharmaceutically acceptable salt, the stereoisomer, the tautomer, the N-oxide, the hydrate, the solvate, or the prodrug thereof according to any one of claims 1 to 15 as an active ingredient; and
(ii) a pharmaceutically acceptable carrier or excipient,
wherein the pharmaceutical composition optionally comprises: (iii) a second active ingredient, wherein preferably, the second active ingredient is an antibacterial agent, preferably selected from: rifampicin, ethambutol, pyrazinamide, isoniazid, levofloxacin, moxifloxacin, gatifloxacin, ofloxacin, kanamycin, amikacin, capreomycin, streptomycin, ethionamide, prothionamide, cycloserine, terizidone, para-aminosalicylic acid, clofazimine, clarithromycin, amoxicillin-clavulanate, delamanid, pretomanid, bedaquiline, sutezolid, TB47, GSK 3036656, gepotidacin, thioacetazone, meropenem-clavulanate, TBA-7371, OPC-167832, Telacebec (Q203), BTZ-043, Contezolid (MRX-4/MRX-1), Delpazolid (LCB01-0371), Macozinone, Pretomanid, SPR720, SQ109, TBI-166, TBI-223, and thioridazine.

17. Use of the compound, or the pharmaceutically acceptable salt, the stereoisomer, the tautomer, the N-oxide, the hydrate, the solvate, or the prodrug thereof according to any one of claims 1 to 15, or the pharmaceutical composition according to claim 16 in the preparation of a medicament for preventing or treating a disease caused by bacterial infection, wherein preferably, the bacterium is selected from: *Mycobacterium tuberculosis,* drug-resistant *Mycobacterium tuberculosis, Mycobacterium smegmatis, Klebsiella pneumoniae, Staphylococcus aureus, Pseudomonas aeruginosa, Acinetobacter baumannii, Mycobacterium leprae, Mycobacterium bovis, Mycobacterium marinum, Corynebacterium diphtheriae, Bordetella pertussis, Haemophilus influenzae,* and *Streptococcus pneumoniae;* particularly preferably, the bacterium is *Mycobacterium tuberculosis* or drug-resistant *Mycobacterium tuberculosis.*

18. The use according to claim 17, wherein the disease is a pulmonary infectious disease, preferably an infectious disease caused by pulmonary tubercle bacillus, and more preferably tuberculosis.
